# EUROPEAN PATENT APPLICATION

(11) **EP 1 057 825 A1**
(43) Date of publication of application: **06.12.2000**
(21) Application number: 99901889.8
(22) Date of filing: 27.01.1999
(51) Int. Cl.: C07D 311/58, A61K 31/35, A23L 1/30, A61K 7/00

(54) **ISOFLAVANE DERIVATIVES AND IMMUNOPOTENTIATING COMPOSITIONS CONTAINING THE SAME**

(30) Priority: 27.01.1998 JP 1393798
(71) Applicant: SHIONOGI & CO., LTD., Osaka-shi, Osaka 541-0045 (JP)
(72) Inventor: MASAKI, Shunichiro, Kosei-cho, Koka-gun, Shiga 520-3242 (JP); TOJYO, Takehiko, Kobe-shi, Hyogo 658-0032 (JP); TAKASHIMA, Akira, Kosei-cho, Koka-gun, Shiga 520-3221 (JP); SEO, Shujiro, Ikoma-shi, Nar a 630-0212 (JP)
(74) Representative: Harding, Charles Thomas
(86) International application number: JP9900346
(87) International publication number: WO9937633

(57) **Abstract**

An objective of the present invention is to provide a specific compound and composition for the prevention or therapy of diseases associated with immunodeficiency.

Isoflavan derivatives which are a glycyrrhiza extract and chemical derivatives thereof, i.e., a compound represented by general formula (II): 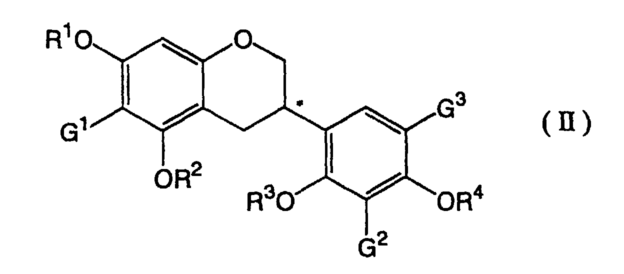 [where R¹, R², R³, and R⁴ may be identical with or different from one another and may each be hydrogen, an alkyl group which may be substituted, an alkenyl group which may be substituted, an alkynyl group which may be substituted, an acyl group which may be substituted, an aralkyl group which may be substituted, or a tri-substituted silyl group; G¹, G², and G³ may be identical with or different from one another and may each be a hydrogen, an acyl group which may be substituted, or an aliphatic hydrocarbon group which may be substituted; symbol (*) indicates the presence of an asymmetric carbon atom, meaning an R form, an S form, or a mixture thereof], or a salt thereof, or a hydrate thereof has immunoactivating actions, and is expected to provide effects as a biophylaxis function promoter by way of lymphocyte function promoting actions and bone marrow function promoting actions.

## Description

### TECHNICAL FIELD

The present invention relates to fields such as medical drugs, animal drugs (zootechnical drugs, aquicultural drugs, etc.), and the like. More particularly, the present invention relates to isoflavan derivatives for the prevention or treatment of diseases associated with immunodeficiency, and compositions containing the same and having immunoactivating action.

### BACKGROUND ART

In recent years, various immunoactivators (main components of bacteria: muramyldipeptide, OK-432; main components of mushrooms: Krestin, lenthinan; synthetic compounds: Levamisole, bestatin; peptide drugs: G-CSF, GM-CSF) have been used for the prevention or treatment of diseases associated with the deterioration of biophylaxis functions, e.g., opportunistic infections, disorders due to radiation, cancer, or infections of cancer patients or HIV patients and the like. However, a number of such drugs have been found to have various side effects. For this reason, compounds with a novel backbone have been desired which hardly have the serious side effects that are observed with known immunoactivators and which can be orally administered.

Since long ago, glycyrrhiza has been well known as a kind of crude drug that has actions such as antitussive, expectoration, and spasmolysis. One of its effective components, glycyrrhizin, is widely used as a hepatotonic drug. Glycyrrhiza also contains many components other than glycyrrhizin. It has been reported that antiviral actions (Japanese Laid-Open Publication No. 1-175942), immunoenhancement actions (mitogen activity, antibody production enhancement actions)(Japanese Laid-Open Publication No. 5-262658), and hepatotonic actions and infection protection actions (Japanese Laid-Open Publication No. 9-143085) are found in a hot or warm-water extract of glycyrrhiza after the extraction of glycyrrhizin therefrom. However, active substances have yet to be identified.

### (Problems to be solved by the invention)

Although glycyrrhiza extracts have been known to have the aforementioned actions, such pharmacological actions are expressed as a complex action of the respective components that are contained in glycyrrhiza, and the pharmacological actions of the individual components have not been clearly described. The present inventors have undertaken research to locate compounds which are related to the immunoactivating actions. It is presumable that the individual components contained in glycyrrhiza also have a number of unknown actions other than those mentioned above.

### DISCLOSURE OF THE INVENTION

After much dedicated effort, the present inventors have found compounds which have an isoflavan backbone having immunoactivating actions.

Specifically, the present invention relates to (1) a compound represented by general formula (I), or a salt thereof, or a hydrate thereof: 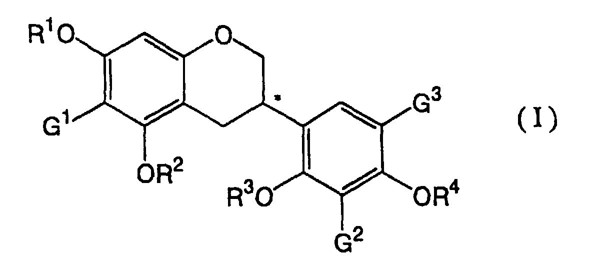 [where R¹, R², R³, and R⁴ may be identical with or different from one another and may each be hydrogen, an alkyl group which may be substituted, an alkenyl group which may be substituted, an alkynyl group which may be substituted, an acyl group which may be substituted, an aralkyl group which may be substituted, or a tri-substituted silyl group; G¹, G², and G³ may be identical with or different from one another and may each be a hydrogen, an acyl group which may be substituted, or an aliphatic hydrocarbon group which may be substituted; symbol (*) indicates the presence of an asymmetric carbon atom, meaning an R form, an S form, or a mixture thereof, with the provisos that:
G³ is not hydrogen in the case where R¹ is hydrogen, are R² is methyl, R³ and R⁴ are hydrogen, and G¹ and G² are 3-methyl-2-butenyl;
G³ is not hydrogen in the case where R¹ is acetyl, R² is methyl, R³ and R⁴ are acetyl, and G¹ and G² are 3-methyl-2-butenyl;
G³ is not hydrogen in the case where R¹ is methyl, R², R³, and R⁴ are hydrogen, and G¹ and G² are 3-methyl-2-butenyl;
G³ is not hydrogen in the case where R¹ and R² are methyl, R³ and R⁴ are hydrogen, and G¹ and G² are 3-methyl-2-butenyl;
G³ is not hydrogen in the case where R¹ is hydrogen, R² is methyl, R³ and R⁴ are hydrogen, G¹ is 3-methyl-2-butenyl, and G² is hydrogen;
G³ is not hydrogen in the case where R¹ and R² are methyl, R³ and R⁴ are hydrogen, G¹ is 3-methyl-2-butenyl, and G² is hydrogen;
G³ is not hydrogen in the case where R¹ and R² are methyl, R³ and R⁴ are acetyl, G¹ is 3-methyl-2-butenyl, and G² is hydrogen;
G³ is not 3-methylbutyl in the case where R¹ is hydrogen, R² is methyl, R³ and R⁴ are hydrogen, G¹ is 3-methylbutyl, and G² is hydrogen;
G³ is not hydrogen in the case where R¹ is hydrogen, R² and R³ are methyl, R⁴ is hydrogen, G¹ is 3-methyl-2-butenyl, and G² is hydrogen;
G³ is not hydrogen in the case where R¹ and R² are methyl, R³ and R⁴ are 2-(trimethylsilyl)ethoxymethyl (SEM), G¹ is 3-methyl-2-butenyl, and G² is hydrogen;
G³ is not hydrogen in the case where R¹ and R² are methyl, R³ and R⁴ are 2-(trimethylsilyl)ethoxymethyl (SEM), G¹ is 2-hydroxy-3-methylbutyl, and G² is hydrogen;
G³ is not hydrogen in the case where R¹ and R² are methyl, R³ is hydrogen, R⁴ is t-butyldimethylsilyl (TBS), G¹ is 3-methyl-2-butenyl, and G² is hydrogen;
G³ is not 3-methyl-2-butenyl in the case where R¹ and R² are methyl, R³ and R⁴ are hydrogen, and G¹ and G² are 3-methyl-2-butenyl;
G³ is not hydrogen in the case where R¹ and R² are methyl, R³ is hydrogen, R⁴ is t-butyldimethylsilyl (TBS), and G¹ and G² are 3-methyl-2-butenyl;
G³ is not hydrogen in the case where R¹ and R² are methyl, R³ and R⁴ are t-butyldimethylsilyl (TBS), G¹ is 3-methyl-2-butenyl, and G² is hydrogen;
G³ is not hydrogen in the case where R¹ is hydrogen, R² is methyl, R³ is hydrogen, R⁴ is methyl, G¹ is hydrogen, and G² is 3-methyl-2-butenyl;
G³ is not hydrogen in the case where R¹ and R² are methyl, R³ and R⁴ are acetyl, and G¹ and G² are hydrogen;
G³ is not hydrogen in the case where R¹ and R² are methyl, and R³, R⁴, G¹ and G² are hydrogen;
G³ is not hydrogen in the case where R¹ is benzyl, R² is methyl, R³ is benzyl, R⁴ is methyl, and G¹ and G² are hydrogen;
G³ is not hydrogen in the case where R¹ is hydrogen, R² is methyl, R³ is hydrogen, R⁴ is methyl, and G¹ and G² are hydrogen;
G³ is not hydrogen in the case where R¹, R², R³ and R⁴ are methyl, and G¹ and G² are hydrogen; and
G³ is not hydrogen in the case where R¹ is acetyl, R² is methyl, R³ is acetyl, R⁴ is methyl, and G¹ and G² are hydrogen].

More preferably, the present invention relates to (2) a compound, or a salt thereof, or a hydrate thereof according to (1), wherein R² is methyl.

More preferably, the present invention relates to (3) a compound, or a salt thereof, or a hydrate thereof according to (1), wherein R³ and/or R⁴ is methyl.

More preferably, the present invention relates to (4) a compound, or a salt thereof, or a hydrate thereof according to (1), wherein G¹ and/or G² is an aliphatic hydrocarbon group which may be substituted with a hydroxyl group, an alkoxy group, an alkenyloxy group, or an acyl group.

Alternatively, the present invention relates to (5) a composition having immunoactivating action comprising a compound represented by general formula (II), or a salt thereof, or a hydrate thereof: 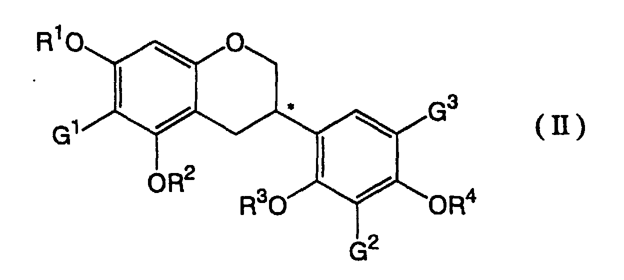 [where R¹, R², R³, and R⁴ may be identical with or different from one another and may each be hydrogen, an alkyl group which may be substituted, an alkenyl group which may be substituted, an alkynyl group which may be substituted, an acyl group which may be substituted, an aralkyl group which may be substituted, or a tri-substituted silyl group: G¹, G², and G³ may be identical with or different from one another and may each be a hydrogen, an acyl group which may be substituted, or an aliphatic hydrocarbon group which may be substituted; symbol (*) indicates the presence of an asymmetric carbon atom, meaning an R form, an S form, or a mixture thereof].

Preferably, the present invention relates to (6) a composition having a bone marrow cellular metabolism promoting action comprising a compound represented by general formula (II), or a salt thereof, or a hydrate thereof according to (5).

Preferably, the present invention relates to (7) a composition having a leukocyte growing action comprising a compound represented by general formula (II), or a salt thereof, or a hydrate thereof according to (5).

Preferably, the present invention relates to (8) a composition having a lymphocyte function regulating action comprising a compound represented by general formula (II), or a salt thereof, or a hydrate thereof according to (5).

Preferably, the present invention relates to (9) a composition according to any of (5) to (8), wherein G¹ and/or G² is an aliphatic hydrocarbon group which may be substituted with a hydroxyl group, an alkoxy group, an alkenyloxy group, or an acyl group.

Preferably, the present invention relates to (10) a composition according to any of (5) to (8), wherein G¹ and/or G² is an alkenyl which may be substituted.

Preferably, the present invention relates to (11) a composition according to (10), wherein the alkenyl which may be substituted is 3-methyl-2-butenyl.

Preferably, the present invention relates to (12) a composition according to any of (5) to (11), wherein R² is an alkyl.

Preferably, the present invention relates to (13) a composition according to (12), wherein the alkyl is methyl.

Preferably, the present invention relates to (14) a composition according to any of (5) to (13), wherein R³ and/or R⁴ is an alkyl.

Preferably, the present invention relates to (15) a composition according to (14), wherein the alkyl is methyl.

Preferably, the present invention relates to (16) a composition according to any of (5) to (15), wherein G³ is hydrogen.

Preferably, the present invention relates to (17) a composition according to any of (5) to (16) as a medicine, animal medicine, food, or cosmetic.

In the present invention, an "alkyl group which may be substituted" includes C1 to C20 alkyls which are in a straight chain or branched form. Examples thereof include methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl. C1 to C9 alkyls are preferable. C1 to C6 alkyls are more preferable. Among others, methyl, ethyl, n-propyl, i-propyl, n-butyl, n-pentyl, and n-hexyl are particularly preferable.

Specific examples of preferable substituents for an "alkyl group which may be substituted" include halogens, hydroxyl groups, substituted or unsubstituted lower alkoxy groups, substituted or unsubstituted lower alkenyloxy groups, substituted or unsubstituted lower alkylcarbonyloxy groups, carboxyl groups, substituted or unsubstituted carbamoyl groups, cyano groups, substituted or unsubstituted amino groups, substituted or unsubstituted amidino groups, azido groups, nitro groups, nitroso groups, mercapto groups, substituted or unsubstituted lower alkylthio groups, sulfo groups, substituted or unsubstituted alicyclic hydrocarbon groups which may be saturated or unsaturated, substituted or unsubstituted heterocyclic ring groups, substituted or unsubstituted acyl groups, and tri-substituted silylalkyloxy groups (e.g., 2-(trimethylsilyl)ethoxy groups), and the like.

In the present specification, a given group being "lower" means that the number of carbons in that group is 1 to 10, preferably 1 to 8, and more preferably 1 to 6.

The number of substituents which may be substituted for the hydrogens in the alkyl group as in an "alkyl group which may be substituted" is 1 to 5, and preferably 1 to 3. The positions of the substituents are not particularly limited. Among the aforementioned substituents, halogens, hydroxyl groups, lower alkoxy groups, lower alkenyloxy groups, and acyl groups are preferable.

In the present invention, an "alkenyl group which may be substituted" includes C2 to C12 alkenyls which are in a straight chain or branched form. These may include as many double bonds as possible in all possible positions, and their arrangement around the double bond may take the (E) configuration or the (Z) configuration. Examples thereof include vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, 3,7,11,15-tetramethyl-1-hexadecenyl, and the like. C2 to C8 alkenyls are preferable. C2 to C6 alkenyls are more preferable. Among others, vinyl, allyl, isopropenyl, 1-propenyl, 2-methyl-1-propenyl, 1-butenyl, 2-butenyl, 3-methyl-2-butenyl are preferable.

Specific examples of preferable substituents for an "alkenyl group which may be substituted" include, for example, halogens, hydroxyl groups, substituted or unsubstituted lower alkoxy groups, substituted or unsubstituted lower alkenyloxy groups, substituted or unsubstituted lower alkylcarbonyloxy groups, carboxyl groups, substituted or unsubstituted carbamoyl groups, cyano groups, substituted or unsubstituted amino groups, substituted or unsubstituted amidino groups, azido groups, nitro groups, nitroso groups, mercapto groups, substituted or unsubstituted lower alkylthio groups, sulfo groups, substituted or unsubstituted alicyclic hydrocarbon groups which may be saturated or unsaturated, substituted or unsubstituted monocyclic or condensed polycyclic aryl groups, substituted or unsubstituted heterocyclic ring groups, and substituted or unsubstituted acyl groups.

The number of substituents for an "alkenyl group which may be substituted" is 1 to 5, and preferably 1 to 3. The positions of the substituents are not particularly limited. Among the aforementioned substituents, halogens, hydroxyl groups, lower alkoxy groups, lower alkenyloxy groups, and acyl groups are preferable.

In the present invention, an "alkynyl group which may be substituted" includes C2 to C12 alkynyls which are in a straight chain or branched form. These may include as many triple bonds as possible in all possible positions. Examples thereof include alkynyl groups having 2 to 20 carbons which may include double bonds, e.g., ethynyl, 1-propynyl, 2-propynyl (propargyl), 2-butynyl, and 2-penten-4-ynyl. C2 to C8 alkynyls are preferable. C2 to C6 alkynyls are more preferable.

Specific examples of preferable substituents for an "alkynyl group which may be substituted" include, for example, halogens, hydroxyl groups, substituted or unsubstituted lower alkoxy groups, substituted or unsubstituted lower alkenyloxy groups, substituted or unsubstituted lower alkylcarbonyloxy groups, carboxyl groups, substituted or unsubstituted carbamoyl groups, cyano groups, substituted or unsubstituted amino groups, substituted or unsubstituted amidino groups, azido groups, nitro groups, nitroso groups, mercapto groups, substituted or unsubstituted lower alkylthio groups, sulfo groups, substituted or unsubstituted alicyclic hydrocarbon groups which may be saturated or unsaturated, substituted or unsubstituted monocyclic or condensed polycyclic aryl groups, substituted or unsubstituted heterocyclic ring groups, and substituted or unsubstituted acyl groups.

The number of substituents for an "alkynyl group which may be substituted" is 1 to 5, and preferably 1 to 3. The positions of the substituents are not particularly limited. Among the aforementioned substituents, halogens, hydroxyl groups, lower alkoxy groups, lower alkenyloxy groups, and acyl groups are preferable.

Examples of "acyl groups which may be substituted" include acyl groups or the like derived from carboxylic acids which may be substituted, oxycarboxylic acids which may be substituted, sulfonic acids which may be substituted, sulfinic acids which may be substituted, and the like. Specifically, groups which can be represented by the formula:

R⁹C(O)-, R¹⁰OC(O)-, R¹¹S(O)₂-, R¹²S(O)-

[where R⁹, R¹⁰, R¹¹, and R¹² each represent a hydrocarbon group or heterocyclic ring group which may be substituted], and the like are included. Groups which can be represented by the formula: R⁹C(O)- are preferable.

Examples of "hydrocarbon groups" as in "hydrocarbon groups or heterocyclic ring groups which may be substituted" represented as R⁹, R¹⁰, R¹¹, and R¹² include, for example, acyclic groups such as aliphatic hydrocarbon groups which are in a straight chain or branched form (e.g., alkyl groups, alkenyl groups, and alkynyl groups), cyclic groups such as saturated or unsaturated alicyclic hydrocarbon groups (e.g., cycloalkyl groups, cycloalkenyl groups, and cycloalkadienyl groups), and monocyclic or condensed polycyclic aryl groups.

Examples of alkyl groups, alkenyl groups, and alkynyl groups for the aforementioned "hydrocarbon groups" similarly include those which have been listed with respect to "aliphatic hydrocarbon groups which may be substituted"

Accordingly, more specific examples of "acyl groups" include acetyl, propionyl, butyryl, and benzoyl.

Specific examples of preferable substituents for an "acyl group which may be substituted" include, for example, halogens, hydroxyl groups, substituted or unsubstituted lower alkoxy groups, substituted or unsubstituted lower alkenyloxy groups, substituted or unsubstituted lower alkylcarbonyloxy groups, carboxyl groups, substituted or unsubstituted carbamoyl groups, cyano groups, substituted or unsubstituted amino groups, substituted or unsubstituted amidino groups, azido groups, nitro groups, nitroso groups, mercapto groups, substituted or unsubstituted lower alkylthio groups, sulfo groups, substituted or unsubstituted alicyclic hydrocarbon groups which may be saturated or unsaturated, substituted or unsubstituted monocyclic or condensed polycyclic aryl groups, substituted or unsubstituted heterocyclic ring groups, and substituted or unsubstituted acyl groups.

The number of substituents which may be substituted for the hydrogens in the acyl group as in an "acyl group which may be substituted" is 1 to 5, and preferably 1 to 3. The positions of the substituents are not particularly limited. Among the aforementioned substituents, halogens, hydroxyl groups, lower alkoxy groups, lower alkenyloxy groups, and acyl groups are preferable.

Preferable examples of "acyl groups which may be substituted" include acetyl groups which may be substituted and benzoyl groups which may be substituted. Herein, examples of the substituents which may be substituted for the hydrogens on the benzene ring of a benzoyl group and their substitution positions include, for example, 2-, 3-, or 4-fluoro; 2-, 3-, or 4-chloro; 2-, 3-, or 4-bromo; 2-, 3-, or 4-iodo; 2-, 3-, or 4-methyl; 2,3-, 2,4-, or 2,5-dimethyl; 2,6-, 3,4-, or 3,5-dimethyl; 2,3,4-, 2,3,5-, 2,3,6-, 2,4,5-, 2,4,6-, or 3,4,5-trimethyl; 2-, 3-, or 4-ethyl; 2-, 3-, or 4-propyl; 2-, 3-, or 4-trifluoromethyl; 2-, 3-, or 4-methoxy; 2,3-, 2,4-, 2,5-, 2,6-, 3,4-, or 3,5-dimethoxy; 2,3,4-, 2,3,5-, 2,3,6-, 2,4,5-, 2,4,6-, or 3,4,5-trimethoxy; 2-, 3-, or 4-ethoxy; 2-, 3-, or 4-propoxy; 2-, 3-, or 4-trifluoromethoxy; 2-, 3-, or 4-cyano; 2-, 3-, or 4-nitro; and any possible combinations of these substituents and substitution positions.

Examples of the aryl moiety of an "aralkyl group which may be substituted" similarly include those which have been listed with respect to "substituted or unsubstituted monocyclic or condensed polycyclic aryl groups" as substituents for "aliphatic hydrocarbon groups which may be substituted". Examples of its alkyl moiety similarly include those which have been listed with respect to the alkyl groups for "aliphatic hydrocarbon groups". Specific examples of aralkyl groups include C7 to C20 aralkyl groups such as benzyl, 1-phenylethyl, 2-phenylethyl (phenethyl), 1-phenylpropyl, and the like. Examples of substituents for "aralkyl groups which may be substituted" similarly include those which have been listed with respect to the substituents which may be substituted for the hydrogens on the benzene ring of a benzoyl group.

Specific examples of preferable substituents for an "aralkyl group which may be substituted" include, for example, halogens, hydroxyl groups, substituted or unsubstituted lower alkoxy groups, substituted or unsubstituted lower alkenyloxy groups, substituted or unsubstituted lower alkylcarbonyloxy groups, carboxyl groups, substituted or unsubstituted carbamoyl groups, cyano groups, substituted or unsubstituted amino groups, substituted or unsubstituted amidino groups, azido groups, nitro groups, nitroso groups, mercapto groups, substituted or unsubstituted lower alkylthio groups, sulfo groups, substituted or unsubstituted alicyclic hydrocarbon groups which may be saturated or unsaturated, substituted or unsubstituted monocyclic or condensed polycyclic aryl groups, substituted or unsubstituted heterocyclic ring groups, and substituted or unsubstituted acyl groups.

The number of substituents which may be substituted for the hydrogens in the aralkyl group as in an "aralkyl group which may be substituted" is 1 to 5, and preferably 1 to 3. The positions of the substituents are not particularly limited. Among the aforementioned substituents, hydroxyl groups, lower alkoxy groups, lower alkenyloxy groups, and acyl groups are preferable.

Preferable examples of "aralkyl groups which may be substituted" include benzyl groups which may be substituted, 1-phenylethyl groups which may be substituted, 1-phenylpropyl groups which may be substituted, and 2-phenylethyl groups which may be substituted.

Herein, examples of the substituents for the benzene ring of each of "benzyl groups which may be substituted", "1-phenylethyl groups which may be substituted", "1-phenylpropyl groups which may be substituted", and "2-phenylethyl groups which may be substituted" and their substitution positions include, for example, 2-, 3-, or 4-fluoro; 2-, 3-, or 4-chloro; 2-, 3-, or 4-bromo; 2-, 3-, or 4-iodo; 2-, 3-, or 4-methyl; 2,3-, 2,4-, or 2,5-dimethyl; 2,6-, 3,4-, or 3,5-dimethyl; 2,3,4-, 2,3,5-, 2,3,6-, 2,4,5-, 2,4,6-, or 3,4,5-trimethyl; 2-, 3-, or 4-ethyl; 2-, 3-, or 4-propyl; 2-, 3-, or 4-trifluoromethyl; 2-, 3-, or 4-methoxy; 2,3-, 2,4-, 2,5-, 2,6-, 3,4-, or 3,5-dimethoxy; 2,3,4-, 2,3,5-, 2,3,6-, 2,4,5-, 2,4,6-, or 3,4,5-trimethoxy; 2-, 3-, or 4-ethoxy; 2-, 3-, or 4-propoxy; 2-, 3-, or 4-trifluoromethoxy; 2-, 3-, or 4-cyano; 2-, 3-, or 4-nitro; and any possible combinations of these substituents and substitution positions.

A "tri-substituted silyl group" means a silyl group (-SiH₃) in which all three hydrogens have been replaced. The tri-substituted silyl group is preferably trialkylsilyl, dialkylmonoarylsilyl, or monoalkyldiarylsilyl, which may be substituted. Specific examples of trialkylsilyl include trimethylsilyl, triethylsilyl, and t-butyldimethylsilyl. Examples of monoalkyldiarylsilyl include t-butyldiphenylsilyl.

In the present invention, aliphatic hydrocarbon groups, as in "aliphatic hydrocarbon groups which may be substituted", mean aliphatic hydrocarbon groups which are in a straight chain or branched form (e.g., alkyl groups, alkenyl groups, and alkynyl groups).

Examples of alkyl groups as the aforementioned "aliphatic hydrocarbon groups" include, for example, alkyl groups and the like having 1 to 20 carbons which are in a straight chain or branched form, e.g., methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl. Alkyl groups having 1 to 10 carbons are more preferable. Among others, methyl, ethyl, n-propyl, i-propyl, n-butyl, i-butyl, n-pentyl, 3-methylbutyl, 2,2-dimethylpropyl, n-hexyl, 3-methylbutyl, 4-methylpentyl, n-heptyl, n-octyl, n-nonyl, tetrahydrogeranyl, n-decyl, and the like are included.

Examples of alkenyl groups as the "aliphatic hydrocarbon groups" include, for example, alkenyl groups and the like having 2 to 20 carbons which are in a straight chain or branched form, e.g., vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, and 3,7,11,15-tetramethyl-1-hexadecenyl. Alkenyl groups having 2 to 8 carbons are more preferable. Among others, 2-butenyl, 2-ethyl-1-butenyl, 3-methyl-2-butenyl, 1-pentenyl, and 2-pentenyl are preferable.

Examples of alkynyl groups as the aforementioned "aliphatic hydrocarbon groups" include, for example, alkynyl groups and the like having 2 to 20 carbons which may include double bonds, e.g., ethynyl, 1-propynyl, 2-propynyl (propargyl), 2-butynyl, and 2-penten-4-ynyl. Alkynyl groups having 2 to 8 carbons are more preferable.

Specific examples of preferable substituents for an "aliphatic hydrocarbon group which may be substituted" include, for example, halogens, hydroxyl groups, lower alkoxy groups, lower alkenyloxy groups, lower alkylcarbonyloxy groups, carboxyl groups, substituted or unsubstituted carbamoyl groups, cyano groups, substituted or unsubstituted amino groups, amidino groups, azido groups, nitro groups, nitroso groups, mercapto groups, lower alkylthio groups, sulfo groups, substituted or unsubstituted alicyclic hydrocarbon groups which may be saturated or unsaturated, substituted or unsubstituted monocyclic or condensed polycyclic aryl groups, substituted or unsubstituted heterocyclic ring groups, and acyl groups. The number of substituents for an "aliphatic hydrocarbon group which may be substituted" is 1 to 5, and preferably 1 to 3. The positions of the substituents are not particularly limited. Among the aforementioned substituents, hydroxyl groups, lower alkoxy groups, lower alkenyloxy groups, and acyl groups are preferable.

Examples of a "halogen" as a substituent for an "aliphatic hydrocarbon group which may be substituted" include fluorine, chlorine, bromine, and iodine, for example. The same also applies to the aforementioned "alkyl groups", "alkenyl groups", "alkynyl groups", "acyl groups", and "aralkyl groups" which "may be substituted".

Examples of the "lower alkoxy group" as a substituent for an "aliphatic hydrocarbon group which may be substituted", whose lower alkyl is under the same definition as the above, include alkoxy groups having 1 to 6 carbons, e.g., methoxy, ethoxy, propoxy, isopropoxy, butoxy, isobutoxy, neobutoxy, t-butoxy, pentoxy, and isopentoxy, for example. The same also applies to the aforementioned "alkyl groups", "alkenyl groups", "alkynyl groups", "acyl groups", and "aralkyl groups" which "may be substituted".

Examples of the "lower alkenyloxy group" as a substituent for an "aliphatic hydrocarbon group which may be substituted", whose lower alkenyl is under the same definition as the above, include alkenyloxy groups having 2 to 7 carbons, e.g., vinyloxy, allyloxy, 1-propenyloxy, 2-methyl-1-propenyloxy, 1-butenyloxy, 2-butenyloxy, 3-butenyloxy, 2-ethyl-1-butenyloxy, 3-methyl-2-butenyloxy, 1-pentenyloxy, 2-pentenyloxy, 3-pentenyloxy, 4-pentenyloxy, and 4-methyl-3-pentenyloxy, for example. The same also applies to the aforementioned "alkyl groups", "alkenyl groups", "alkynyl groups", "acyl groups", and "aralkyl groups" which "may be substituted".

Examples of the "lower alkylcarbonyloxy group" as a substituent for an "aliphatic hydrocarbon group which may be substituted", whose lower alkyl is under the same definition as the above, include alkylcarbonyloxy groups having 2 to 7 carbons, e.g., methylcarbonyloxy, ethylcarbonyloxy, propylcarbonyloxy, isopropylcarbonyloxy, butylcarbonyloxy, isobutylcarbonyloxy, t-butylcarbonyloxy, pentylcarbonyloxy, isopentylcarbonyloxy, neopentylcarbonyloxy, t-pentylcarbonyloxy, and hexylcarbonyloxy, for example. The same also applies to the aforementioned "alkyl groups", "alkenyl groups", "alkynyl groups", "acyl groups", and "aralkyl groups" which "may be substituted".

Examples of "substituted carbamoyl groups" as a substituent for an "aliphatic hydrocarbon group which may be substituted" include, for example, N-monolower-alkylcarbamoyl groups, N,N-dilower-alkylcarbamoyl groups, N-hydroxycarbamoyl groups, N-lower-alkoxycarbamoyl groups, N-hydroxy-N-lower-alkylcarbamoyl groups, N-lower-alkoxy-N-lower alkylcarbamoyl groups, N-phenylcarbamoyl groups, and N-substituted phenylcarbamoyl groups. The same also applies to the aforementioned "alkyl groups", "alkenyl groups", "alkynyl groups", "acyl groups", and "aralkyl groups" which "may be substituted".

Examples of the aforementioned "N-monolower-alkylcarbamoyl groups", whose lower alkyl is under the same definition as the above, include N-methylcarbamoyl groups, N-ethylcarbamoyl groups, N-propylcarbamoyl groups, N-isopropylcarbamoyl groups, N-pentylcarbamoyl groups, N-isopentylcarbamoyl groups, N-neopentylcarbamoyl groups, N-t-pentylcarbamoyl groups, N-1-ethylpropylcarbamoyl groups, and N-hexylcarbamoyl groups, for example.

Examples of the aforementioned "N,N-dilower-alkylcarbamoyl groups", whose lower alkyl is under the same definition as the above, include N,N-dimethylcarbamoyl groups, N-ethyl-N-methylcarbamoyl groups, N,N-diethylcarbamoyl groups, N-methyl-N-propylcarbamoyl groups, N-butyl-N-methylcarbamoyl groups, N-butyl-N-ethylcarbamoyl groups, N-butyl-N-propylcarbamoyl groups, N-butyl-N-isopropylcarbamoyl groups, N,N-dibutylcarbamoyl groups, N-ethyl-N-propylcarbamoyl groups, N,N-dipropylcarbamoyl groups, N-isopropyl-N-n-propylcarbamoyl groups, and N-isopropyl-N-methylcarbamoyl groups, for example.

Examples of the aforementioned "N-hydroxy-N-lower-alkylcarbamoyl groups", whose lower alkyl is under the same definition as the above, include N-hydroxy-N-lower-alkylcarbamoyl groups having 2 to 7 carbons, e.g., N-hydroxy-N-methylcarbamoyl groups, N-hydroxy-N-ethylcarbamoyl groups, N-hydroxy-N-propylcarbamoyl groups, N-hydroxy-N-butylcarbamoyl groups, N-hydroxy-N-isopropylcarbamoyl groups, N-hydroxy-N-isobutylcarbamoyl groups, N-hydroxy-N-sec-butylcarbamoyl groups, N-hydroxy-N-t-butylcarbamoyl groups, N-hydroxy-N-pentylcarbamoyl groups, N-hydroxy-N-isopentylcarbamoyl groups, and N-hydroxy-N-neopentylcarbamoyl groups, for example.

Examples of the aforementioned "N-lower-alkoxy-N-lower alkylcarbamoyl groups", whose lower alkyl is under the same definition as the above, include N-lower-alkoxy-N-lower alkylcarbamoyl groups whose total number of carbons is between 3 and 13, e.g., N-methoxy-N-methylcarbamoyl groups, N-methoxy-N-ethylcarbamoyl groups, N-methoxy-N-propylcarbamoyl groups, N-methoxy-N-butylcarbamoyl groups, N-methoxy-N-isopropylcarbamoyl groups, N-methoxy-N-isobutylcarbamoyl groups, N-methoxy-N-sec-butylcarbamoyl groups, N-methoxy-N-t-butylcarbamoyl groups, N-methoxy-N-pentylcarbamoyl groups, N-methoxy-N-isopentylcarbamoyl groups, and N-methoxy-N-neopentylcarbamoyl groups, for example.

Examples of substituents for the aforementioned "N-substituted phenylcarbamoyl groups" include lower alkyl groups, lower alkoxy groups, hydroxyl groups, and the like, which are under the same definition as the above. Specific preferable examples of "N-substituted phenylcarbamayl groups" include, for example, (4-methylphenyl)carbamoyl, (4-ethylphenyl)carbamoyl, (4-hydroxyphenyl)carbamoyl, (4-methoxyphenyl)carbamoyl, (2,3-dihydroxyphenyl)carbamoyl, (2,3-dimethoxyphenyl)carbamoyl, (2,3-dihydroxyphenyl)carbamoyl, (2,4-dimethoxyphenyl)carbamoyl, (2,6-dihydroxyphenyl)carbamoyl, (2,6-dimethoxyphenyl)carbamoyl, (2,4,6-trihydroxyphenyl)carbamoyl, (2,4,6-trimethoxyphenyl)carbamoyl, (2,4-dimethoxy-6-hydroxyphenyl)carbamoyl, (2,6-dimethoxy-4-hydroxyphenyl)carbamoyl, (4,6-dihydroxy-2-methoxyphenyl)carbamoyl, (2,6-dihydroxy-4-methoxyphenyl)carbamoyl, (2,3,4-trimethoxyphenyl)carbamoyl, (2,3-dimethoxy-4-hydroxyphenyl)carbamoyl, (2,4-dimethoxy-3-hydroxyphenyl)carbamoyl, (2,3-dihydroxy-4-methoxyphenyl)carbamoyl, (3,4-dimethoxy-2-hydroxyphenyl)carbamoyl, (2,4-dihydroxy-3-methoxyphenyl)carbamoyl, (2,4-dimethoxy-6-methyiphenyl)carbamoyl, and (2,6-dimethoxy-4-methylphenyl)carbamoyl.

Examples of "substituted amino groups" as a substituent for "aliphatic hydrocarbon groups which may be substituted" include, for example, monolower-alkylamino groups, dilower-alkylamino groups, and lower-alkylcarbonylamino groups. The same also applies to the aforementioned "alkyl groups", "alkenyl groups", "alkynyl groups", "acyl groups", and "aralkyl groups" which "may be substituted".

Examples of the aforementioned "monolower-alkylamino groups", whose lower alkyl is under the same definition as the above, include monolower-alkylamino groups having 1 to 6 carbons, e.g., methylamino, ethylamino, propylamino, isopropylamino, butylamino, isobutylamino, sec-butylamino, t-butylamino, pentylamino, isopentylamino, and hexylamino, for example.

Examples of the aforementioned "dilower-alkylamino groups", whose lower alkyl is under the same definition as the above, include dilower-alkylamino groups whose total number of carbons is between 2 and 20, e.g., dimethylamino, ethylmethylamino, diethylamino, methylpropylamino, ethylpropylamino, isopropylmethylamino, isopropylethylamino, butylmethylamino, butylethylamino, isobutylmethylamino, and isobutylethylamino, for example.

Examples of the aforementioned "lower-alkylcarbonylamino groups", whose lower alkyl is under the same definition as the above, include alkylcarbonylamino groups having 2 to 7 carbons, e.g., methylcarbonylamino, ethylcarbonylamino, propylcarbonylamino, isopropylcarbonylamino, butylcarbonylamino, isobutylcarbonylamino, sec-butylcarbonylamino, t-butylcarbonylamino, pentylcarbonylamino, and isopentylcarbonylamino, for example.

Examples of "lower alkylthio groups" as a substituent for "aliphatic hydrocarbon groups which may be substituted", whose lower alkyl is under the same definition as the above, include lower alkylthio groups having 1 to 6 carbons, e.g., methylthio, ethylthio, propylthio, isopropylthio, butylthio, isobutylthio, neobutylthio, t-butylthio, pentylthio, hexylthio, for example. The same also applies to the aforementioned "alkyl groups", "alkenyl groups", "alkynyl groups", "acyl groups", and "aralkyl groups" which "may be substituted".

Examples of "substituted or unsubstituted alicyclic hydrocarbon groups which may be saturated or unsaturated" as a substituent for "aliphatic hydrocarbon groups which may be substituted" include cycloalkyl groups, cycloalkenyl groups, cycloalkadienyl groups, for example. The same also applies to the aforementioned "alkyl groups", "alkenyl groups", "alkynyl groups", "acyl groups", and "aralkyl groups" which "may be substituted".

Examples of the aforementioned cycloalkyl groups include, for example, cycloalkyl groups having 3 to 20 carbons, e.g., cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl, and adamantyl.

Examples of the aforementioned cycloalkenyl groups include cycloalkenyl groups having 4 to 20 carbons, e.g., 2-cyclopentyl-1-yl, 3-cyclopenten-1-yl, 2-cyclohexen-1-yl, and 3-cyclohexen-1-yl, for example.

Examples of the aforementioned cycloalkadienyl groups include cycloalkadienyl groups having 4 to 20 carbons, e.g., 2,4-cyclopentadien-1-yl, 2,4-cyclohexadien-1-yl, and 2,5-cyclohexadien-1-yl, for example.

Examples of "substituted or unsubstituted monocyclic or condensed polycyclic aryl groups" as a substituent for "aliphatic hydrocarbon groups which may be substituted" include aryl groups having 6 to 20 carbons, e.g., phenyl, indenyl, naphthyl, (1-naphthyl, 2-naphthyl, etc.), anthryl, phenanthryl, acenaphthylenyl, fluorenyl (9-fluorenyl, 1-fluorenyl, etc.), for example. The same also applies to the aforementioned "alkenyl groups", "alkynyl groups", "acyl groups", and "aralkyl groups" which "may be substituted".

The heterocyclic ring groups, as in "substituted or unsubstituted heterocyclic ring groups" as a substituent for "aliphatic hydrocarbon groups which may be substituted", mean heterocyclic ring groups including at least one heteroatom of oxygen, sulfur, or nitrogen as a component atom of the ring system, and preferably are aromatic heterocyclic ring groups, e.g., aromatic monocyclic heterocyclic ring groups, and aromatic condensed heterocyclic groups which are dicyclic or tricyclic. Specific examples of such monocyclic heterocyclic ring groups include, for example, furyl, thienyl, pyrrolyl, oxazolyl, isoxazolyl, thiazolyl, isothiazolyl, imidazolyl, pyrazolyl, 1,2,3-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, and quinolyl. Specific examples of such aromatic condensed heterocyclic groups which are dicyclic or tricyclic include, for example, benzofuranyl, isobenzofuranyl, benzo[b]thienyl, indolyl, isoindolyl, 1H-indazolyl, benzoimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, and 1,2,4-triazolo[4,3-a]pyridazinyl. Among others, heterocyclic ring groups including only oxygen atoms as ring system atoms are more preferable, e.g., furyl, benzo[b]furyl, 2H-pyran-3-yl, isobenzofuran, 2H-chromen-3-yl, xanthenyl, chromanyl, isochromanyl, 2H-furo[3,2-b]pyran, cyclopenta[b]pyran, and 2H-benzopyranyl. The same also applies to the aforementioned "alkyl groups", "alkenyl groups", "alkynyl groups", "acyl groups", and "aralkyl groups" which "may be substituted".

Preferable examples of substituents for the aforementioned "substituted alicyclic hydrocarbon groups which may be saturated or unsaturated", "substituted monocyclic or condensed polycyclic aryl groups", and "substituted heterocyclic ring groups" include, for example, halogens, hydroxyl groups, lower alkyl groups, lower alkyl groups which have been substituted with substituents (hydroxyl groups, lower alkoxy groups or lower alkylcarbonyl groups), lower alkenyl groups, lower alkynyl groups, lower alkoxy groups, lower alkenyloxy groups, lower alkylcarbonyloxy groups, carboxyl groups, lower alkylcarbonyl groups, lower alkoxycarbonyl groups, carbamoyl groups, lower alkylcarbamoyl groups, N-dilower-alkylcarbamoyl groups, N-hydroxycarbamoyl groups, N-hydroxy-N-lower-alkylcarbamoyl groups, N-phenylcarbamoyl groups, N-substituted phenylcarbamoyl groups, cyano groups, amino groups, monolower-alkylamino groups, dilower-alkylamino groups, lower alkylcarbonylamino groups, amidino groups, azido groups, nitro groups, nitroso groups, mercapto groups, lower alkylthio groups, sulfo groups, alicyclic hydrocarbon groups which may be saturated or unsaturated, monocyclic or condensed polycyclic aryl groups, and heterocyclic ring groups. The number of substituents, if any, is 1 to 3, and preferably one. The positions of the substituents are not particularly limited. Among the aforementioned substituents, hydroxyl groups, lower alkyl groups, lower alkoxy groups, lower alkenyloxy groups, lower alkylcarbonyloxy groups, or lower alkyl groups which have been substituted with hydroxyl groups, lower alkoxy groups, or lower alkylcarbonyl groups, are preferable.

Preferable examples of the aforementioned "lower alkylcarbonyl groups", whose lower alkyl is under the same definition as the above, include alkanoyl groups having 2 to 6 carbons, e.g., acetyl, propionyl, butyryl, isobutyryl, valeryl, isovaleryl, pivaloyl, and hexanoyl, for example.

Preferable examples of the aforementioned "lower alkoxycarbonyl groups", whose lower alkoxy is under the same definition as the above, include alkoxycarbonyl groups having 2 to 7 carbons, e.g., methoxycarbonyl, ethoxycarbonyl, n-propoxycarbonyl, and n-butoxycarbonyl.

The other substituents have meanings as described in the terminology of substituents for "aliphatic hydrocarbon groups which may be substituted".

Examples of an "acyl group" as a substituent for an "aliphatic hydrocarbon group which may be substituted" include acyl groups or the like derived from carboxylic acids which may be substituted, oxycarboxylic acids which may be substituted, sulfonic acids which may be substituted, sulfinic acids which may be substituted, and the like. Specifically, groups which can be represented by the formula:

R⁵C(O)-, R⁶OC(O)-, R⁷S(O)₂-, R⁸S(O)-

[where R⁵, R⁶, R⁷, and R⁸ each represent a hydrocarbon group or heterocyclic ring group which may be substituted], and the like are included. Groups which can be represented by the formula: R⁵C(O)- are preferable.

Examples of "hydrocarbon groups" as in "hydrocarbon groups or heterocyclic ring groups which may be substituted" represented as R⁵, R⁶, R⁷ and R⁸ include, for example, acyclic groups such as aliphatic hydrocarbon groups which are in a straight chain or branched form (e.g., alkyl groups, alkenyl groups, and alkynyl groups), cyclic groups such as saturated or unsaturated alicyclic hydrocarbon groups (e.g., cycloalkyl groups, cycloalkenyl groups, and cycloalkadienyl groups), and monocyclic or condensed polycyclic aryl groups.

Examples of alkyl groups, alkenyl groups, and alkynyl groups for the aforementioned "hydrocarbon groups" similarly include those which have been listed with respect to "aliphatic hydrocarbon groups which may be substituted".

Examples of cycloalkyl groups, cycloalkenyl groups, and cycloalkadienyl groups for the aforementioned "hydrocarbon groups" similarly include those which have been listed with respect to the substituents for "aliphatic hydrocarbon groups which may be substituted".

Examples of aryl groups for the aforementioned "hydrocarbon groups" include aryl groups having 6 to 20 carbons, e.g., phenyl, indenyl, naphthyl, (1-naphthyl, 2-naphthyl, etc.), anthryl, phenanthryl, acenaphthylenyl, fluorenyl (9-fluorenyl, 1-fluorenyl, etc.), for example.

The "heterocyclic ring groups", as in the aforementioned "hydrocarbon groups or heterocyclic ring groups which may be substituted", mean heterocydlic ring groups including at least one heteroatom of oxygen, sulfur, or nitrogen as a component atom of the ring system, and preferably are aromatic heterocyclic ring groups, e.g., aromatic monocyclic heterocyclic ring groups, and aromatic condensed heterocyclic groups which are dicyclic or tricyclic. Specific examples of such monocyclic heterocyclic ring groups include, for example, furyl, thienyl, pyrrolyl, oxazolyl, isoxazolyl, thiazolyl, isothiazolyl, imidazolyl, pyrazolyl, 1,2,3-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, and quinolyl. Specific examples of such aromatic condensed heterocyclic groups which are dicyclic or tricyclic include, for example, benzofuranyl, isobenzofuranyl, benzo[b]thienyl, indolyl, isoindolyl, 1H-indazolyl, benzoimidazolyl, benzoxazolyl, 1,2-benzoisoxazolyl, benzothiazolyl, 1,2-benzoisothiazolyl, 1H-benzotriazolyl, quinolyl, isoquinolyl, cinnolinyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, and 1,2,4-triazolo[4,3-a]pyridazinyl. Among others, heterocyclic ring groups including only oxygen atoms as ring system atoms are more preferable, e.g., furyl, benzo[b]furyl, 2H-pyran-3-yl, isobenzofuran, 2H-chromen-3-yl, xanthenyl, chromanyl, isochromanyl, 2H-furo[3,2-b]pyran, cyclopenta[b]pyran, and 2H-benzopyranyl.

Examples of substituents for the aforementioned "hydrocarbon groups or heterocyclic groups which may be substituted" similarly include those which have been listed with respect to the substituents for "substituted alicyclic hydrocarbon groups which may be saturated or unsaturated", "substituted monocyclic or condensed polycyclic aryl groups" and "substituted heterocyclic groups" which are substituents for "aliphatic hydrocarbon groups which may be substituted".

Preferable specific examples of "acyl groups" as substituents for "aliphatic hydrocarbon groups which may be substituted" include alkanoyl groups having 1 to 6 carbons, e.g., formyl, acetyl, propionyl, butyryl, isobutyryl, valeryl, isovaleryl, pivaloyl, and hexanoyl, benzoyl, 2,4-dihydroxyphenylcarbonyl, and 2,4-dihydroxy-3-(3-methyl-2-butenyl)phenylcarbonyl, for example.

Specific preferable examples of "aliphatic hydrocarbon groups which may be substituted" include, in addition to the below-listed particularly preferable specific examples, isopentenyl, 2-hydroxy-3-methylbutyl, 3-hydroxy-2-phenylpropyl, 3-(2,4-dihydroxyphenylcarbonyl)butyl, 2-methoxy-3-methylbutyl, 3-methoxy-2-phenylpropyl, 2-(2-butenyloxy)-3-methylbutyl, 3-(2,4-dihydroxyphenyl)propyl, 3-(2,4-dimethoxyphenylcarbonyl)butyl, 2-hydroxybutyl, 2-hydroxy-3-methylpentyl, 2-methoxybutyl, 2-methoxy-3-methylpentyl, for example.

Specific particularly preferable examples of "aliphatic hydrocarbon groups which may be substituted" include, for example, methyl, ethyl, n-propyl, i-propyl, n-butyl, i-butyl, n-pentyl, 3-methylbutyl, 2,2-dimethylpropyl, n-hexyl, 3-methylbutyl, 4-methylpentyl, n-heptyl, n-octyl, n-nonyl, tetrahydrogeranyl, n-decyl, n-pentadecyl, trifluoromethyl, 2-propenyl, 2-butenyl, 3-butenyl, 2-ethyl-1-butenyl, 3-methyl-2-butenyl, 1-pentenyl, 2-pentenyl, 4-pentenyl, geranyl, 2-propynyl (propargyl), and 2-butynyl.

The aforementioned definitions of the substituents R¹, R², R³, R⁴, G¹, G², and G³ are identical for the case of being used in general formula (I) and the case of being used in general formula (II). However, the combinations of these substituents have differences as respectively described for general formula (I) and general formula (II).

That is, the combinations of the substituents R¹, R³, R⁴, G¹ and G² in the isoflavan derivatives of the present invention are limited in accordance with the description following "with the provisos that" for general formula (I).

"Symbol (*)" indicates the presence of an asymmetric carbon atom, meaning an R form, an S form (which are stereoisomers), or a mixture thereof. Among others, stereoisomers such that the 3 position of 2H-1-benzopyran takes an R position are preferable.

Preferable combinations of the substituents R¹, R², R³, R³, R⁴, G¹ and G² of isoflavan derivatives for better achieving the effects of the present invention include the following, for example:
(1) combinations in which G¹ and G² may be identical with or different from one another and may each be an aliphatic hydrocarbon group which may be substituted with a hydroxyl group, a lower alkoxy group, a lower alkenyloxy group, or an acyl group; G³ is hydrogen; and R¹, R², R³, and R⁴ may be identical with or different from one another and may each be hydrogen, a lower alkyl group, a lower alkenyl group, an acyl group, or an aralkyl group;
(2) combinations in which G¹ and G² may be identical with or different from one another and may each be an alkenyl group; G³ is hydrogen; and R¹, R², R³, and R⁴ may be identical with or different from one another and may each be hydrogen, a lower alkyl group, or a lower alkenyl group;
(3) combinations in which G¹ and G² may be identical with or different from one another and may each be hydrogen or an unsubstituted aliphatic hydrocarbon group; G³ is hydrogen; and R¹, R², R³, and R⁴ may be identical with or different from one another and may each be hydrogen, a lower alkyl group, a lower alkenyl group, an acyl group, or an aralkyl group;
(4) combinations in which G¹ and G² may be identical with or different from one another and may each be hydrogen or an unsubstituted aliphatic hydrocarbon group; G³ is hydrogen; and R¹, R², R³, and R⁴ may be identical with or different from one another and may each be hydrogen, a lower alkyl group, or a lower alkenyl group;
(5) combinations in which G¹ and G² may be identical with or different from one another and may each be an aliphatic hydrocarbon group which may be substituted with a hydroxyl group, a lower alkoxy group, or a lower alkenyloxy group; G³ is hydrogen; and R¹, R², R³, and R⁴ may be identical with or different from one another and may each be hydrogen, a lower alkyl group, or a lower alkenyl group; and
(6) combinations in which G¹ and G² may be identical with or different from one another and may each be an unsubstituted aliphatic hydrocarbon group; G³ is hydrogen; and R¹, R², R³, and R⁴ may be identical with or different from one another and may each be hydrogen or a lower alkyl group.

The following eight compounds, which are described in the Tables and in the Examples, are more preferable:
4-[3,4-dihydro-7-hydroxy-5-methoxy-6-(3-methyl-2-butenyl)-2H-1-benzopyran-3-yl]-2-(3-methyl-2-butenyl)-1,3-benzenediol (licoricidin (Compound No. A-1): Example 1);
4-[3,4-dihydro-5,7-dimethoxy-6-(3-methyl-2-butenyl)-2H-1-benzopyran-3-yl]-2-(3-methyl-2-butenyl)-1,3-benzenediol (licorisoflavan A (Compound No. A-2): Example 2);
3-(2,4-dimethoxy-3-(3-methyl-2-butenyl)phenyl)-3,4-dihydro-5,7-dimethoxy-6-(3-methyl-2-butenyl)-2H-1-benzopyran (Compound A (Compound No. A-3): Example 3);
3-(2-hydroxy-4-methoxy-3-(3-methyl-2-butenyl)-phenyl)-3,4-dihydro-5,7-dimethoxy-6-(3-methyl-2-butenyl)-2H-1-benzopyran (Compound B (Compound No. A-4): Example 4);
3-(4-hydroxy-2-methoxy-3-(3-methyl-2-butenyl)-phenyl)-3,4-dihydro-5,7-dimethoxy-6-(3-methyl-2-butenyl)-2H-1-benzopyran (Compound C (Compound No. A-5): Example 5);
3-(2,4-dimethoxy-3-(3-methyl-2-butenyl)phenyl)-3,4-dihydro-5-hydroxy-7-methoxy-6-(3-methyl-2- butenyl)-2H-1-benzopyran (Compound D (Compound No. A-6): Example 6);
3-(2-hydroxy-4-methoxy-3-(3-methyl-2-butenyl)-phenyl)-3,4-dihydro-5-methoxy-7-hydroxy-6-(3-methyl-2-butenyl)-2H-1-benzopyran (Compound E (Compound No. A-7): Example 7); and
3-(4-hydroxy-2-methoxy-3-(3-methyl-2-butenyl)-phenyl)-3,4-dihydro-5-methoxy-7-hydroxy-6-(3-methyl-2-butenyl)-2H-1-benzopyran (Compound F (Compound No. A-8): Example 8).

The compound represented by general formula (I) and the uses, e.g., immunoactivating actions, of the compound represented by general formula (II) are novel. Preferable examples thereof include compounds in which G¹ to G³ take one of the combinations A001 to A168 shown in Table 1 below and in which R¹ to R⁴ take one of the combinations B001 to J453 shown in Table 2 below, among others. Referring to the symbols used in the tables, Compound No. is indicated in the column captioned as "No."; MOM represents methoxymethyl: TBS represents t-butyldimethylsilyl; and SEM represents 2-(trimethylsilyl)ethoxymethyl.

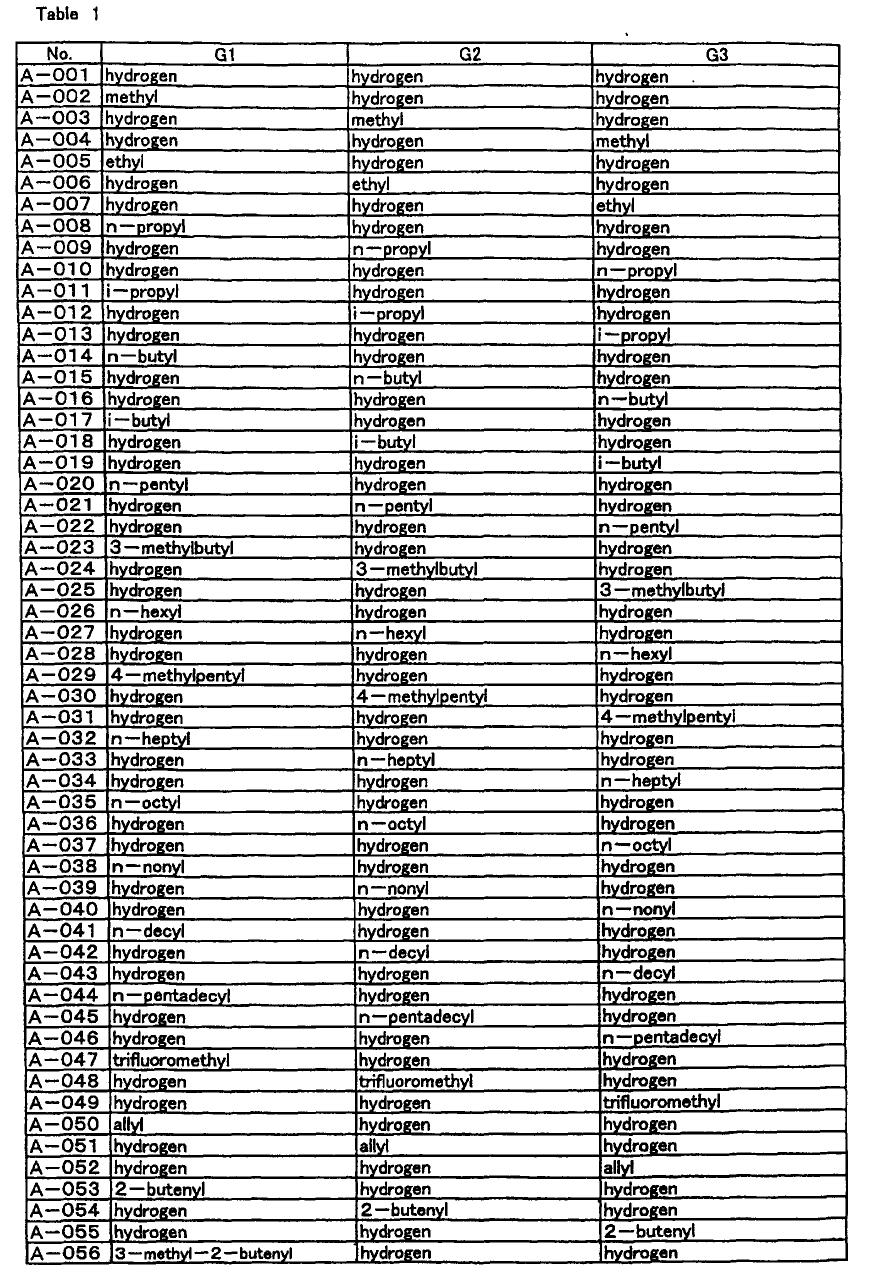

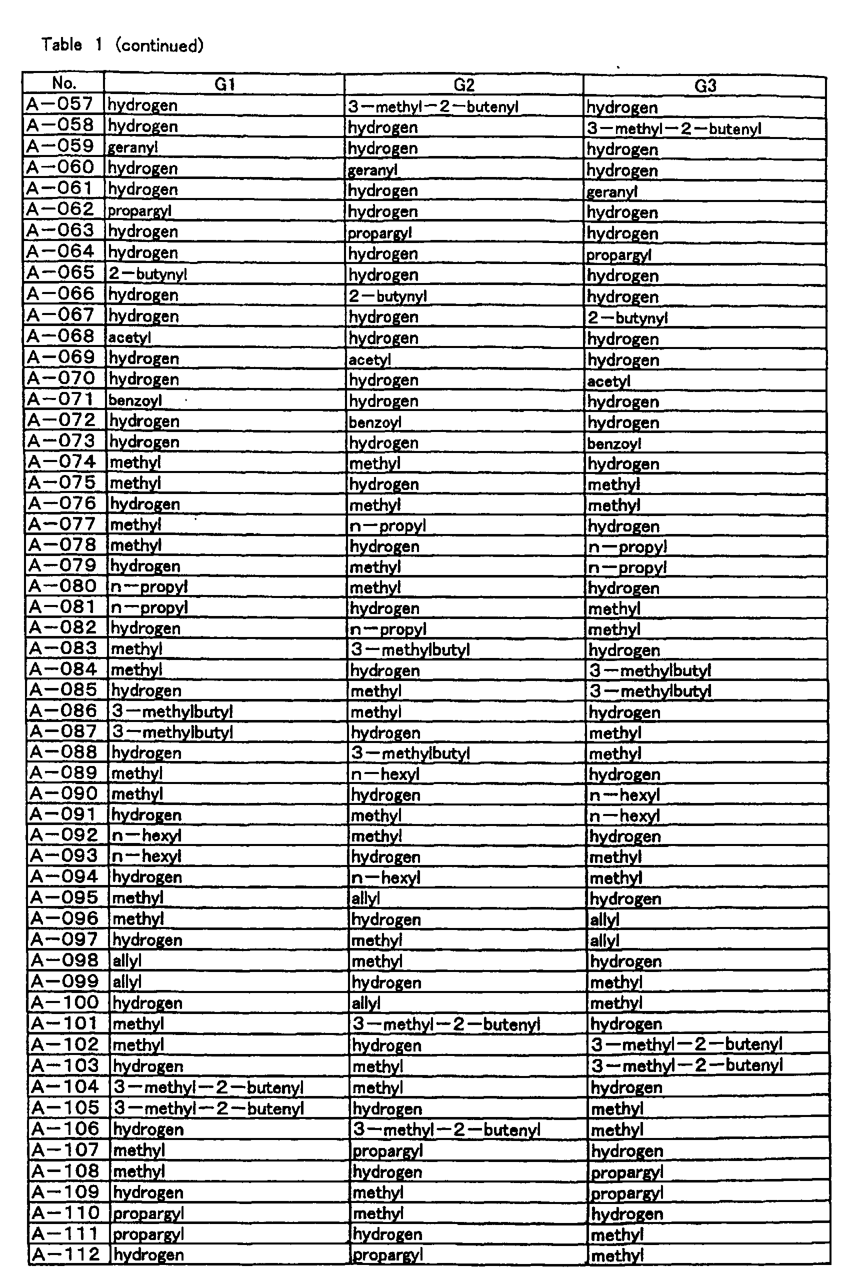

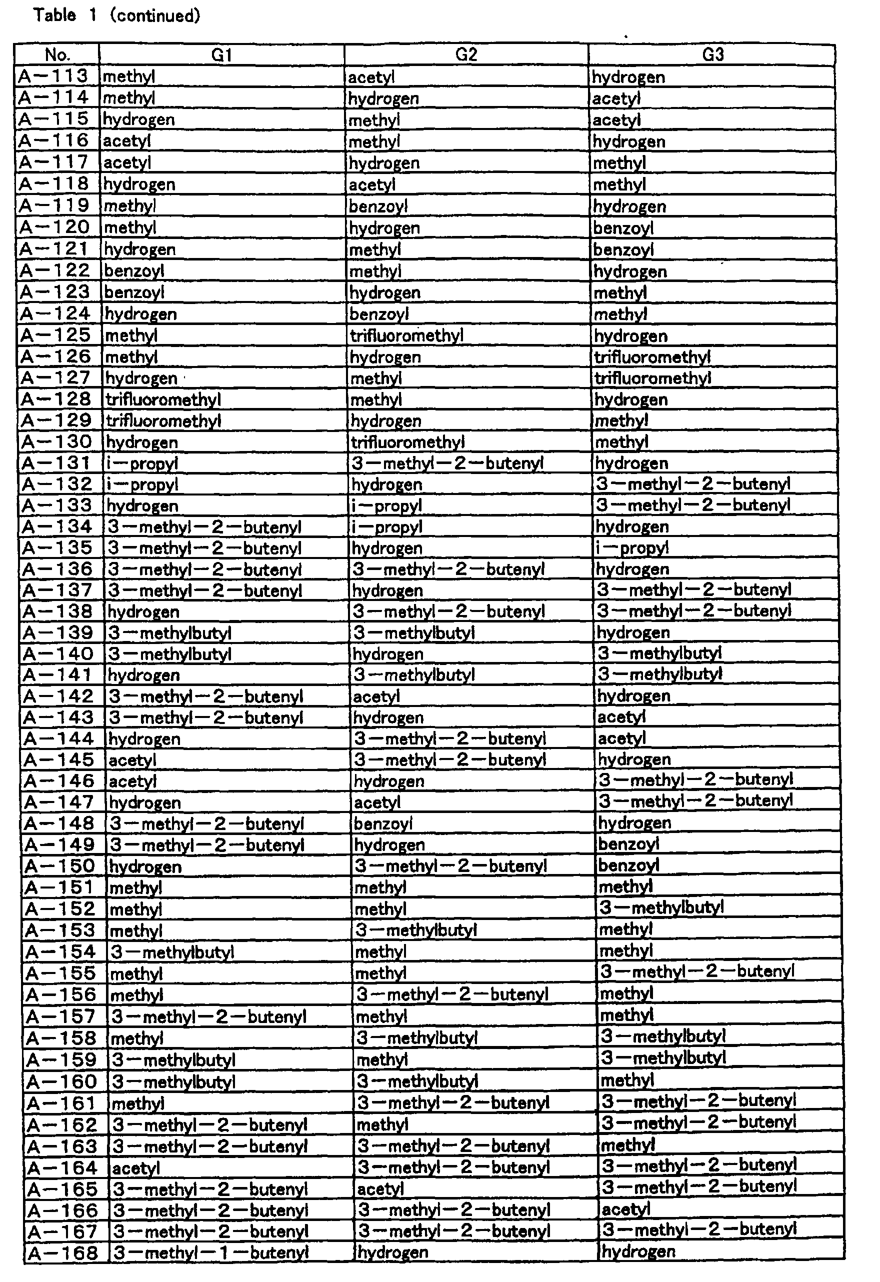

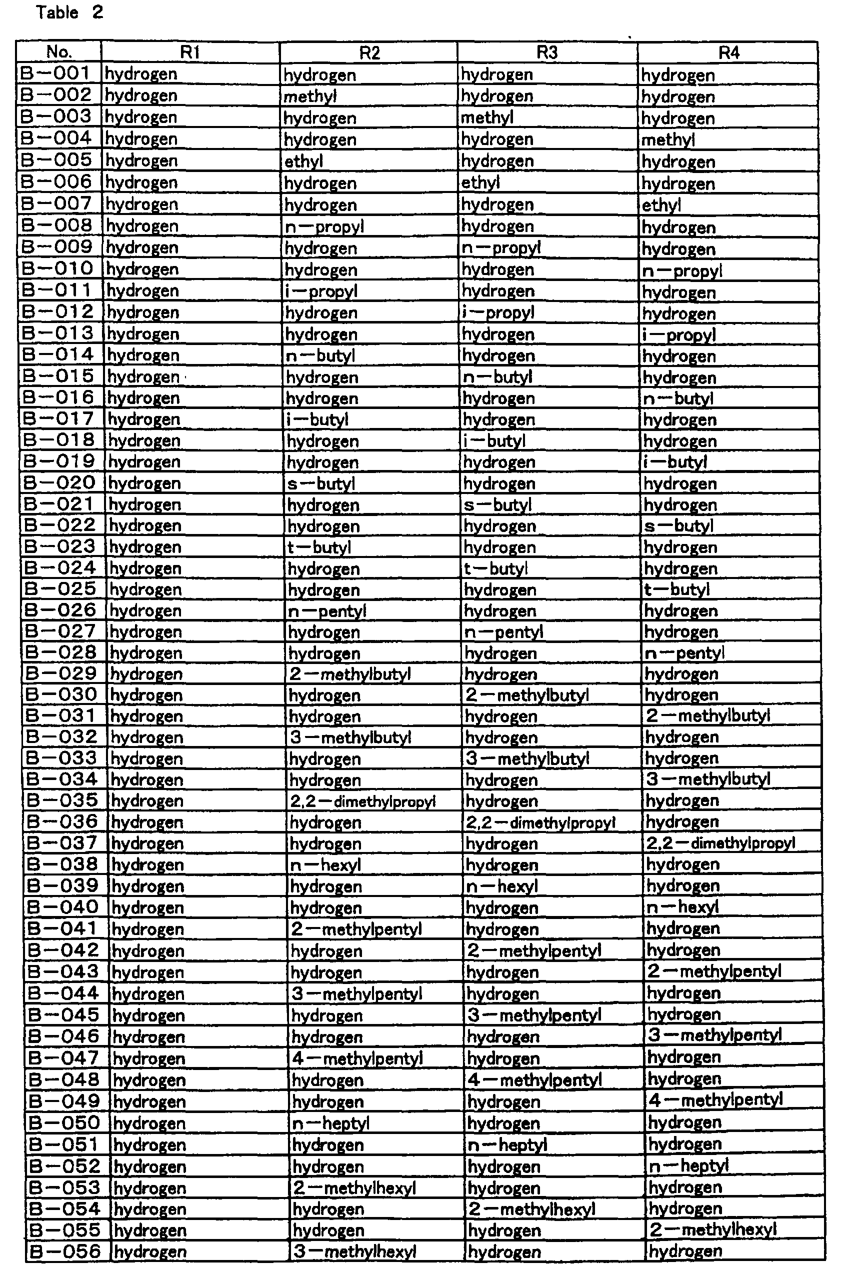

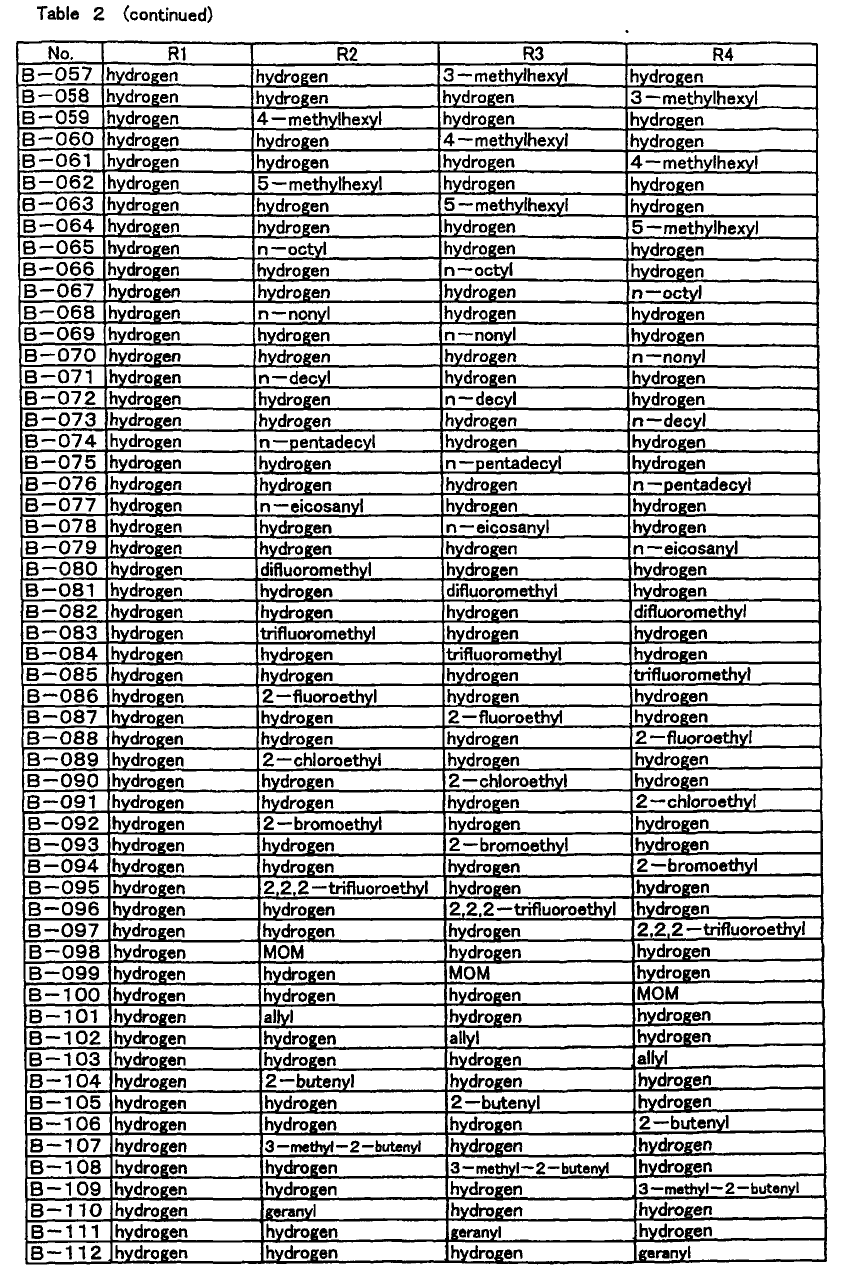

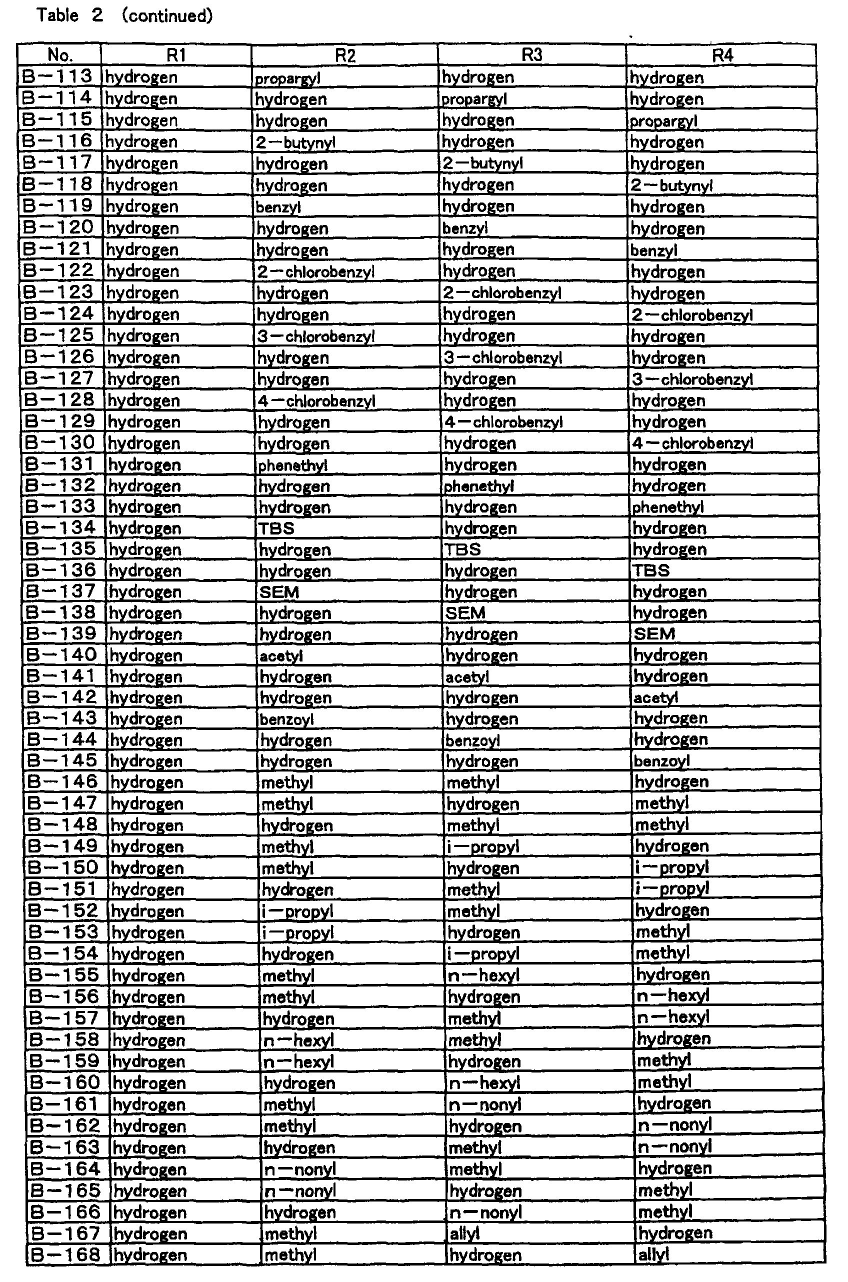

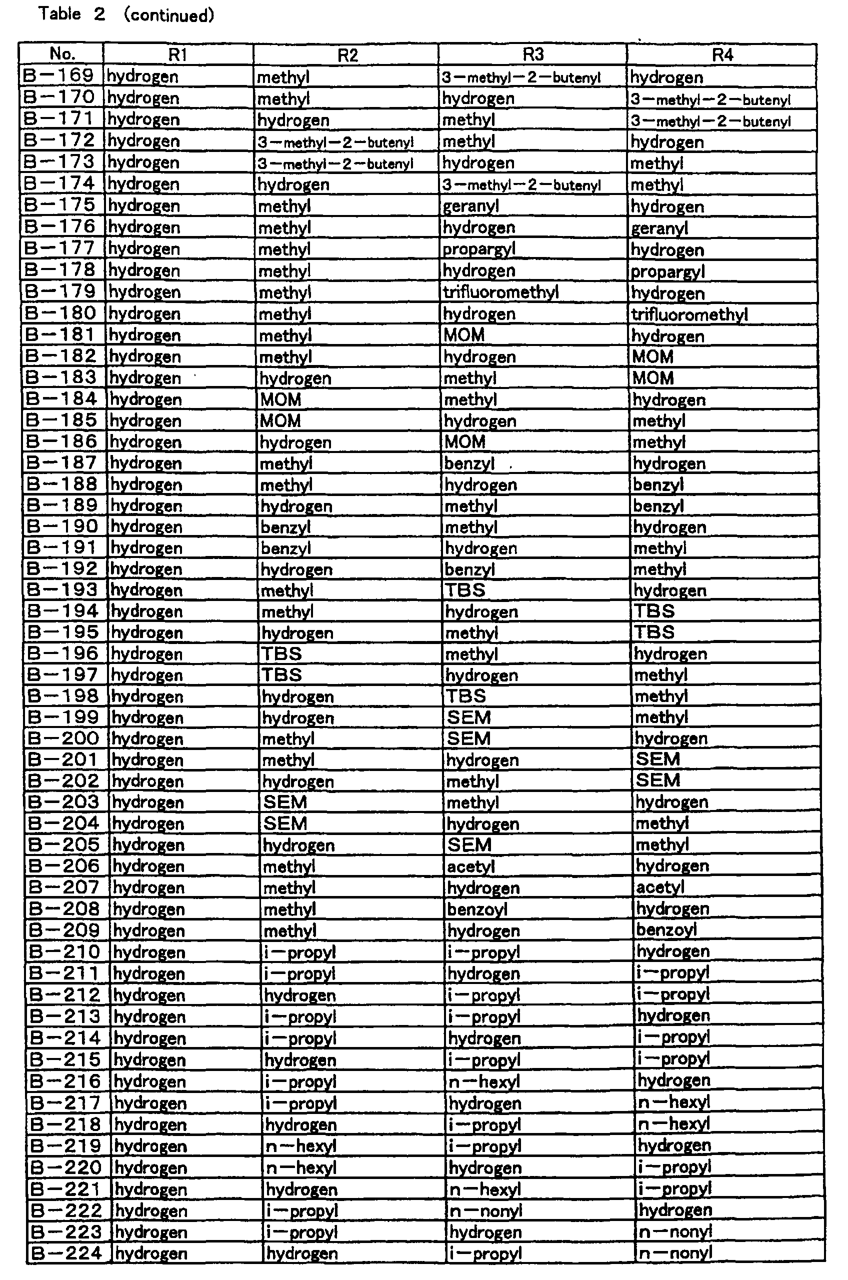

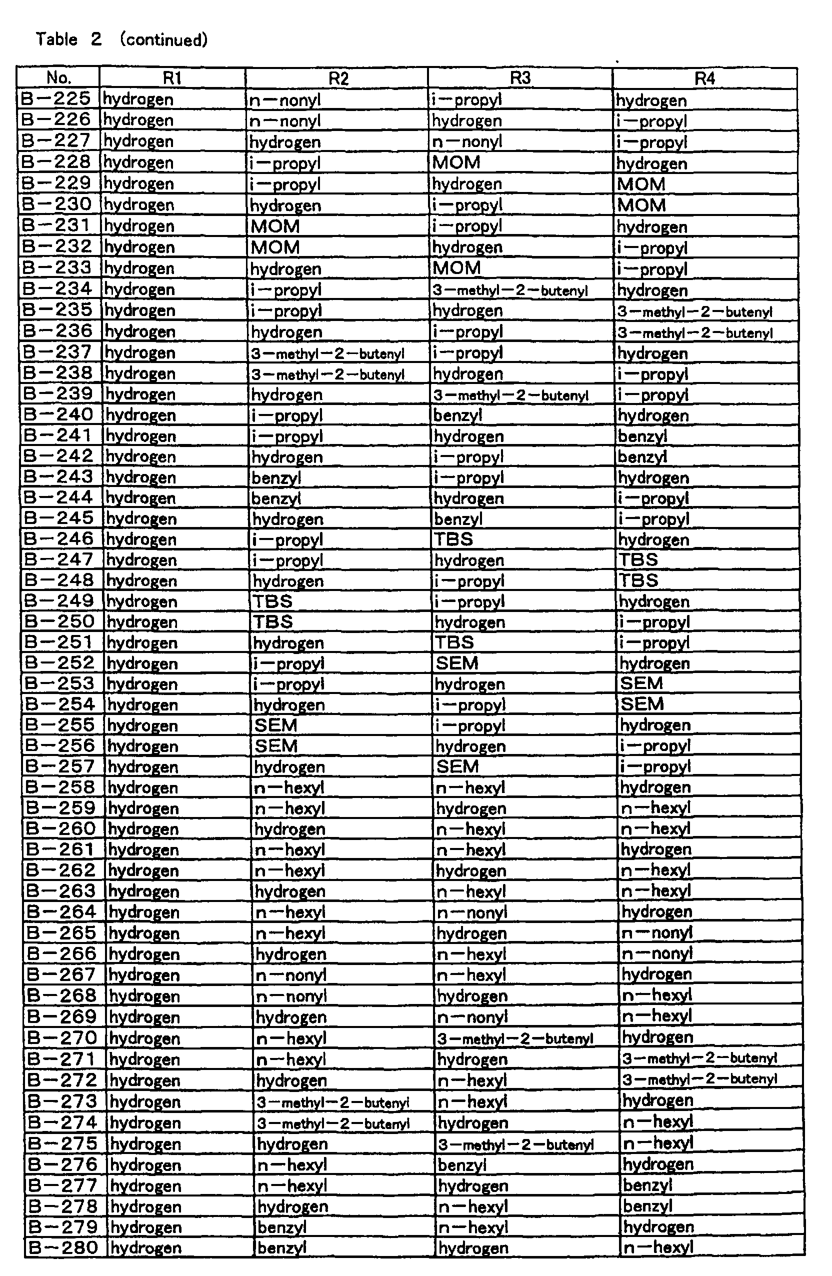

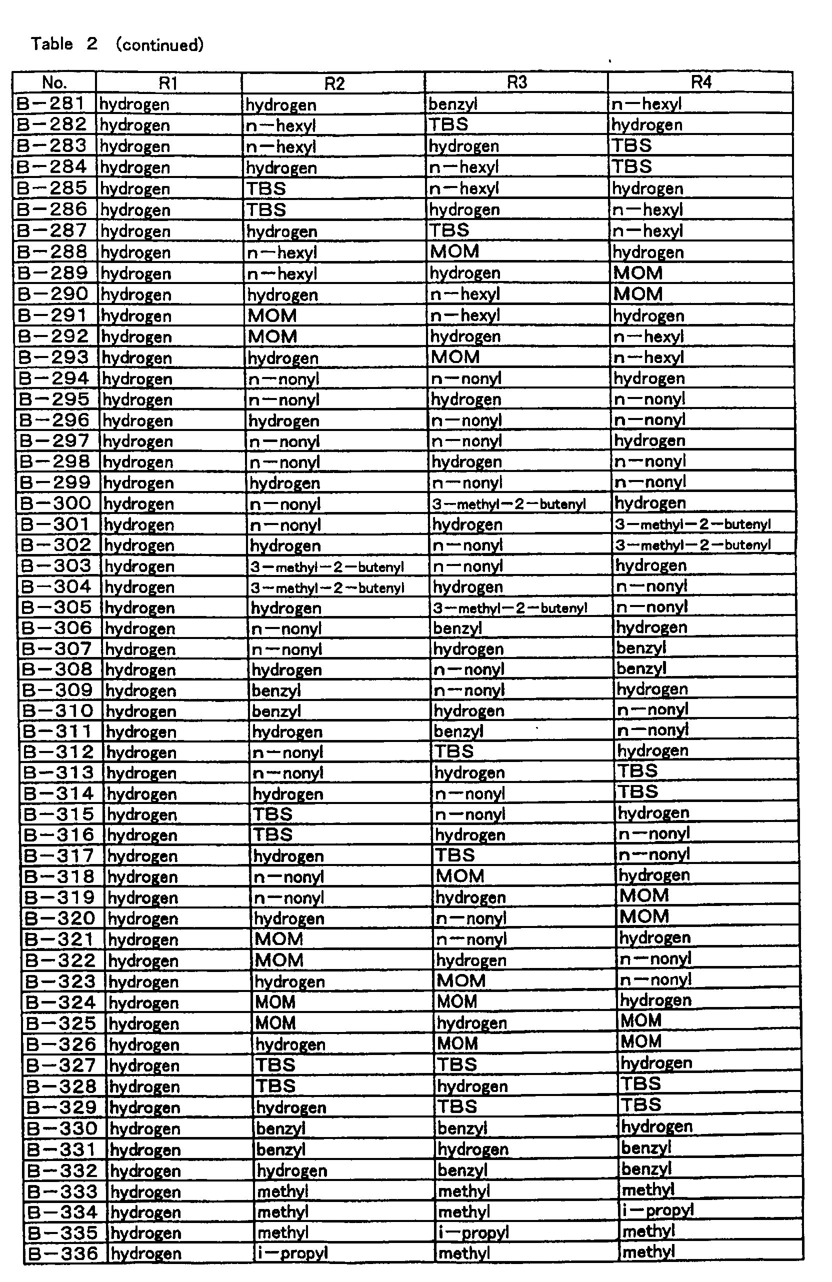

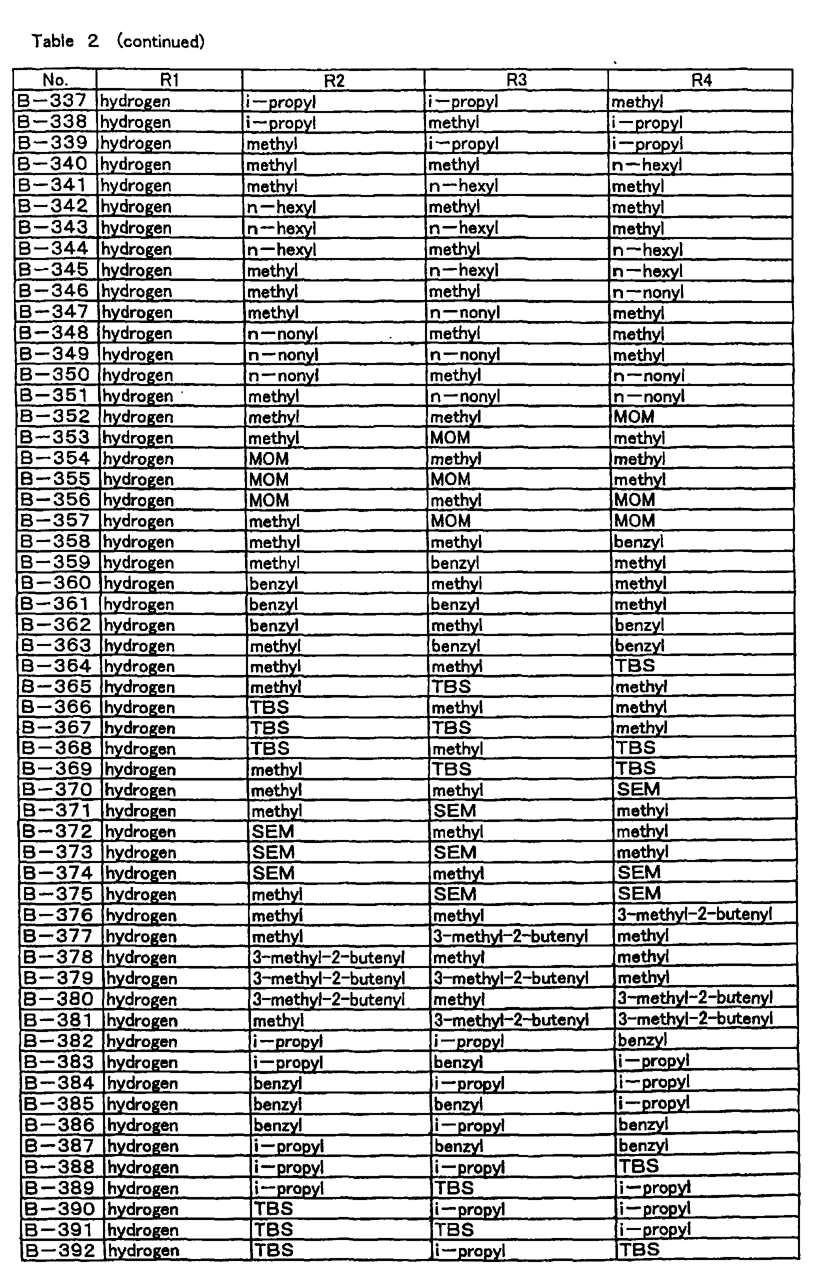

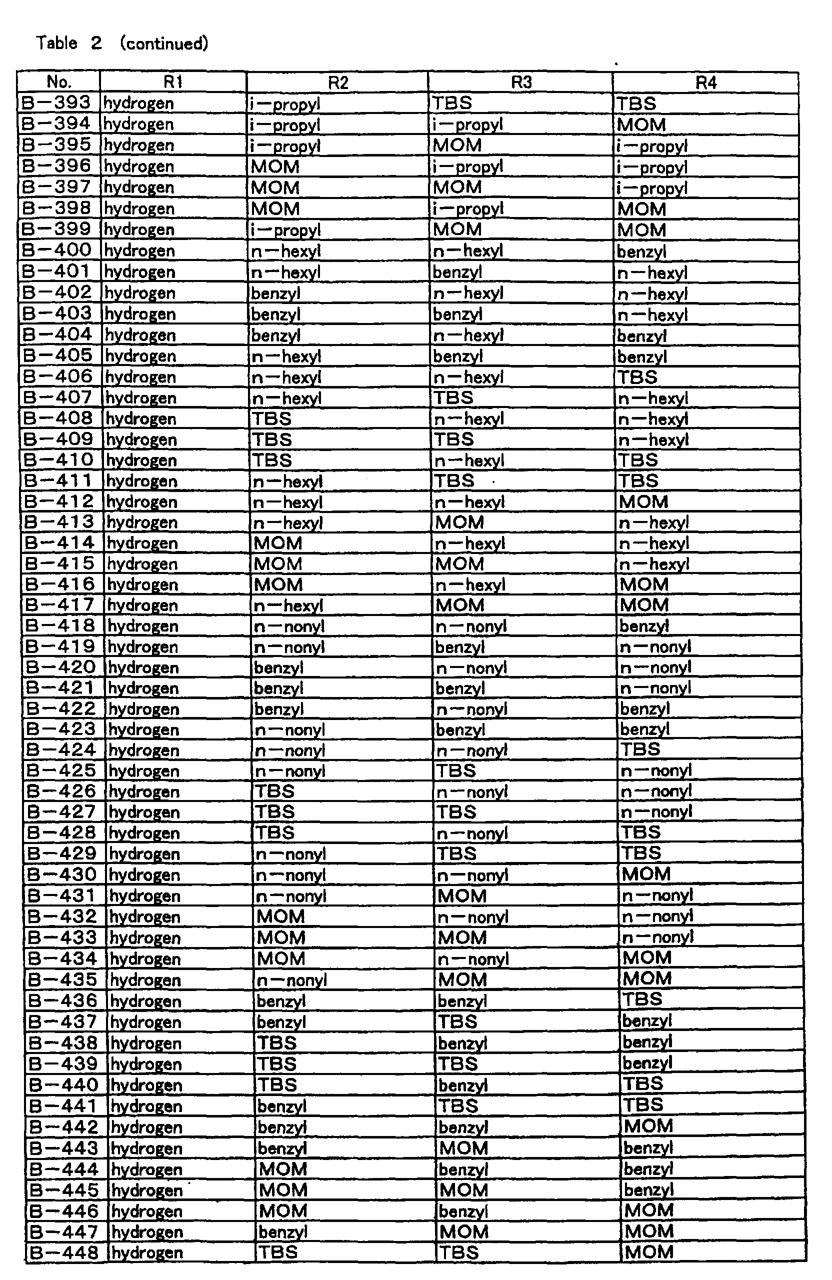

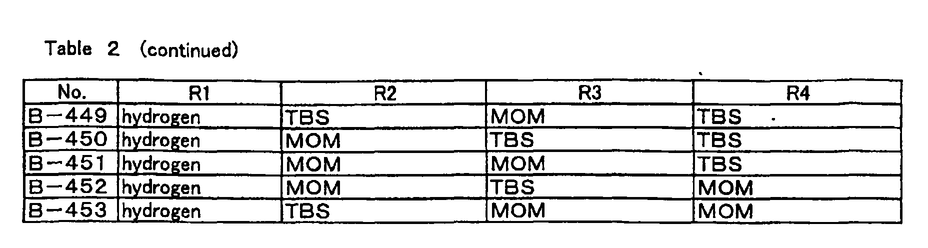

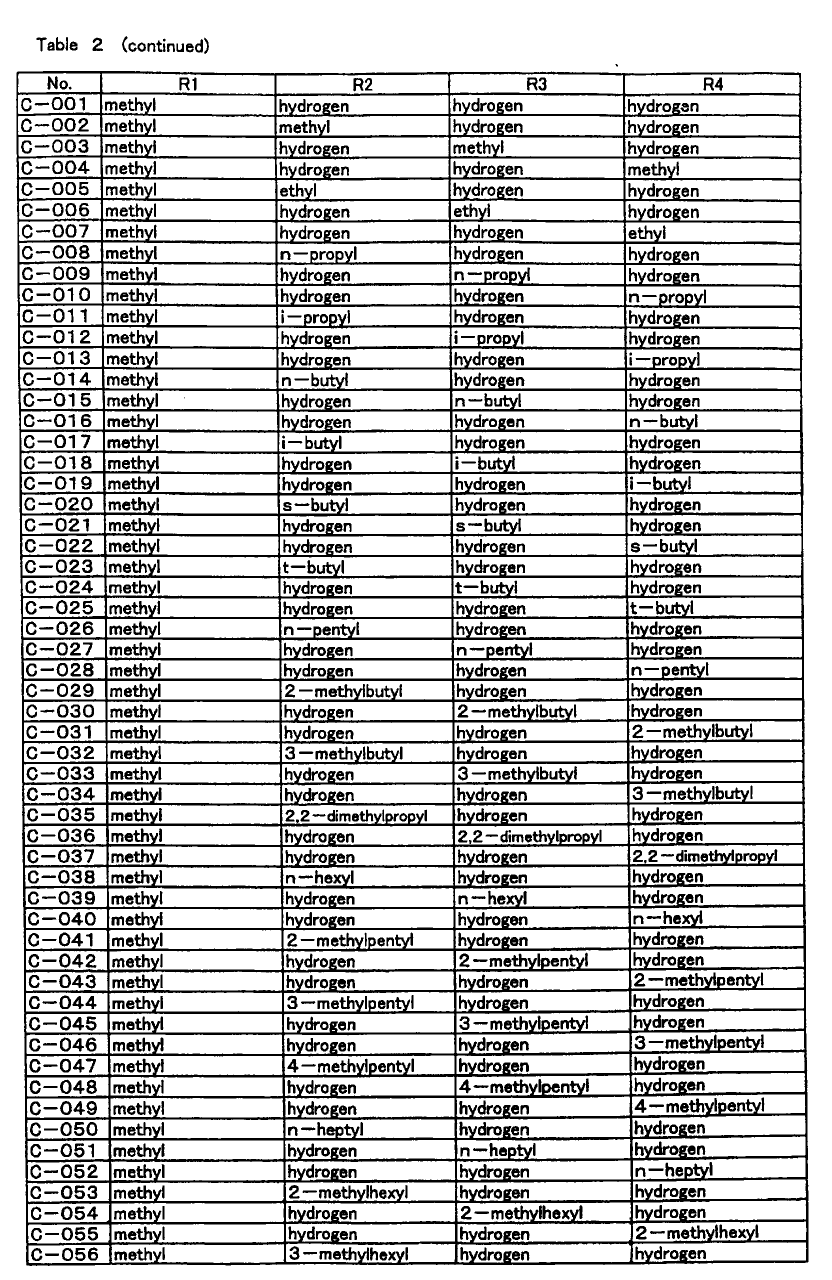

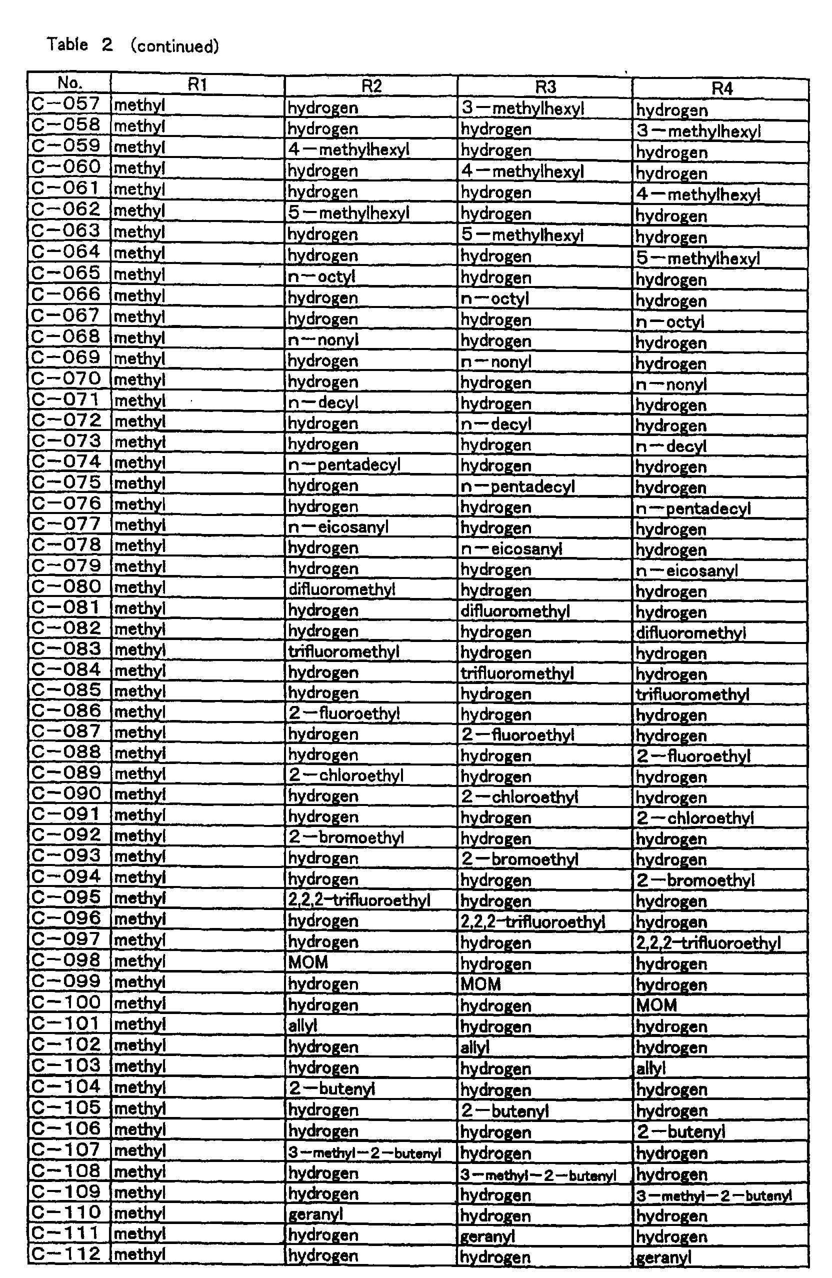

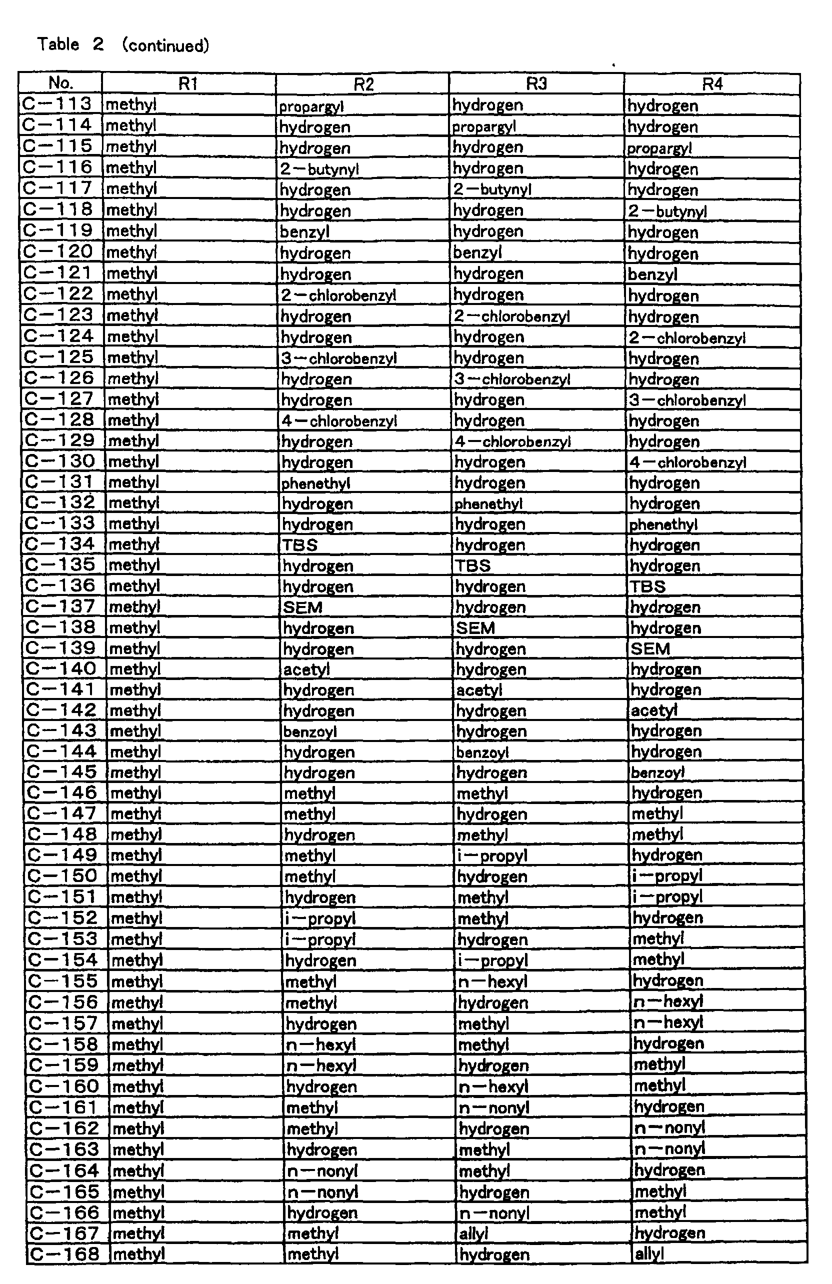

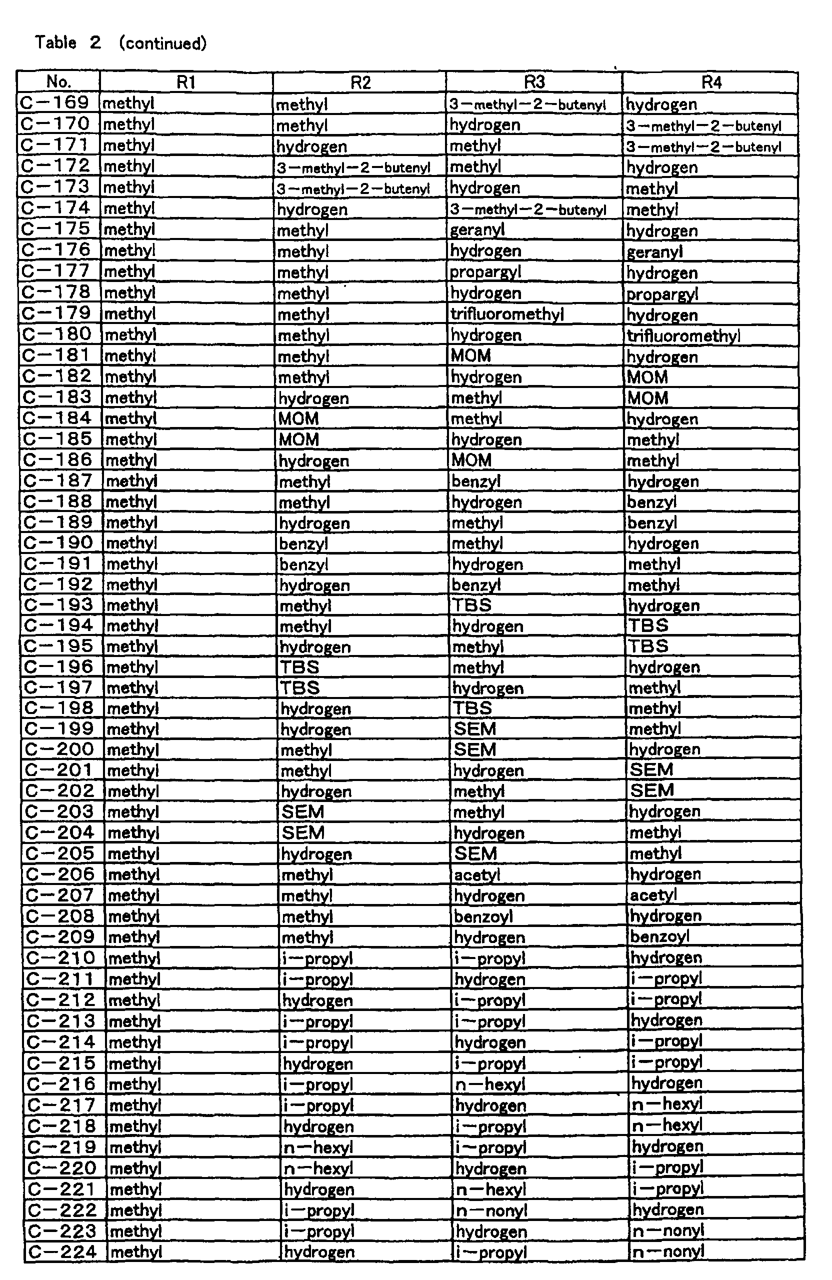

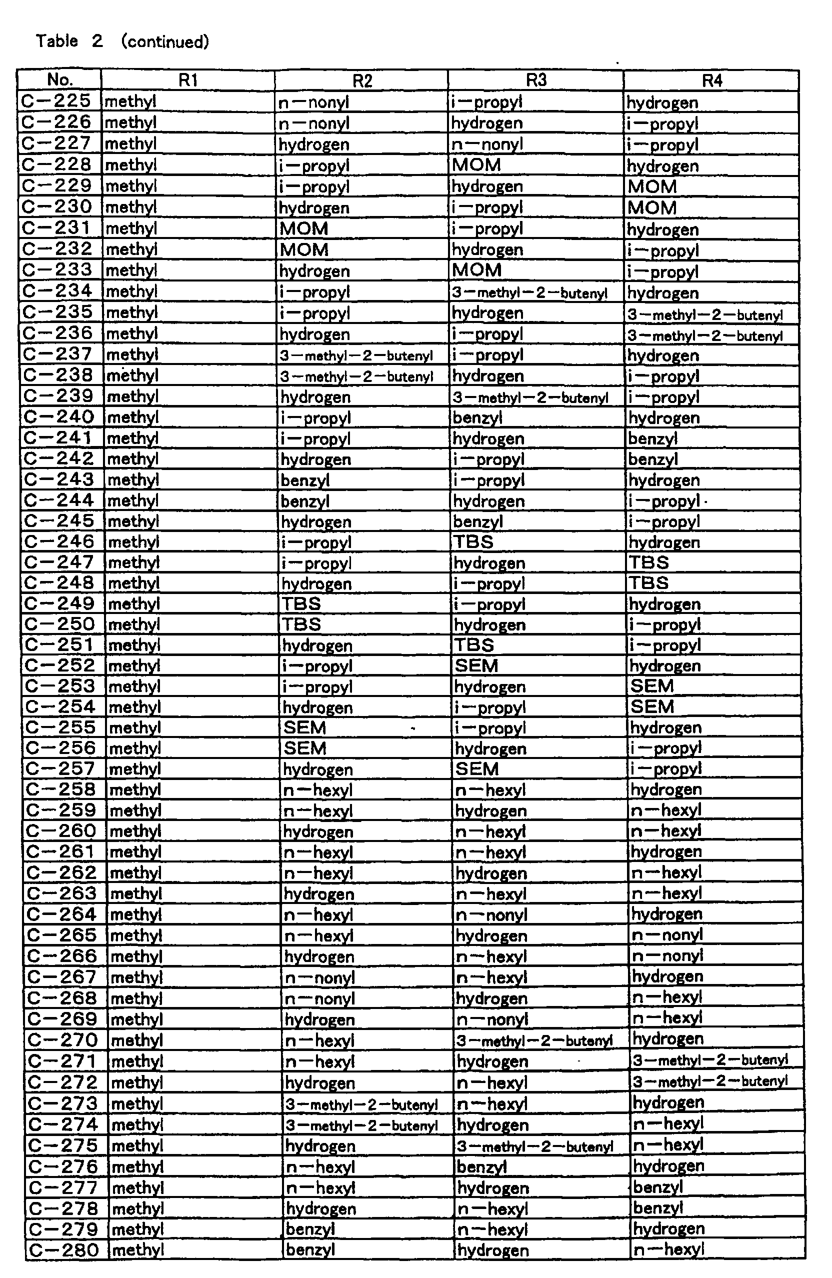

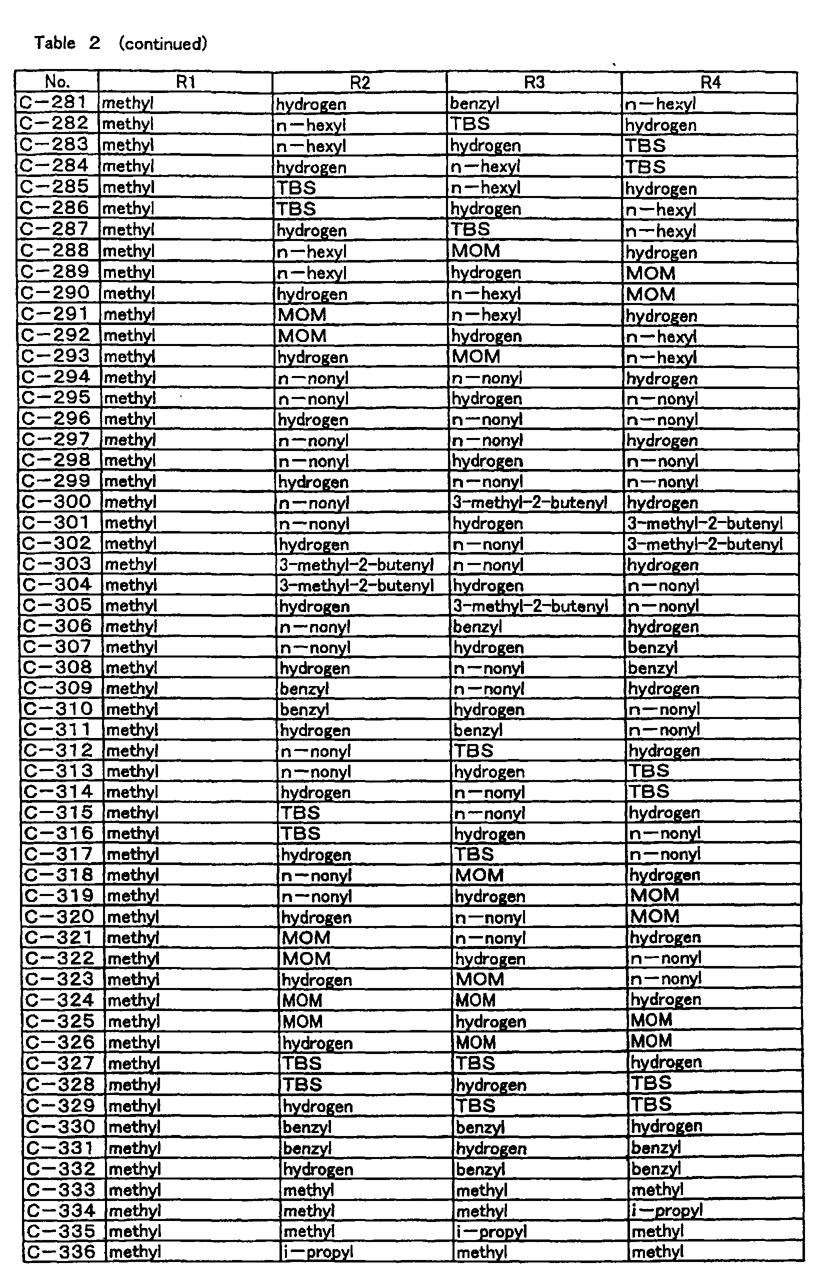

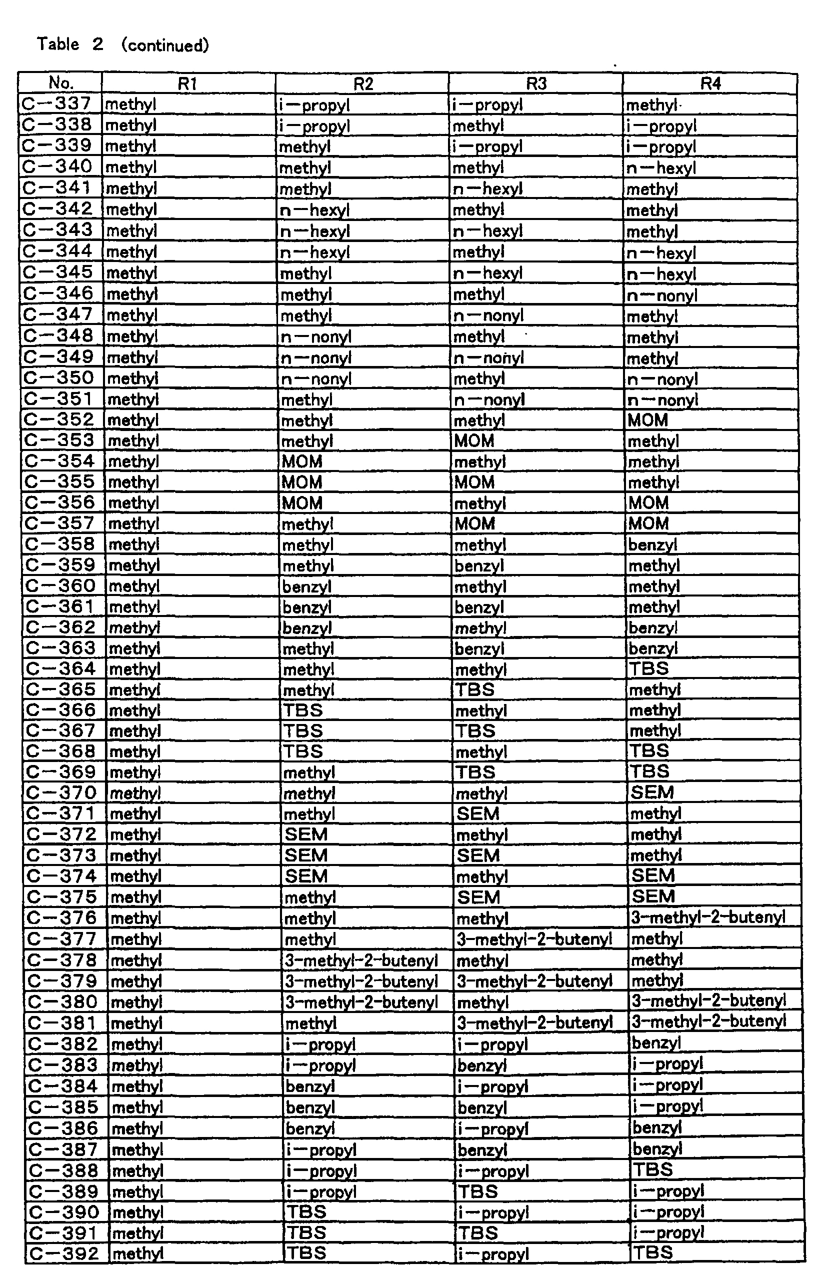

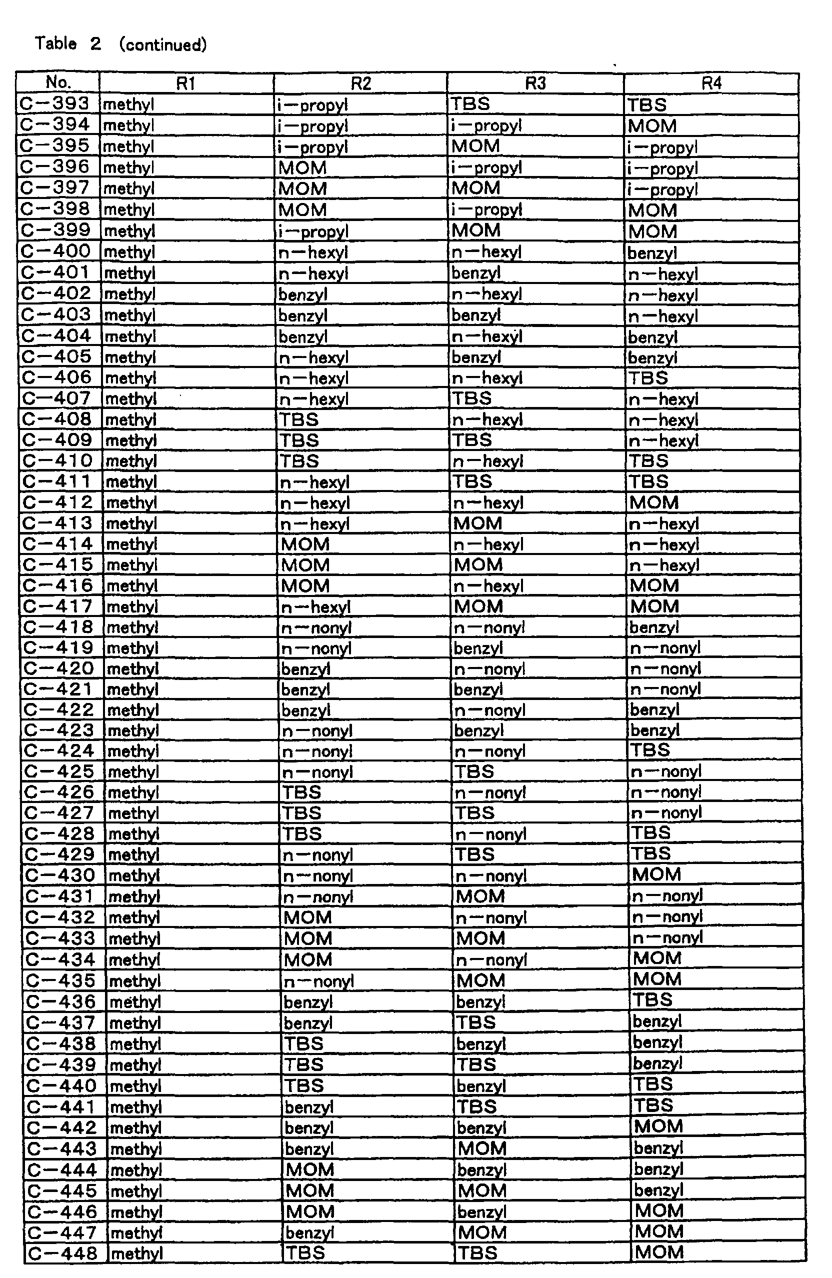

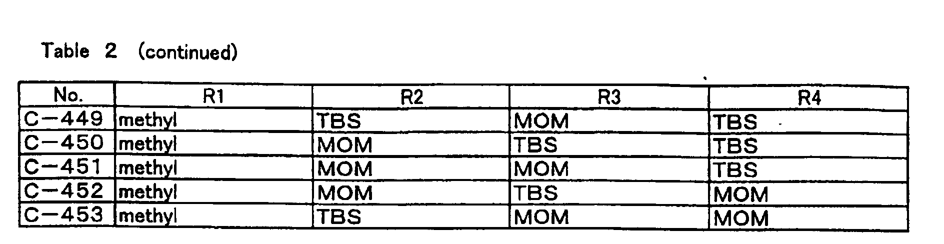

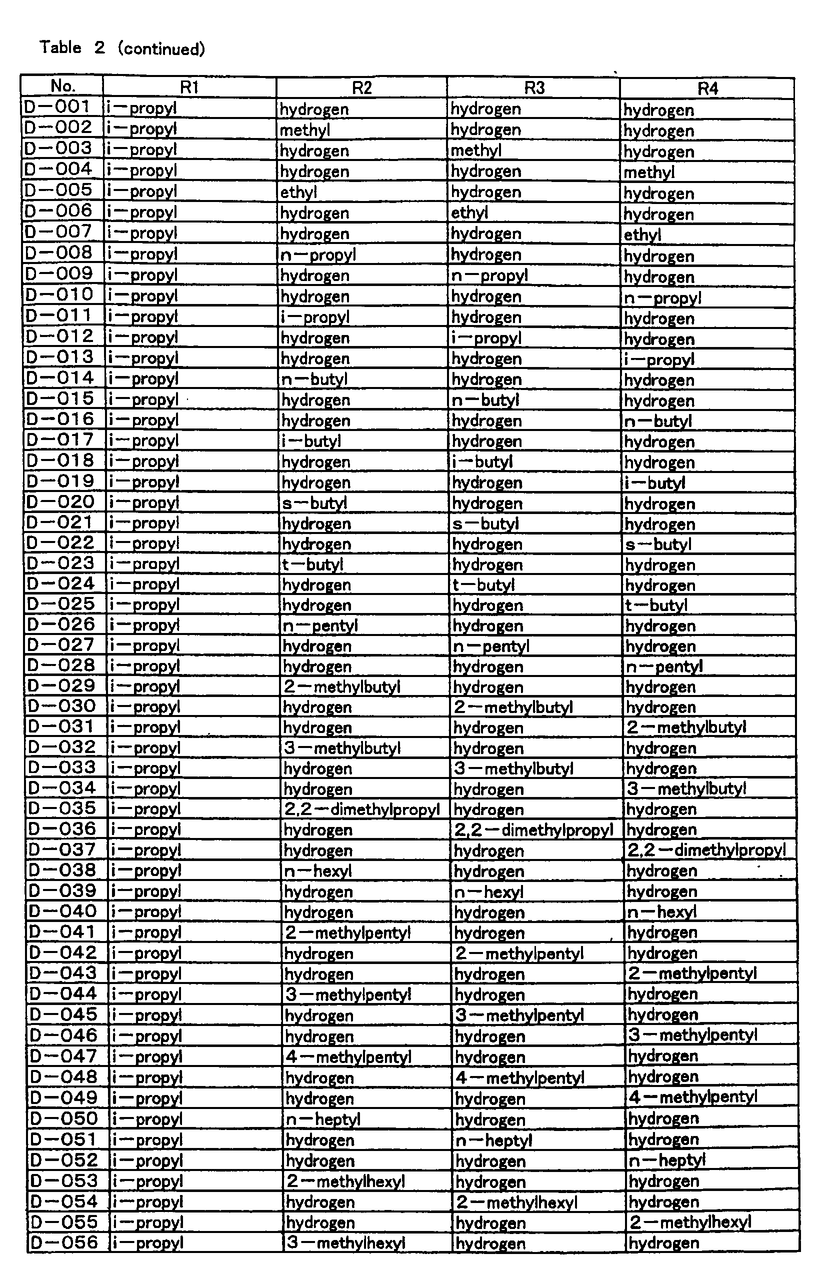

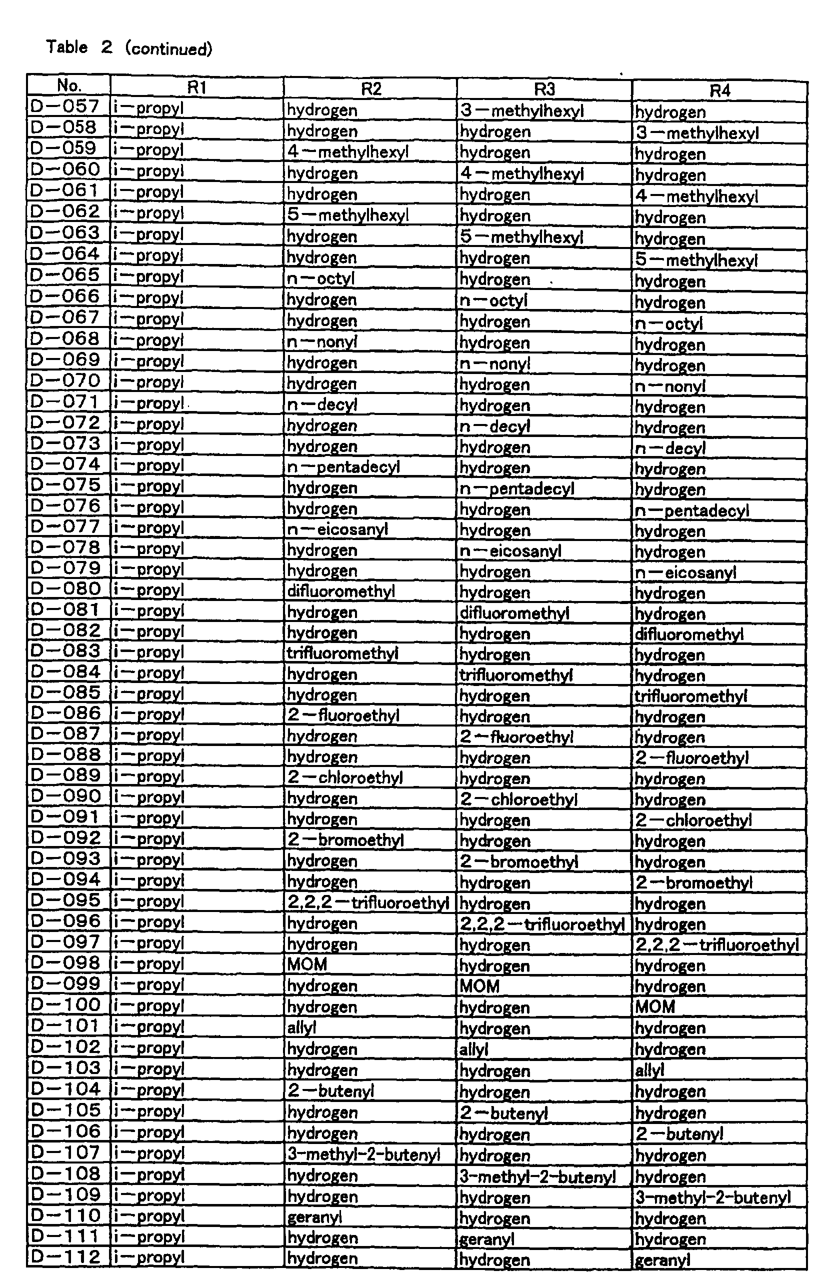

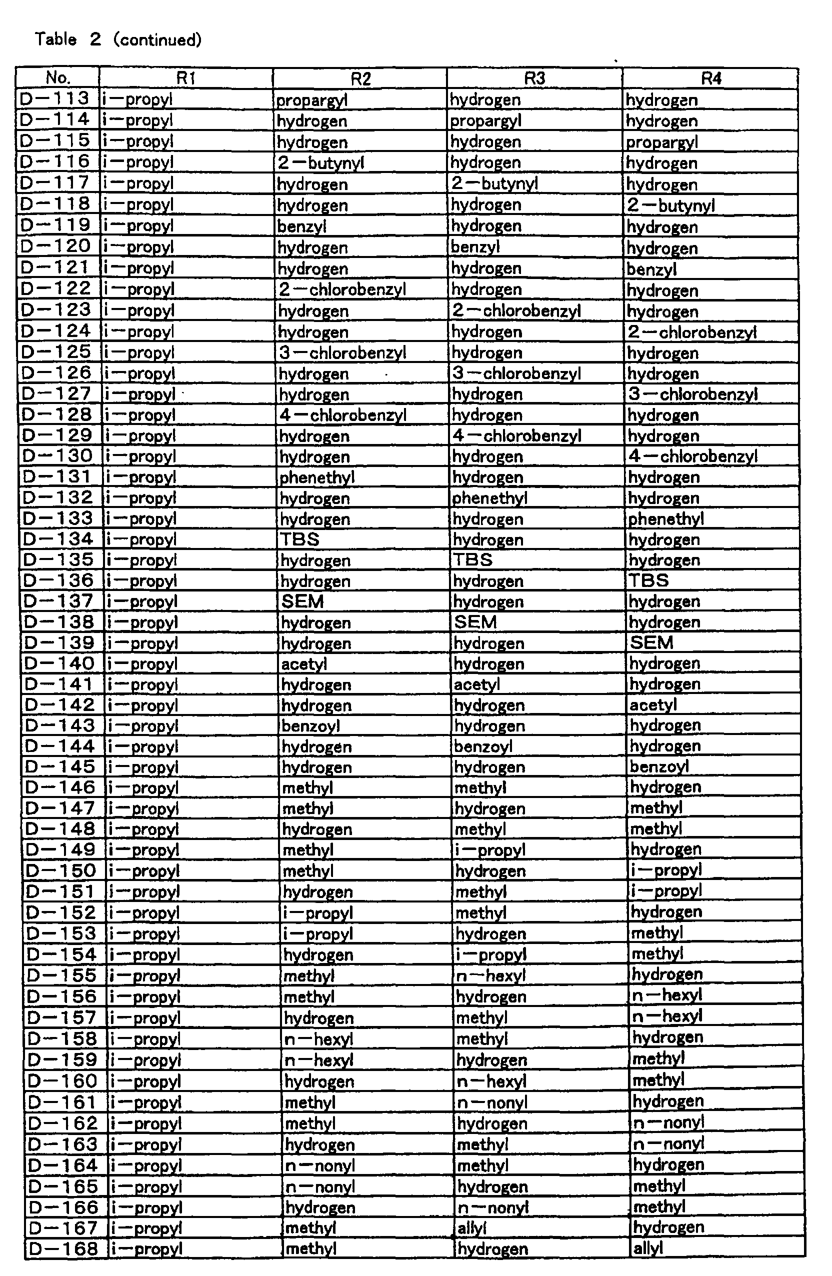

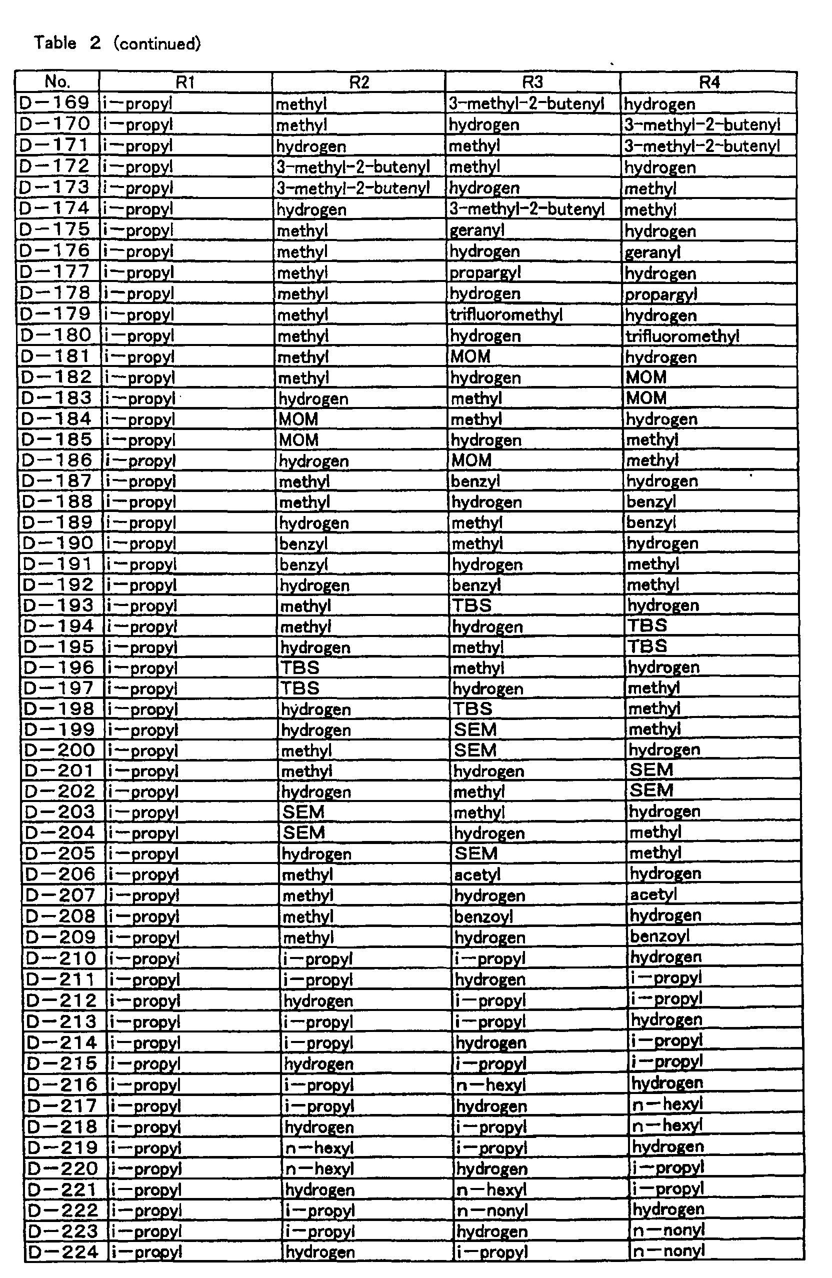

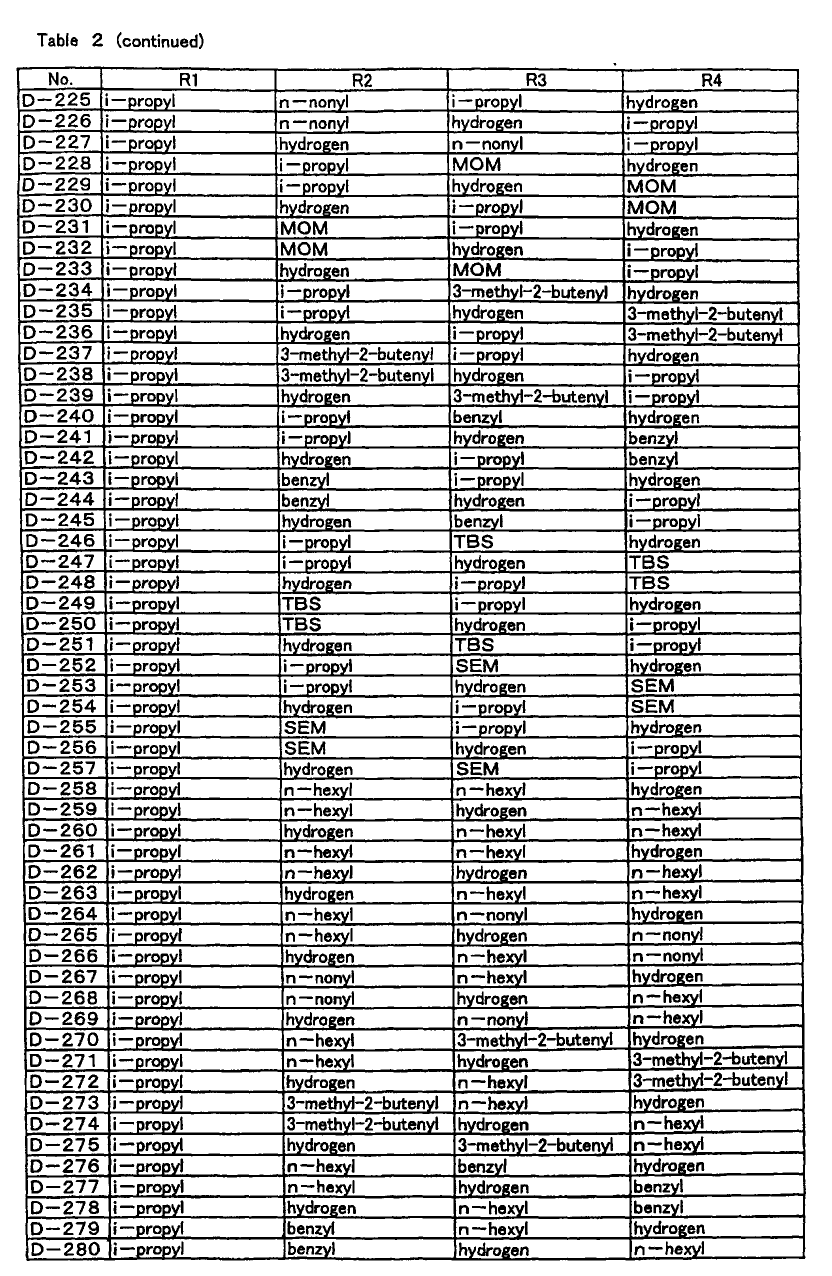

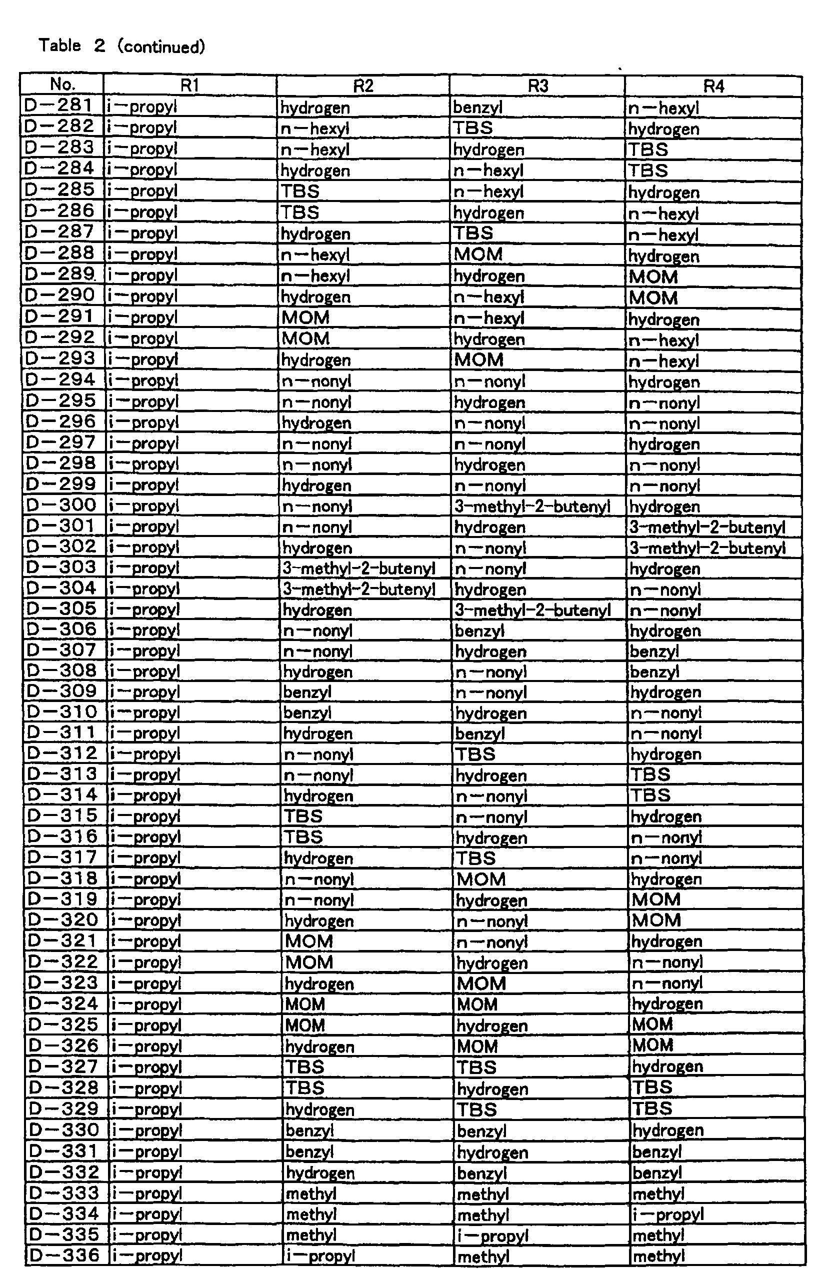

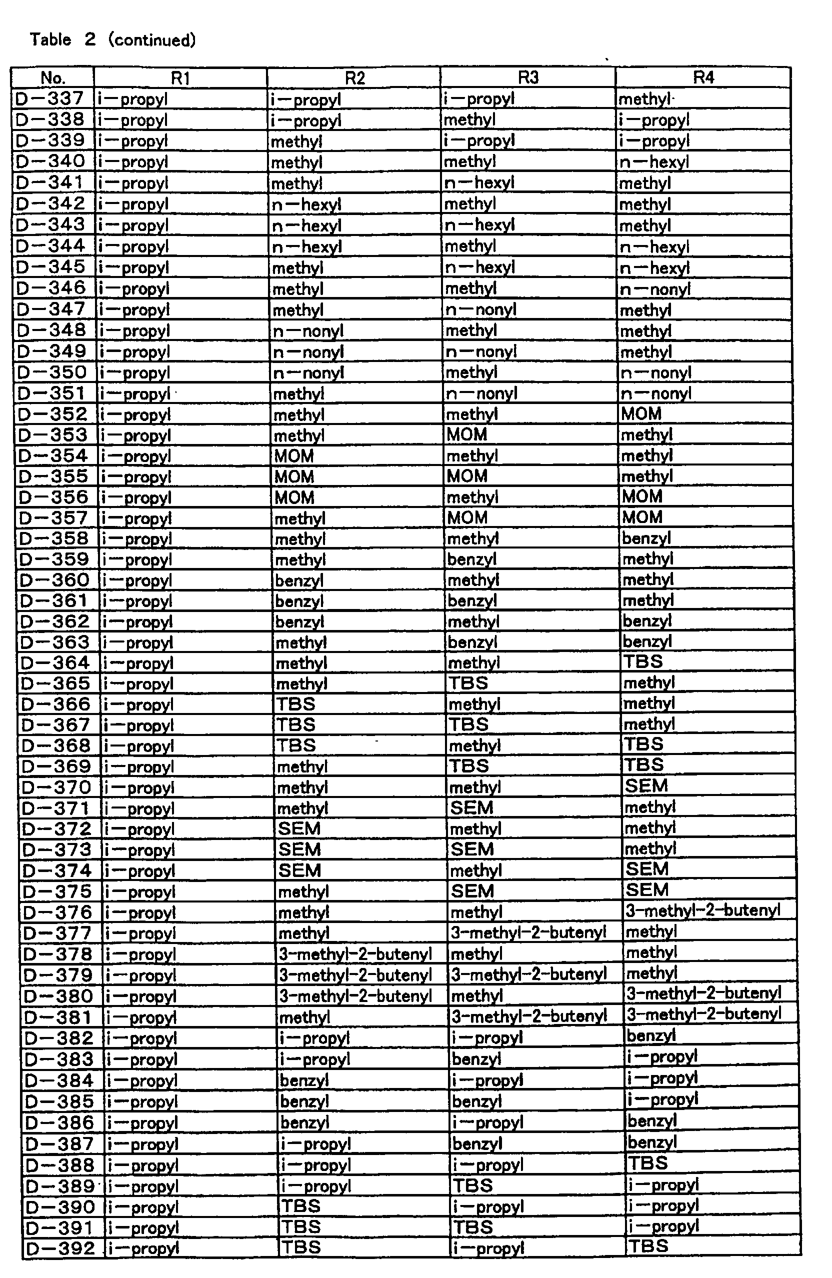

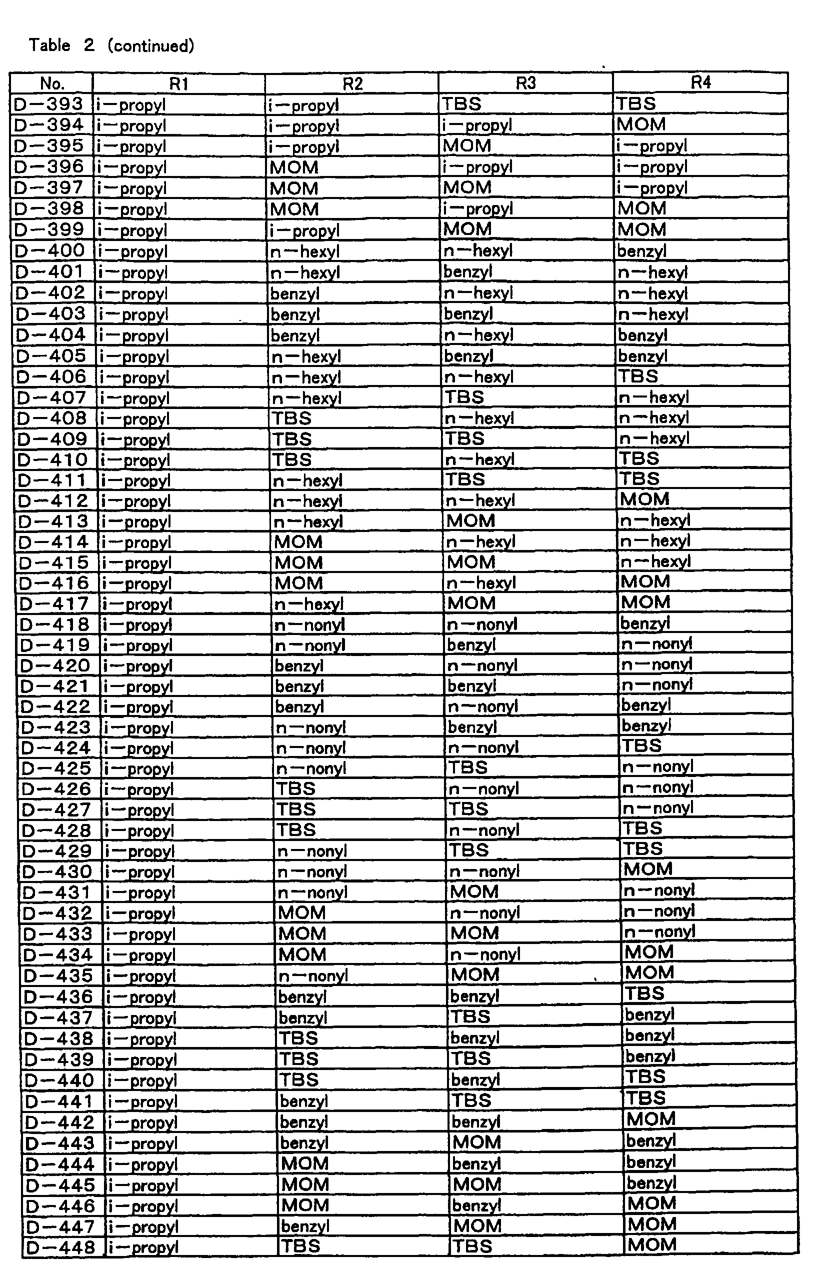

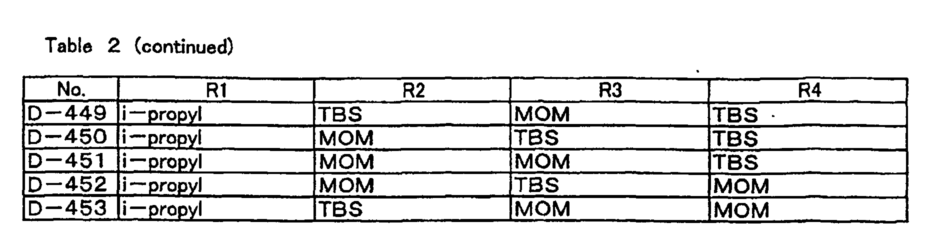

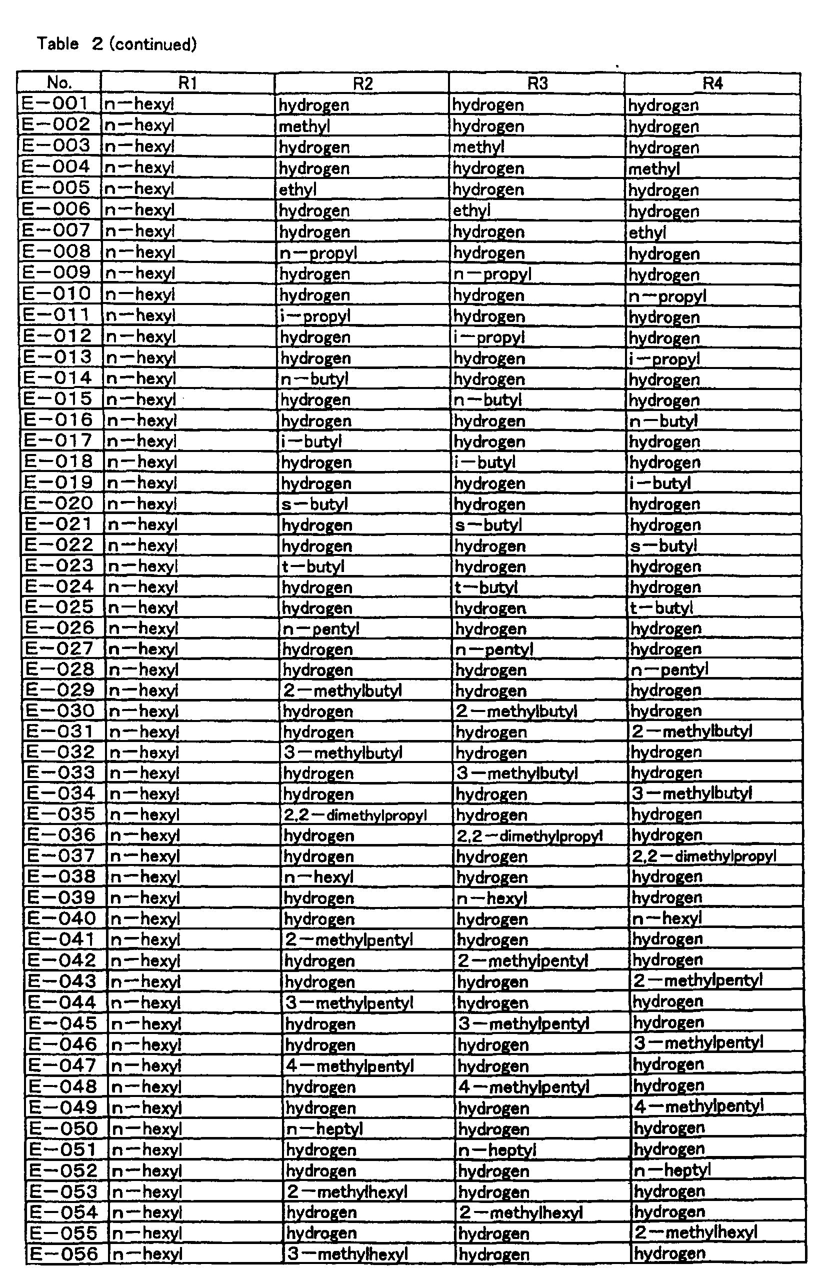

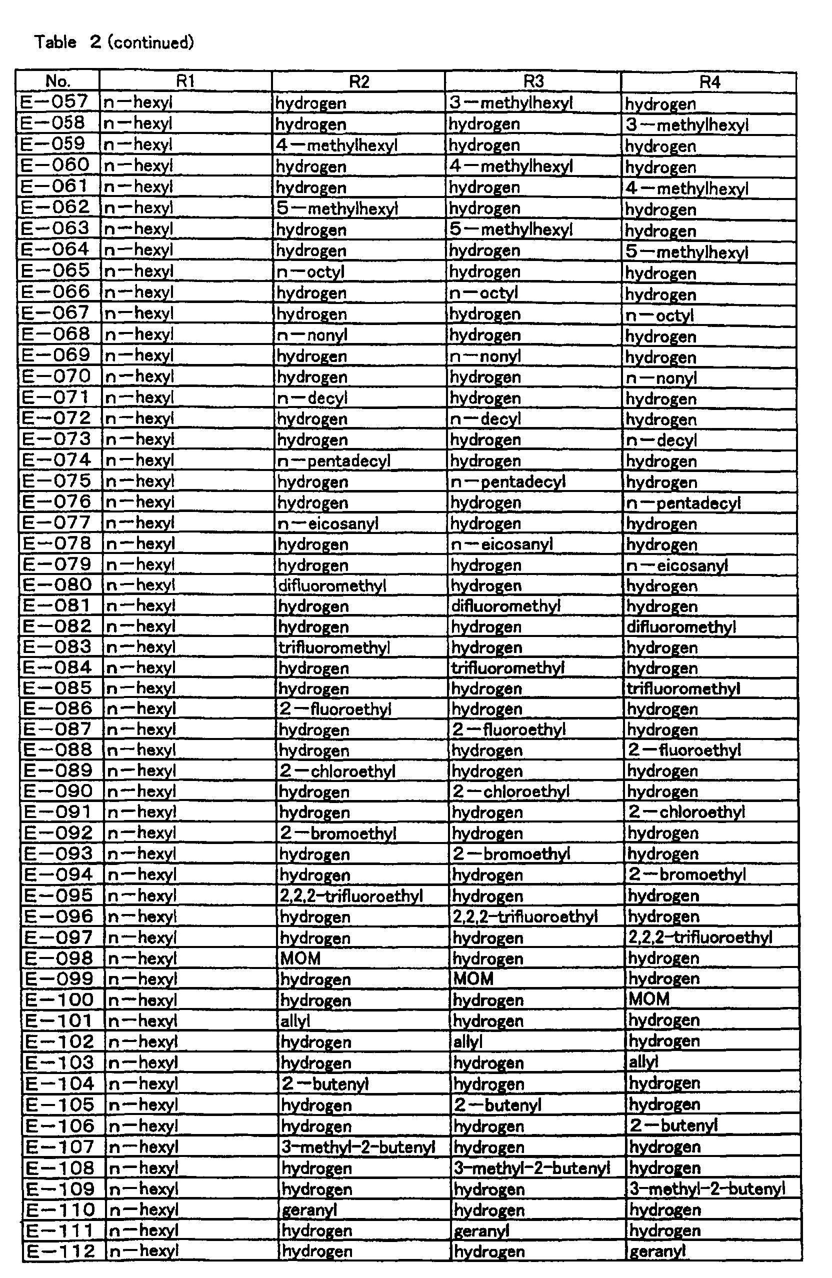

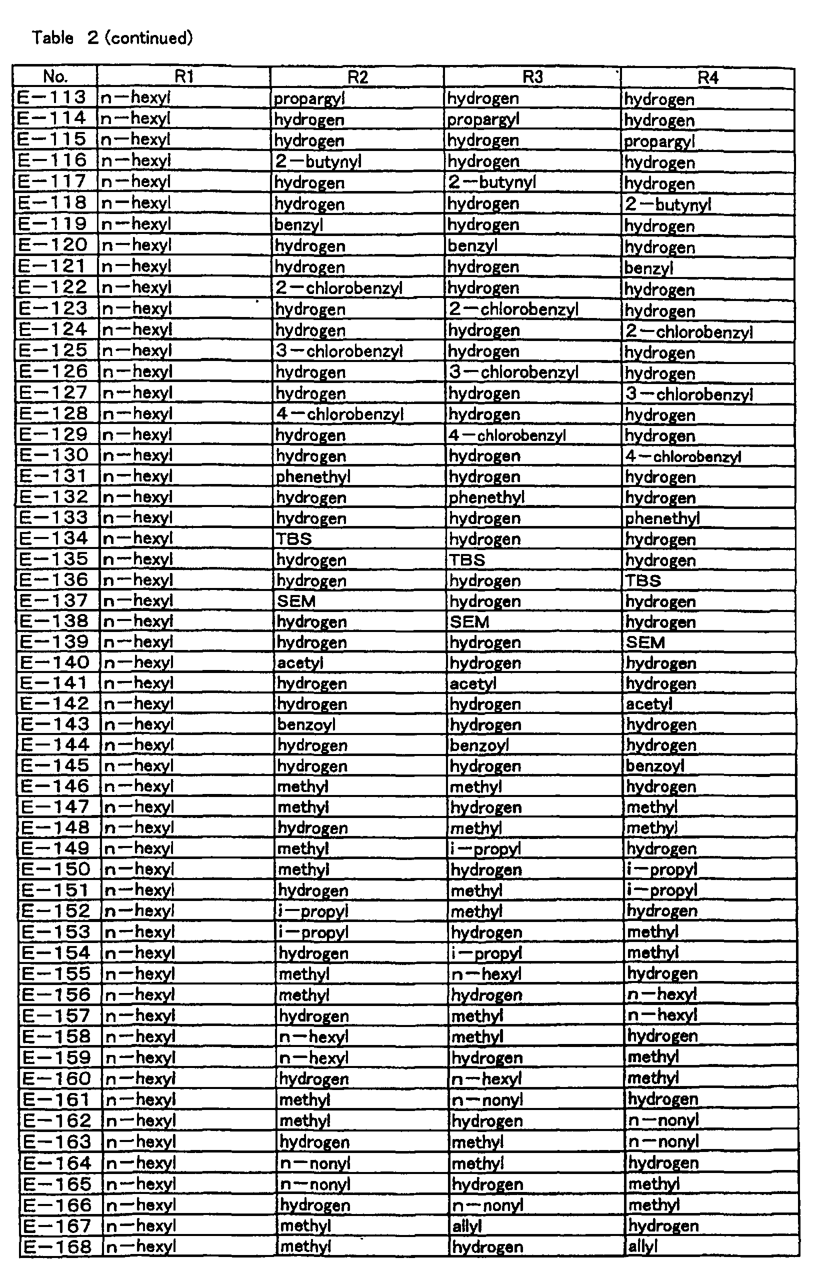

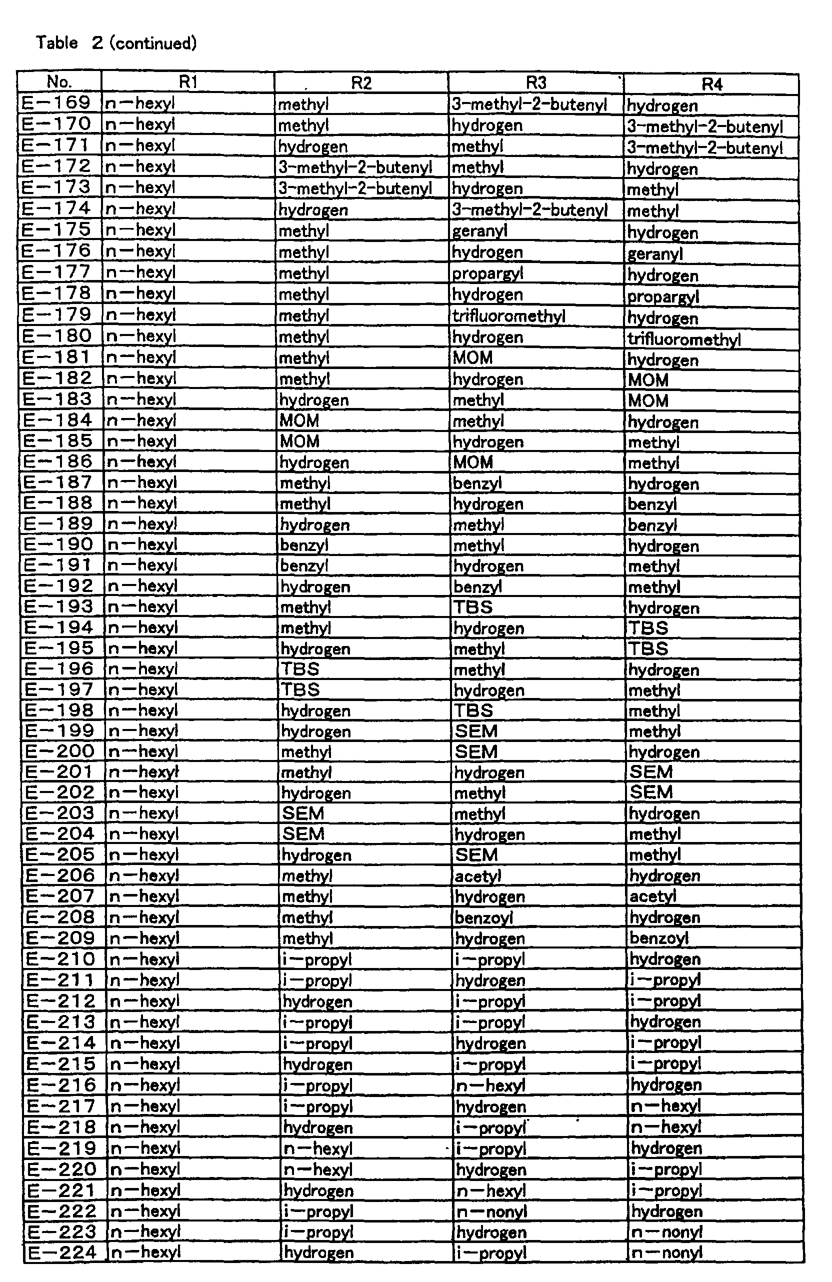

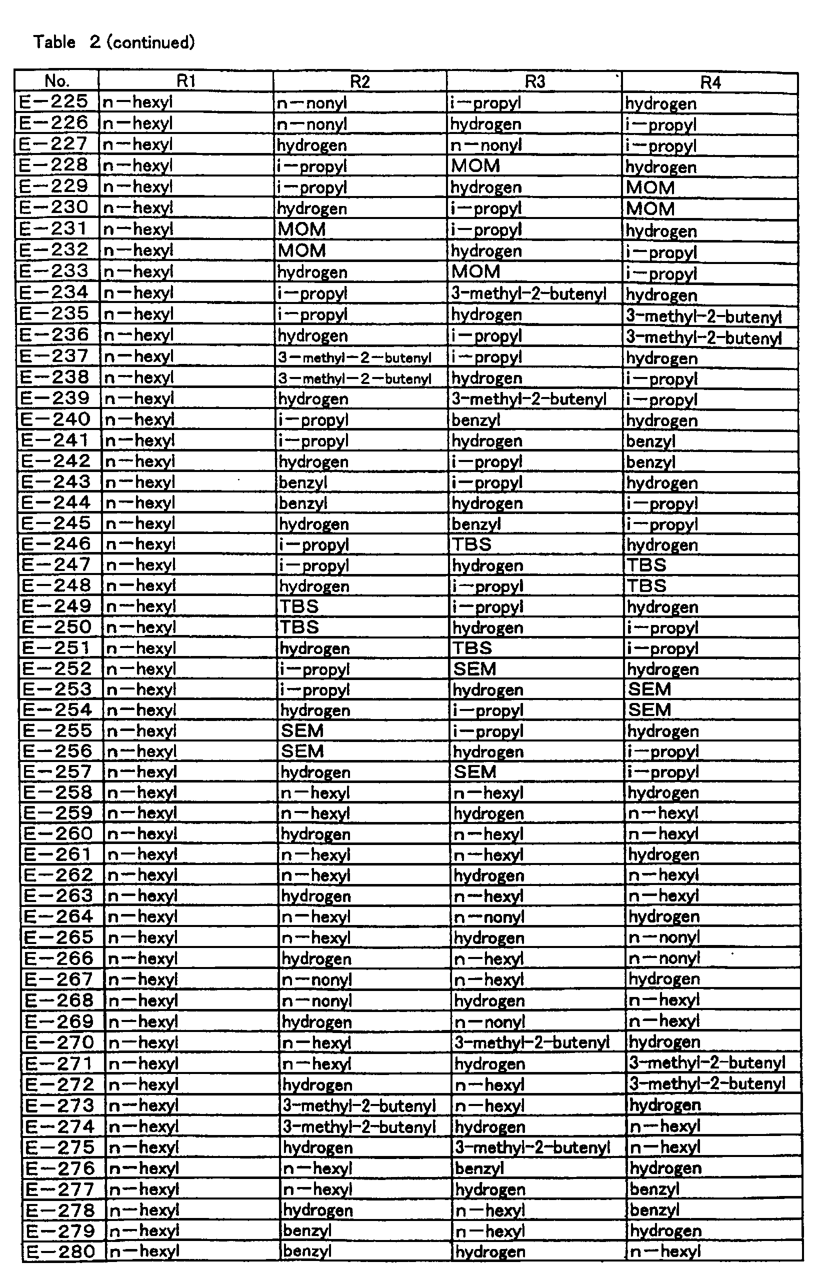

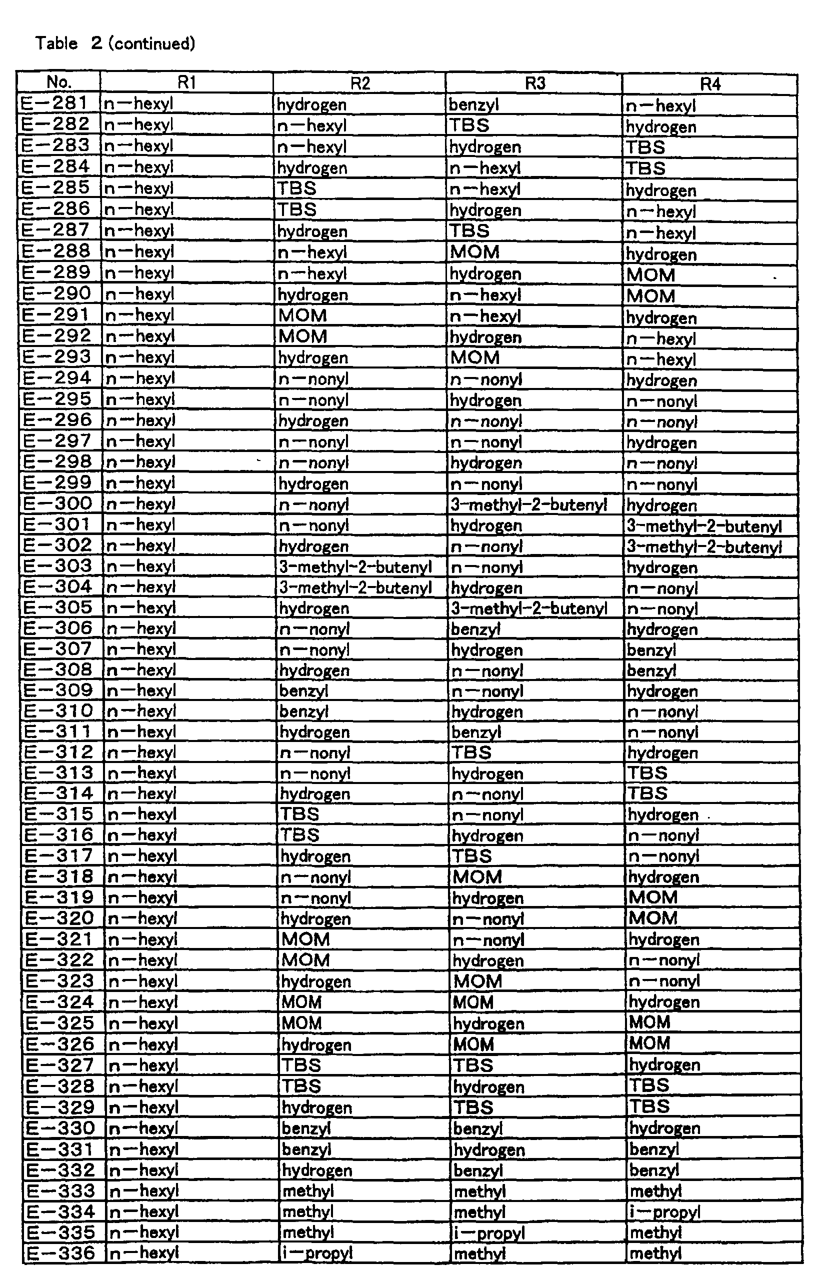

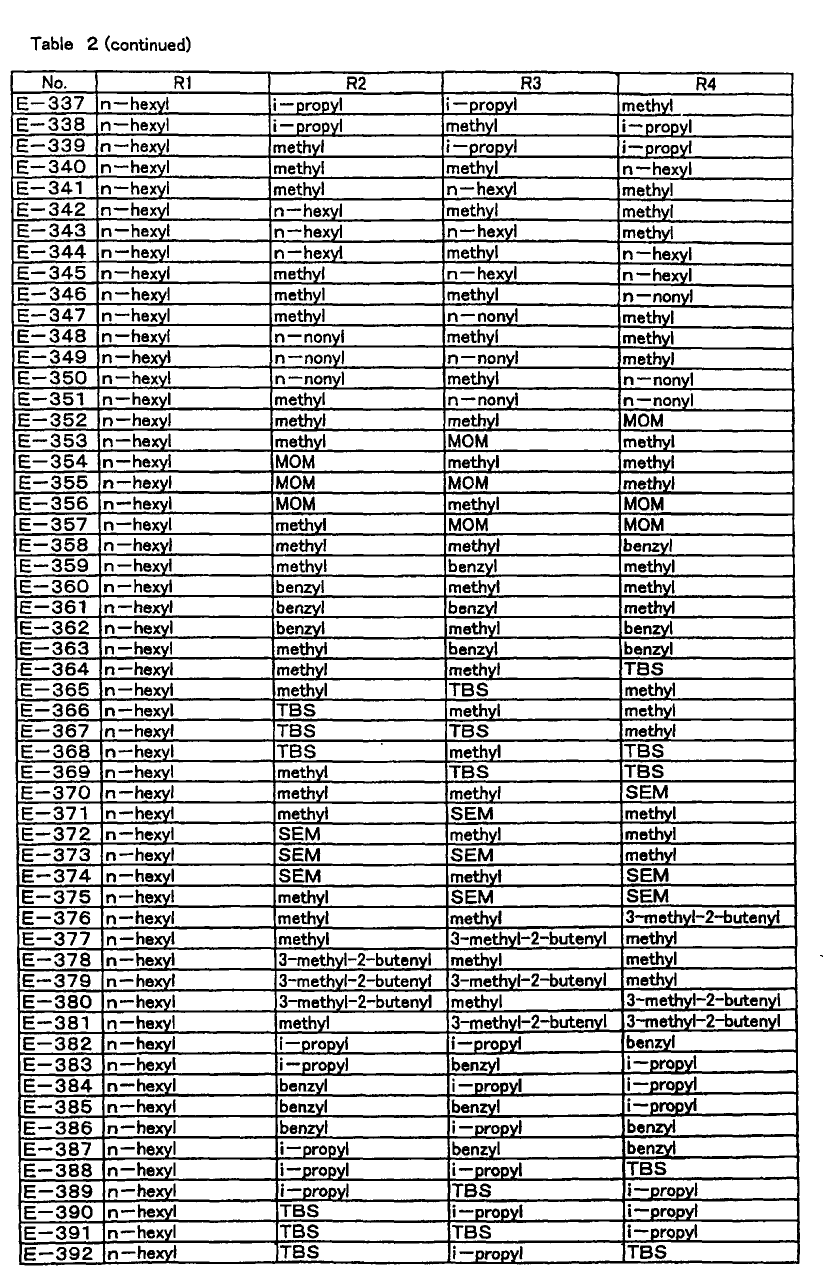

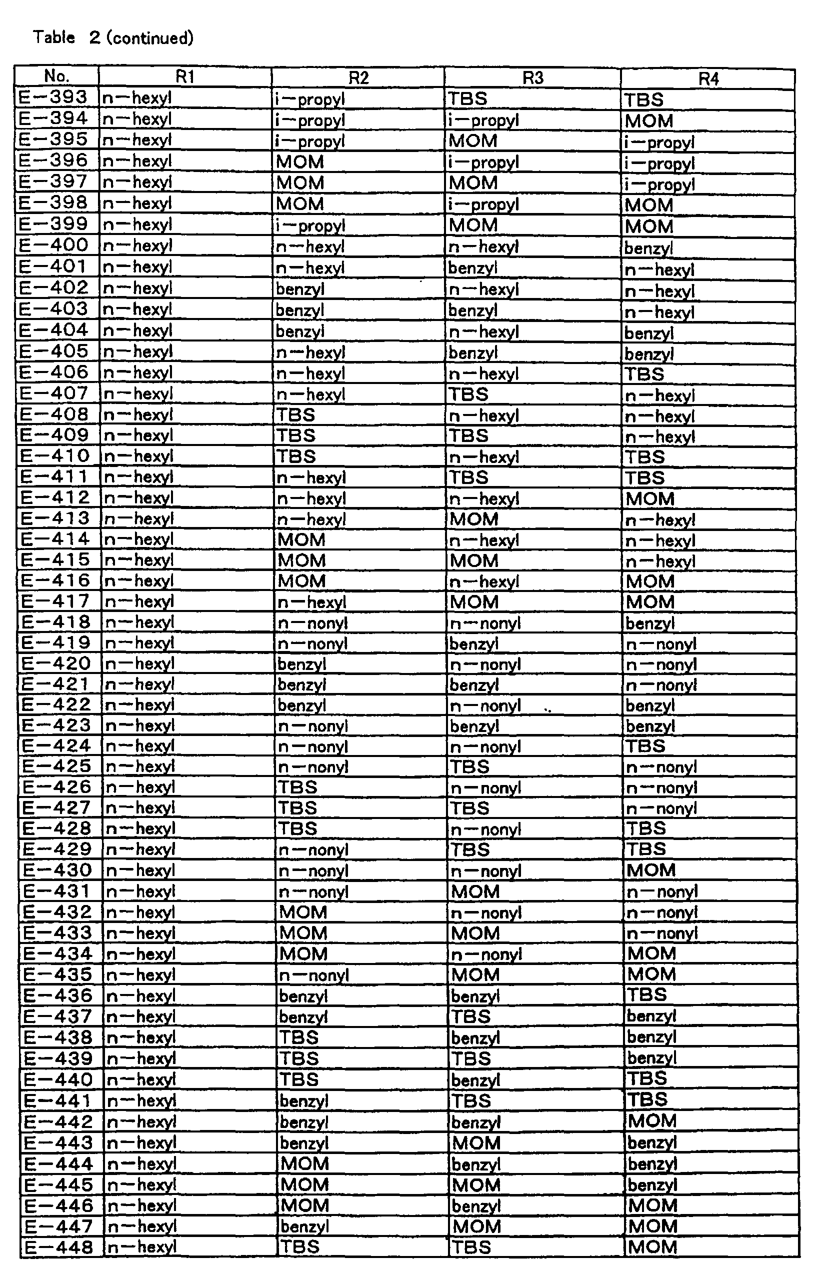

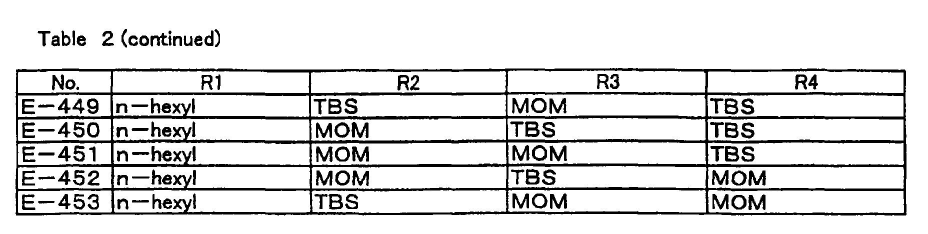

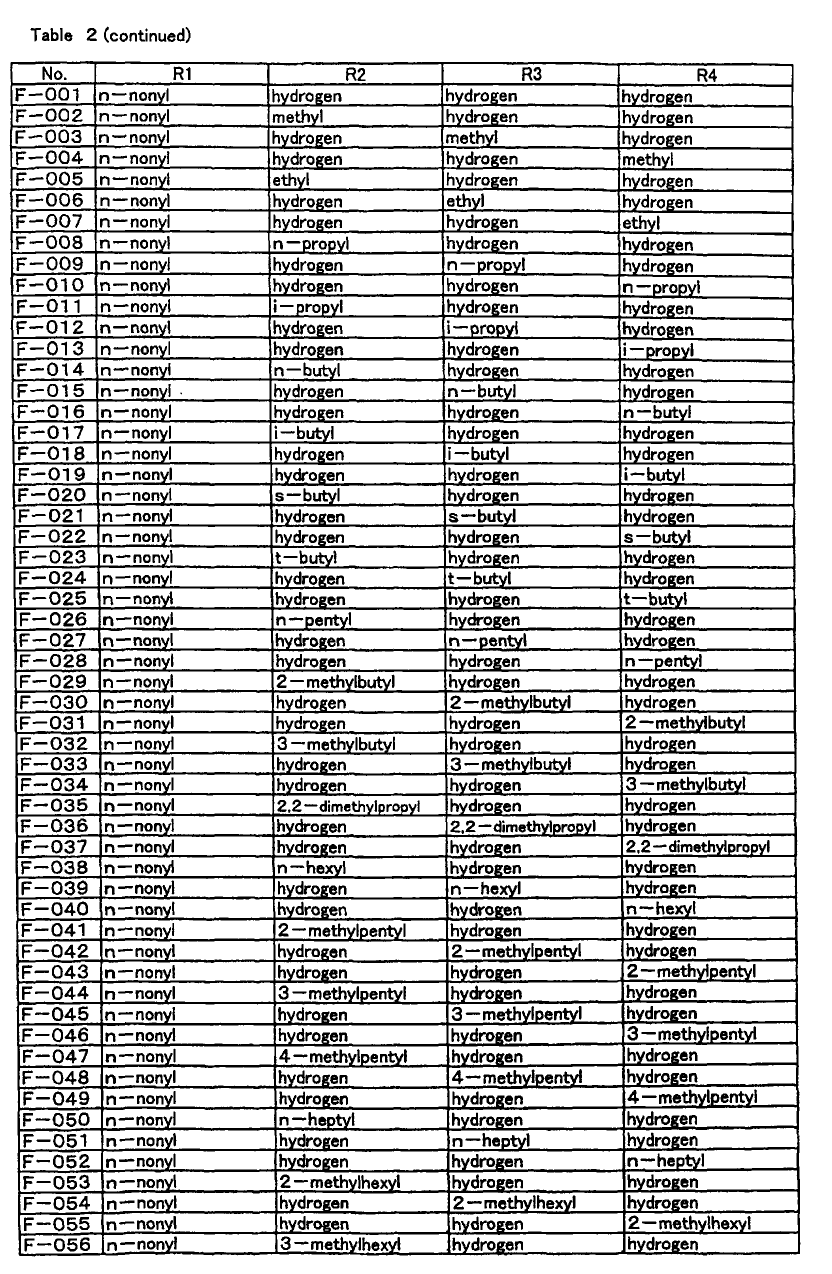

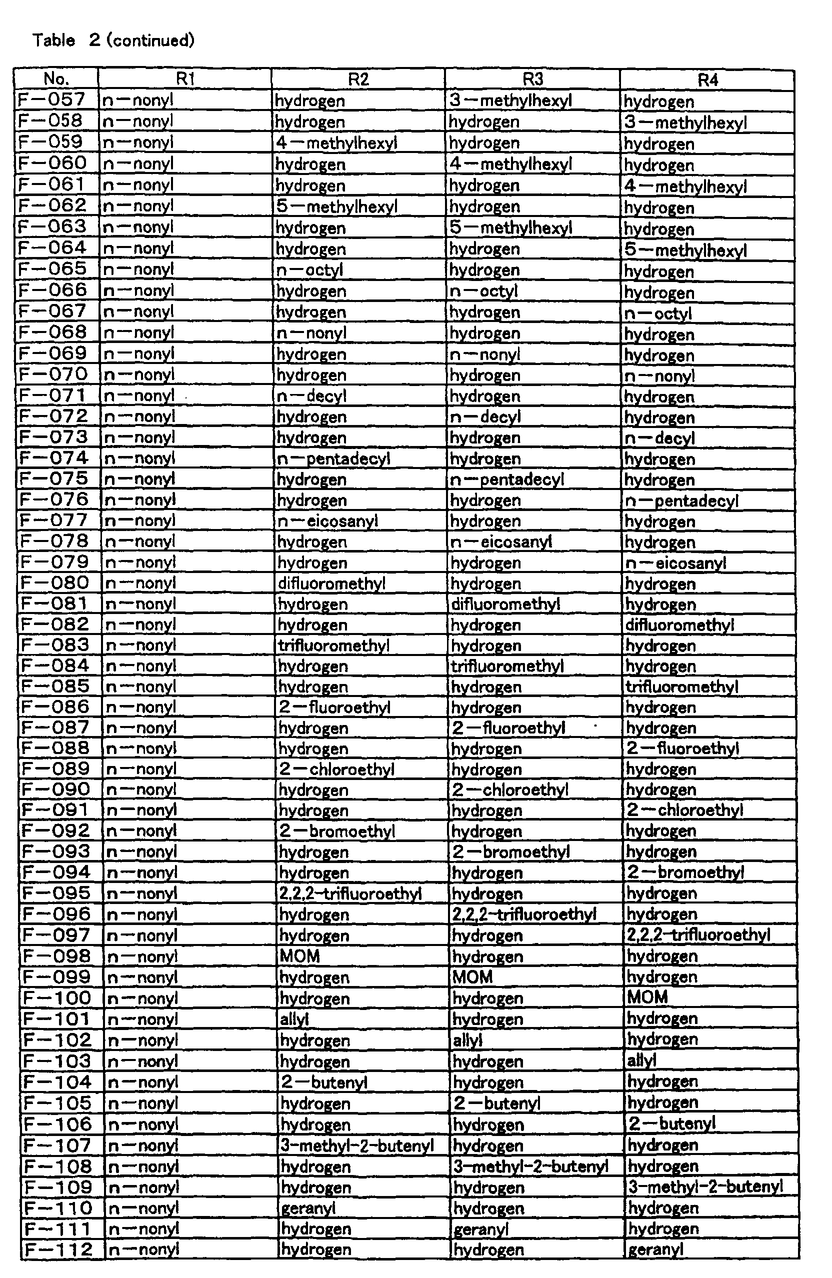

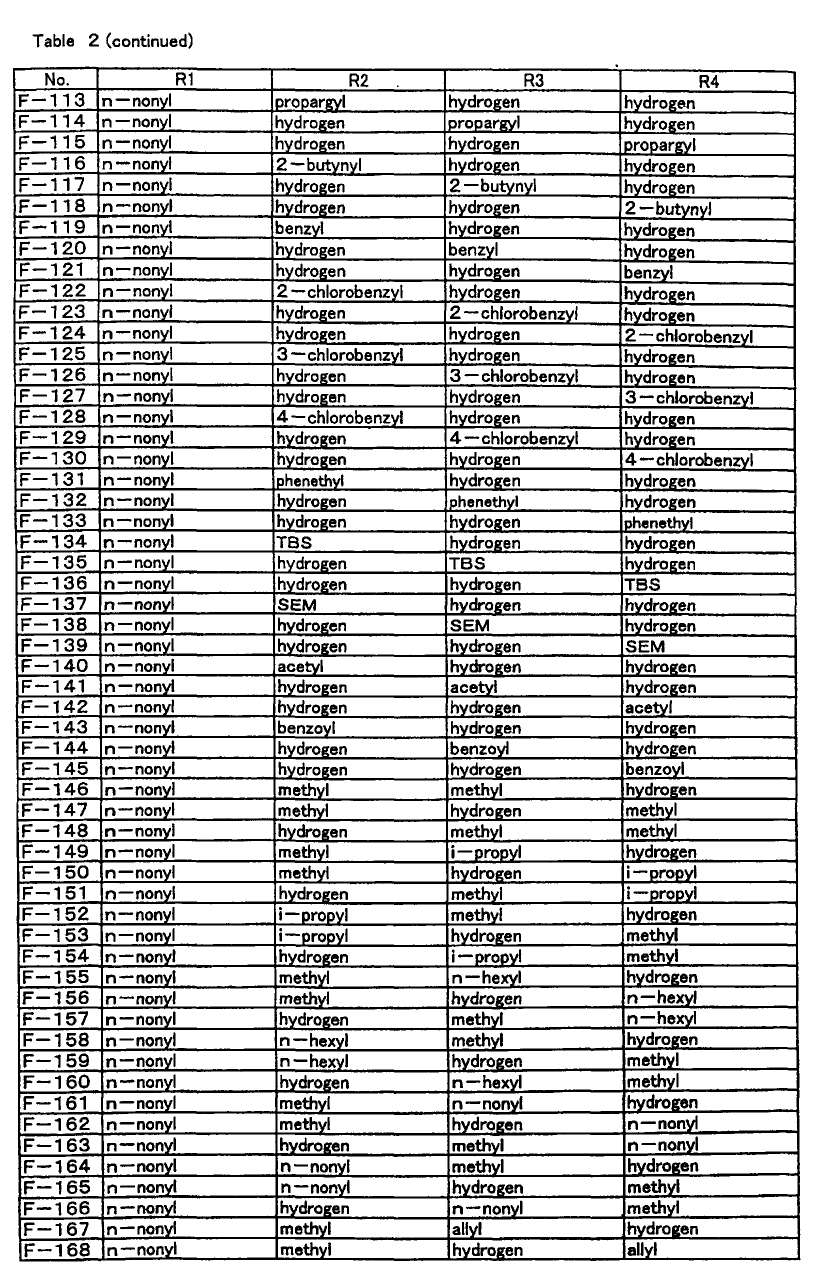

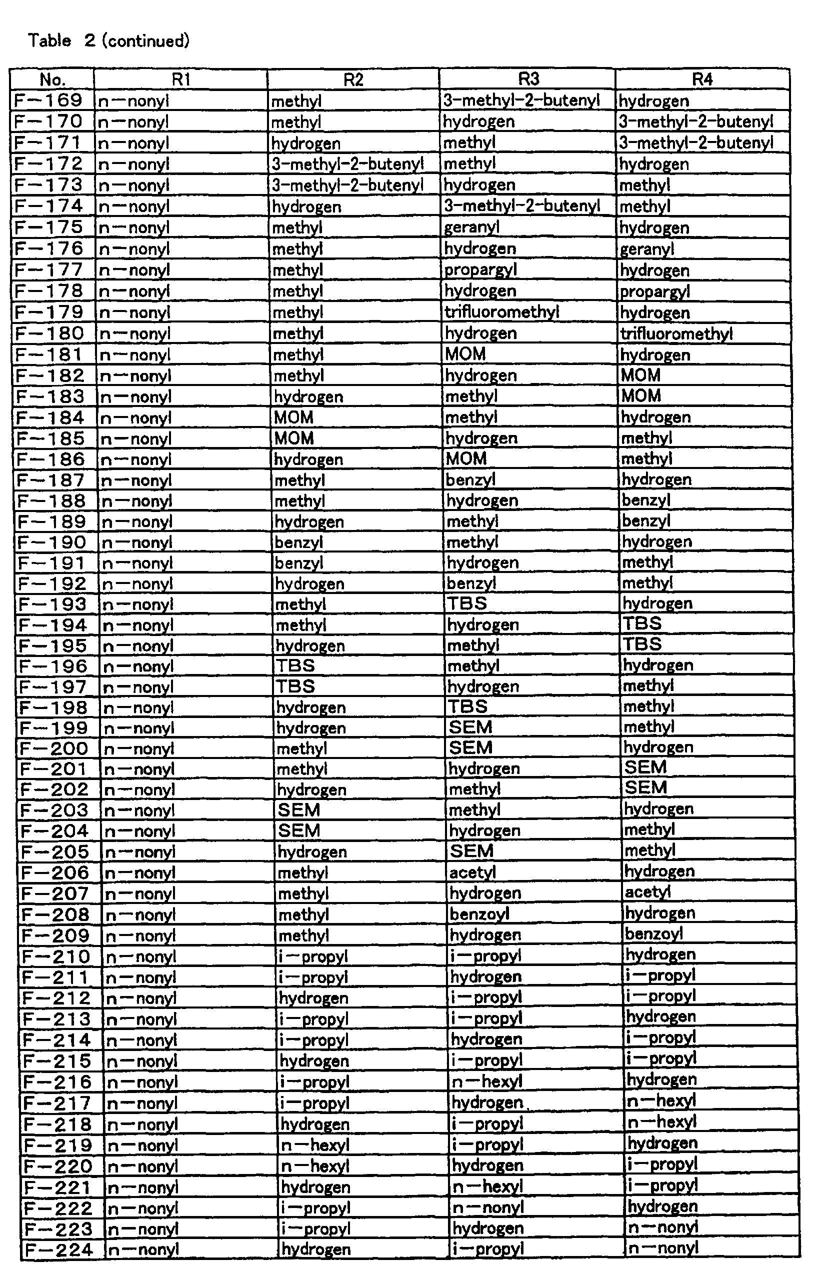

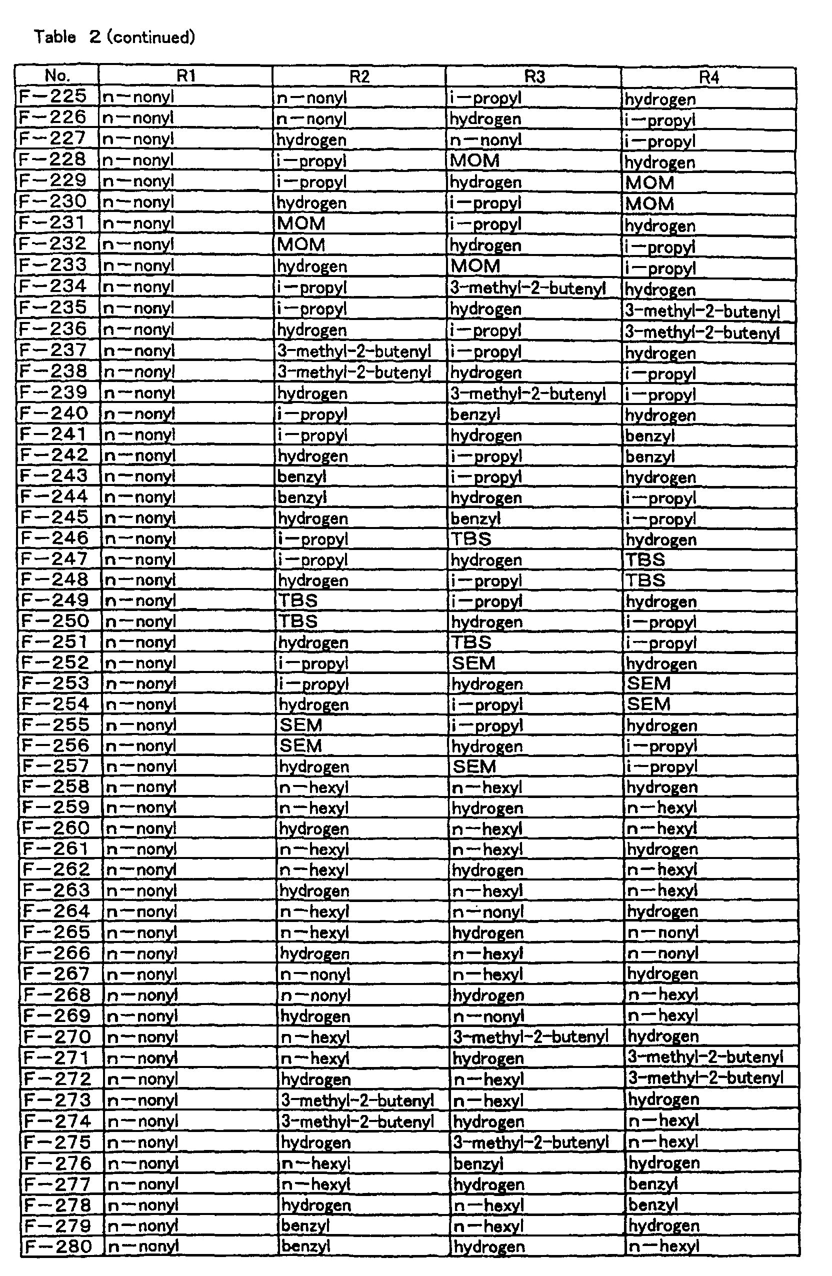

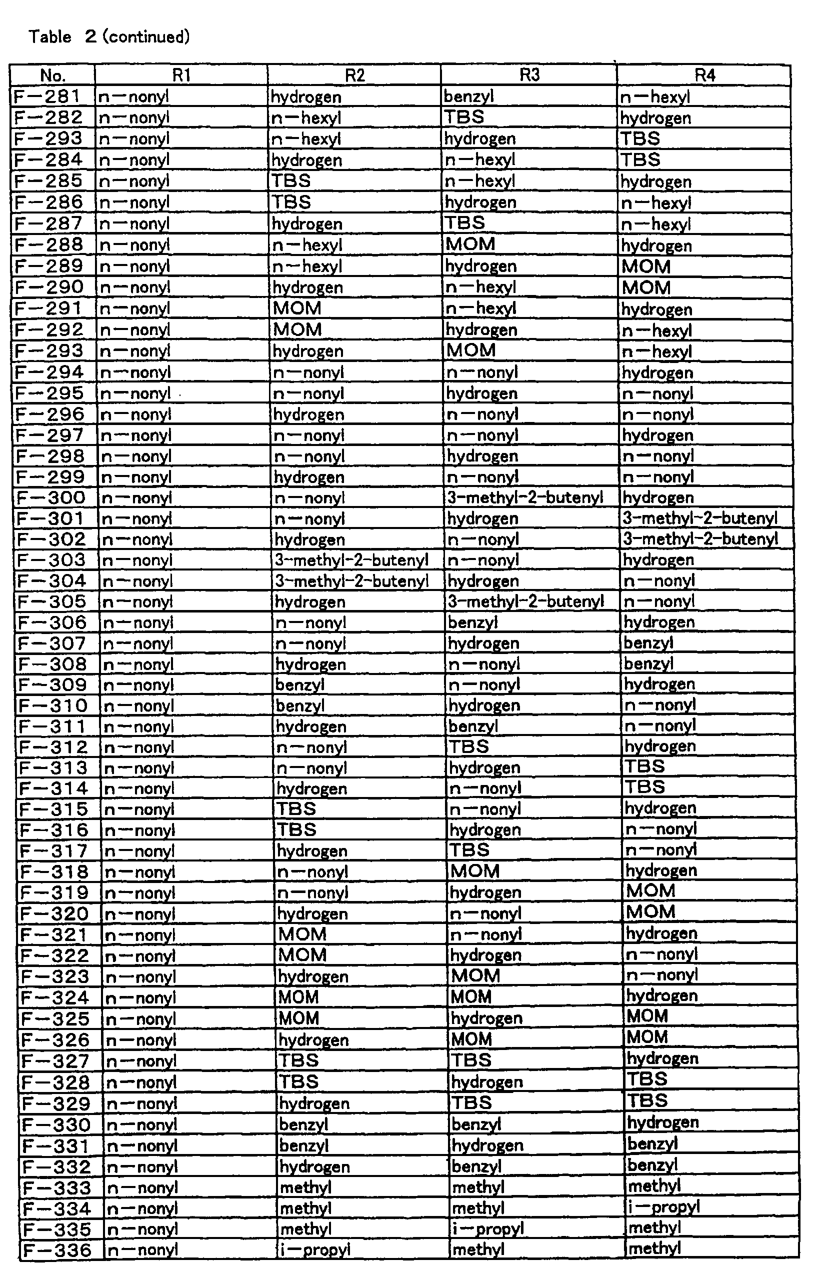

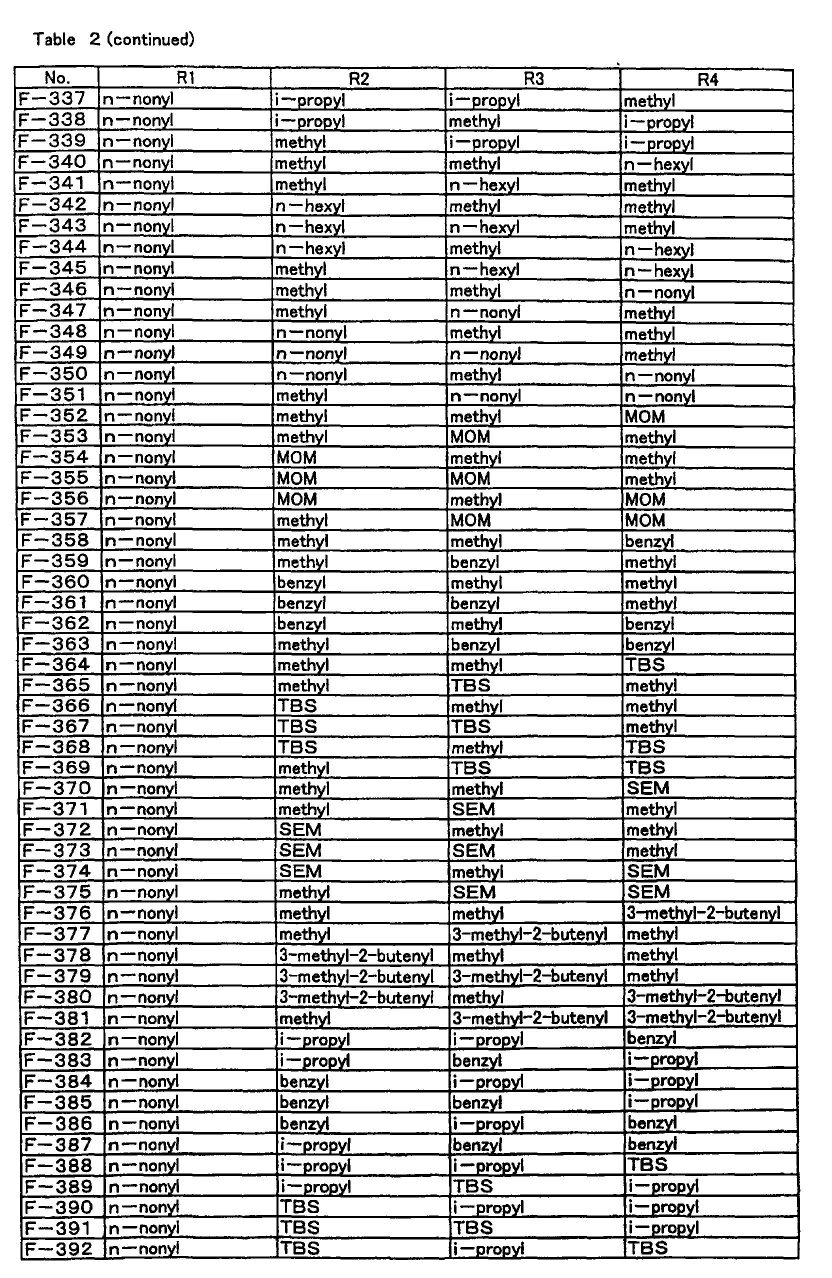

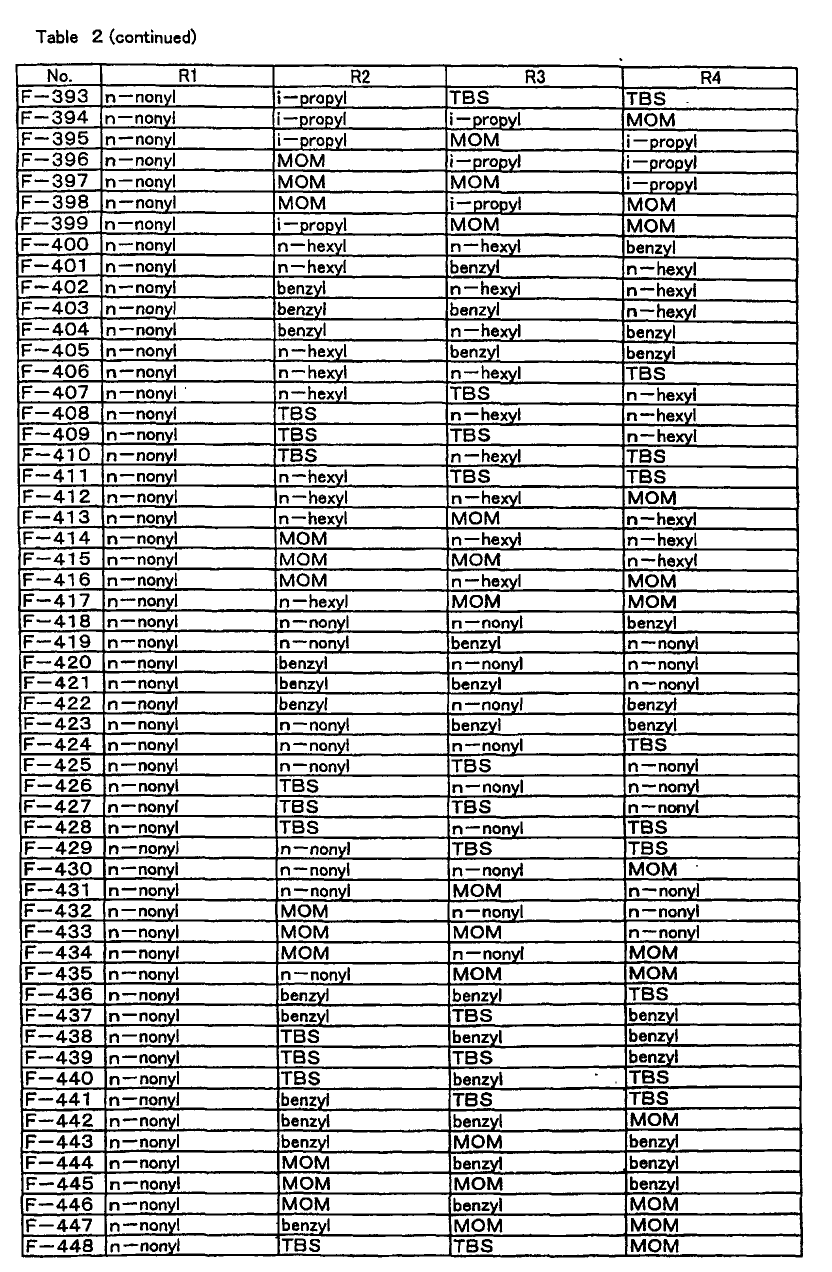

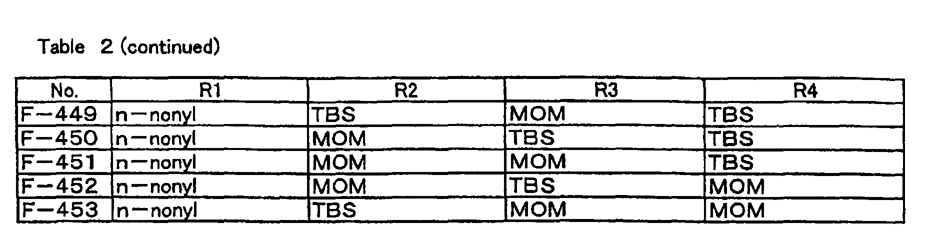

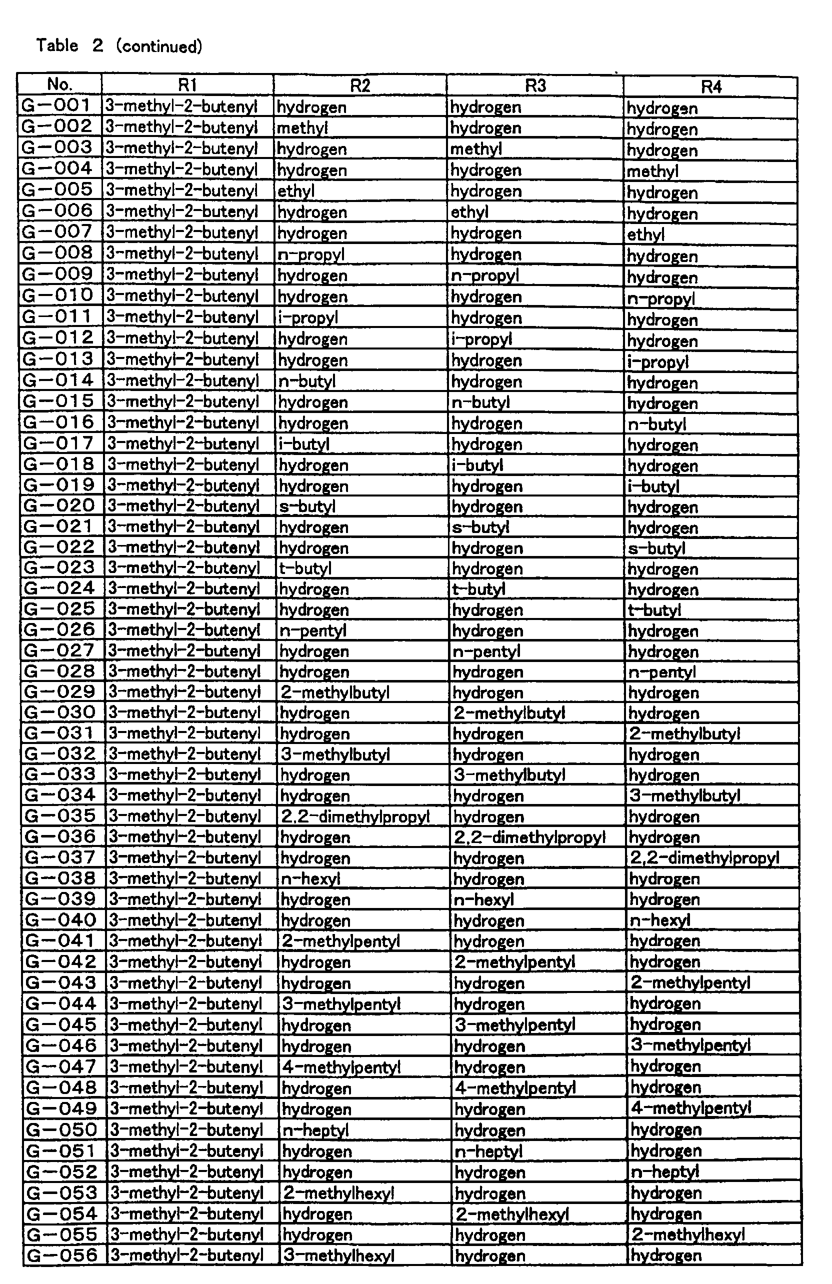

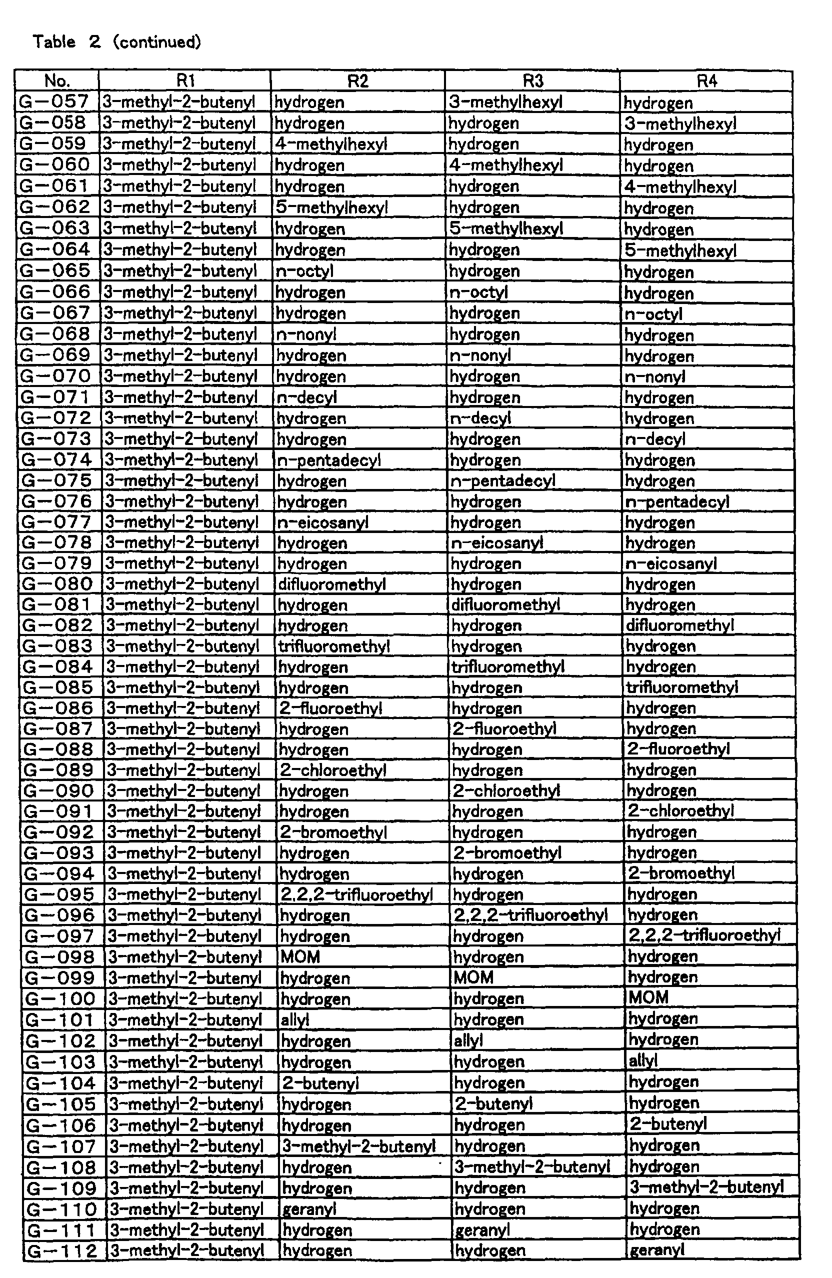

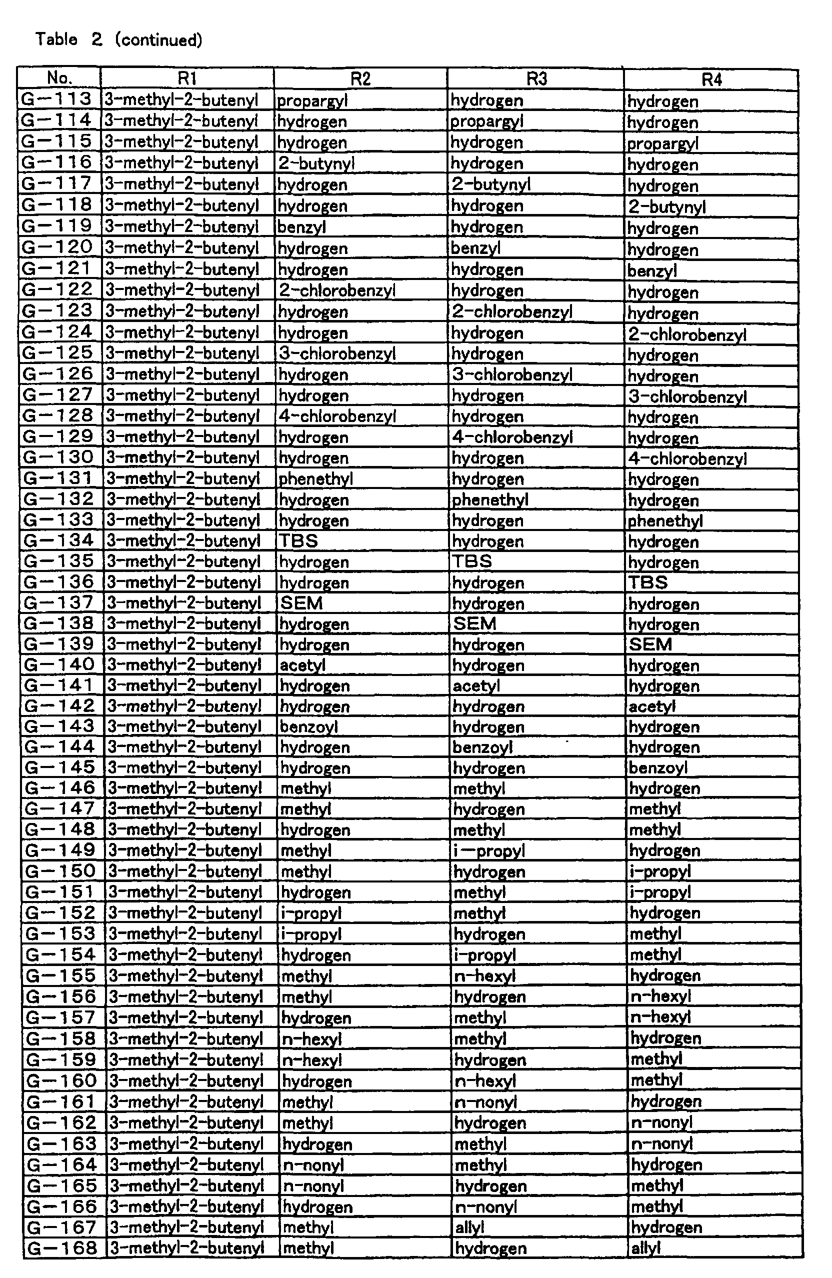

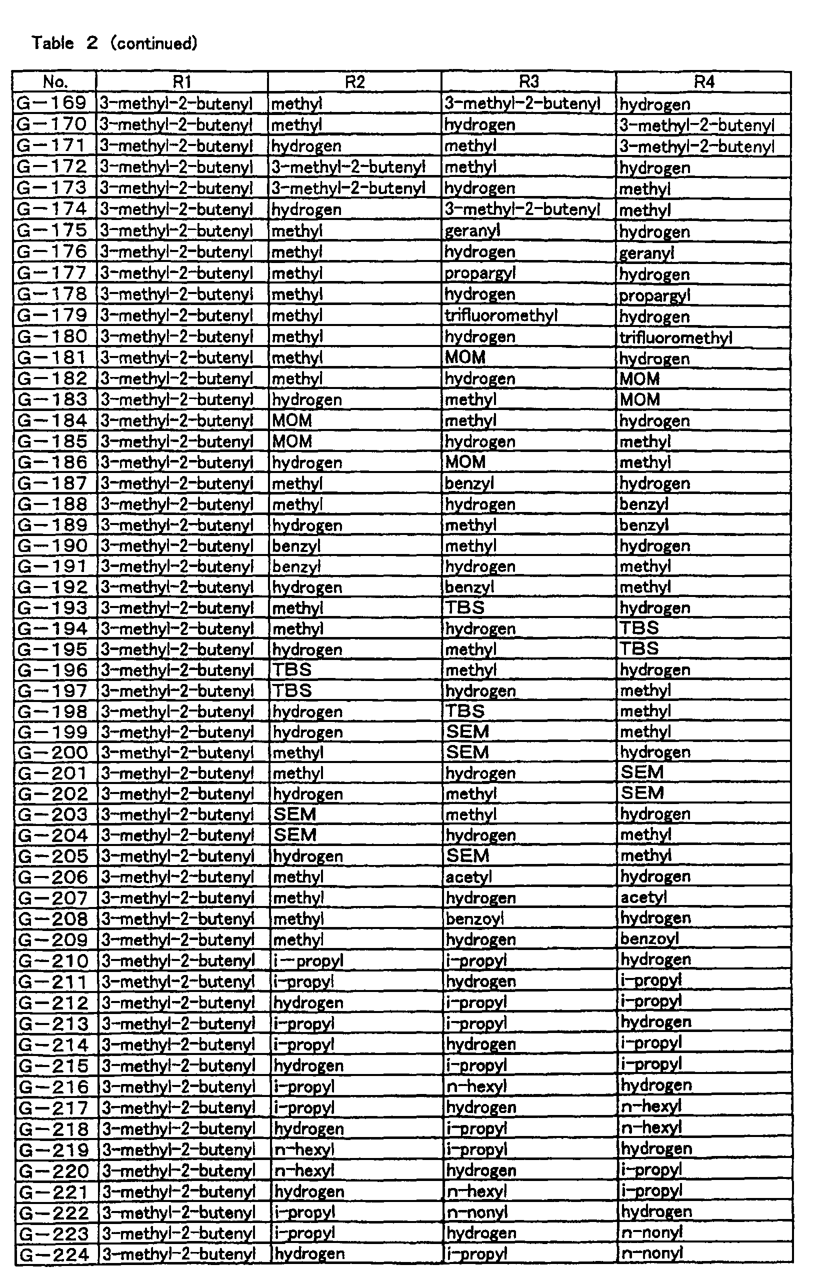

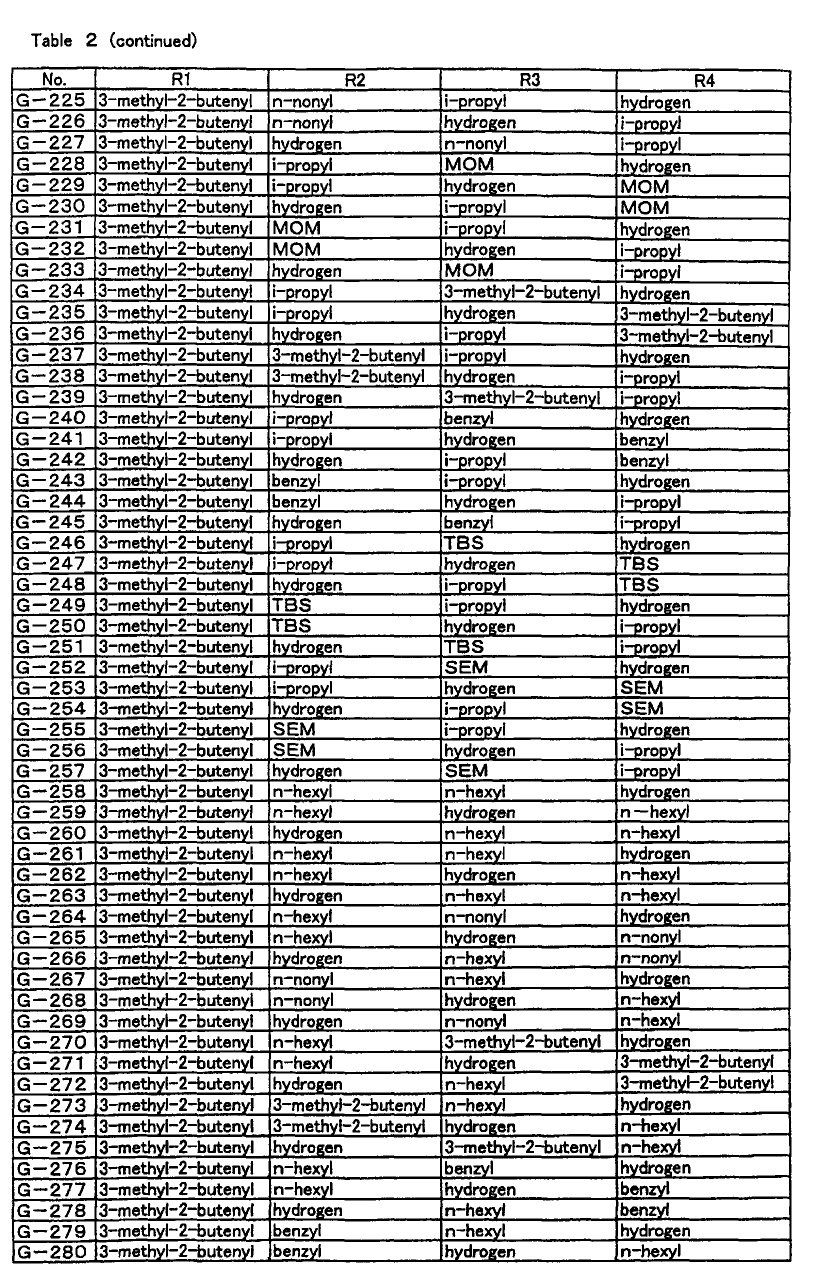

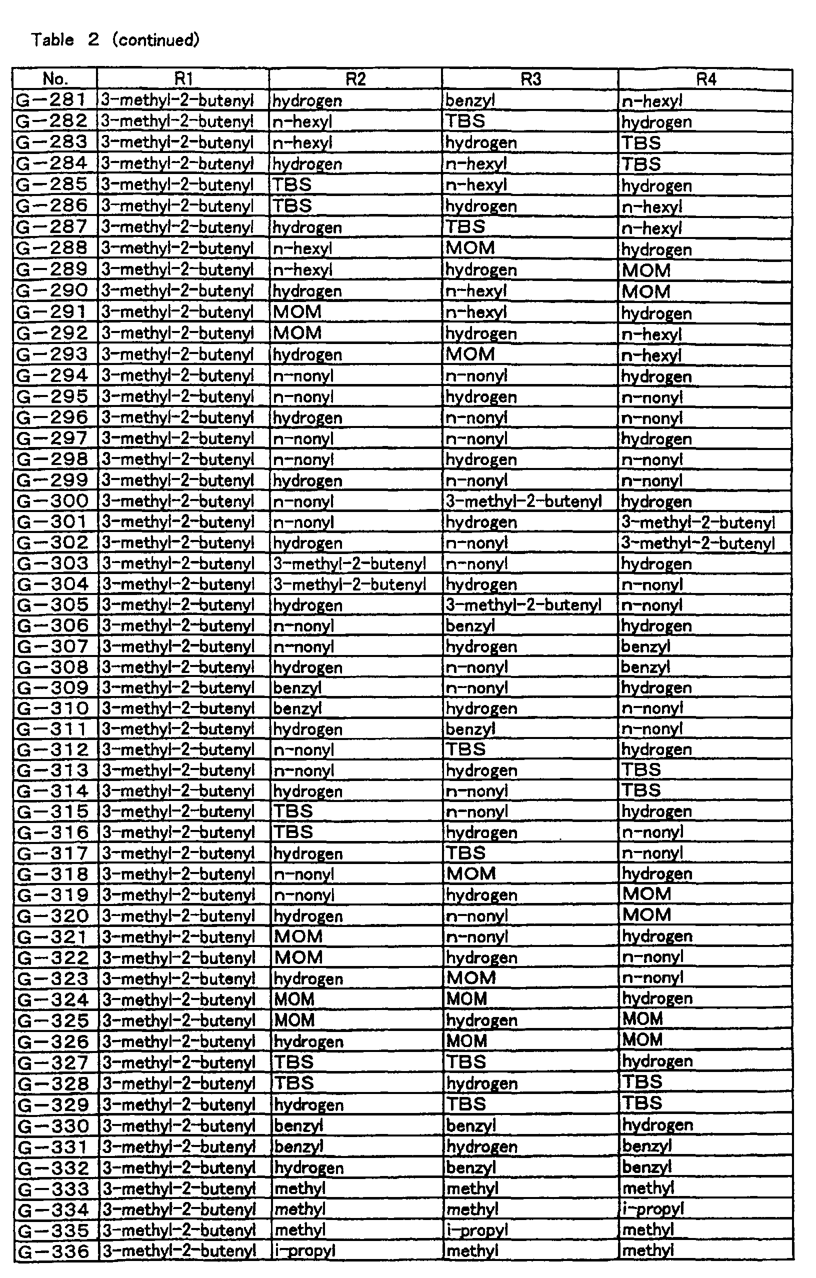

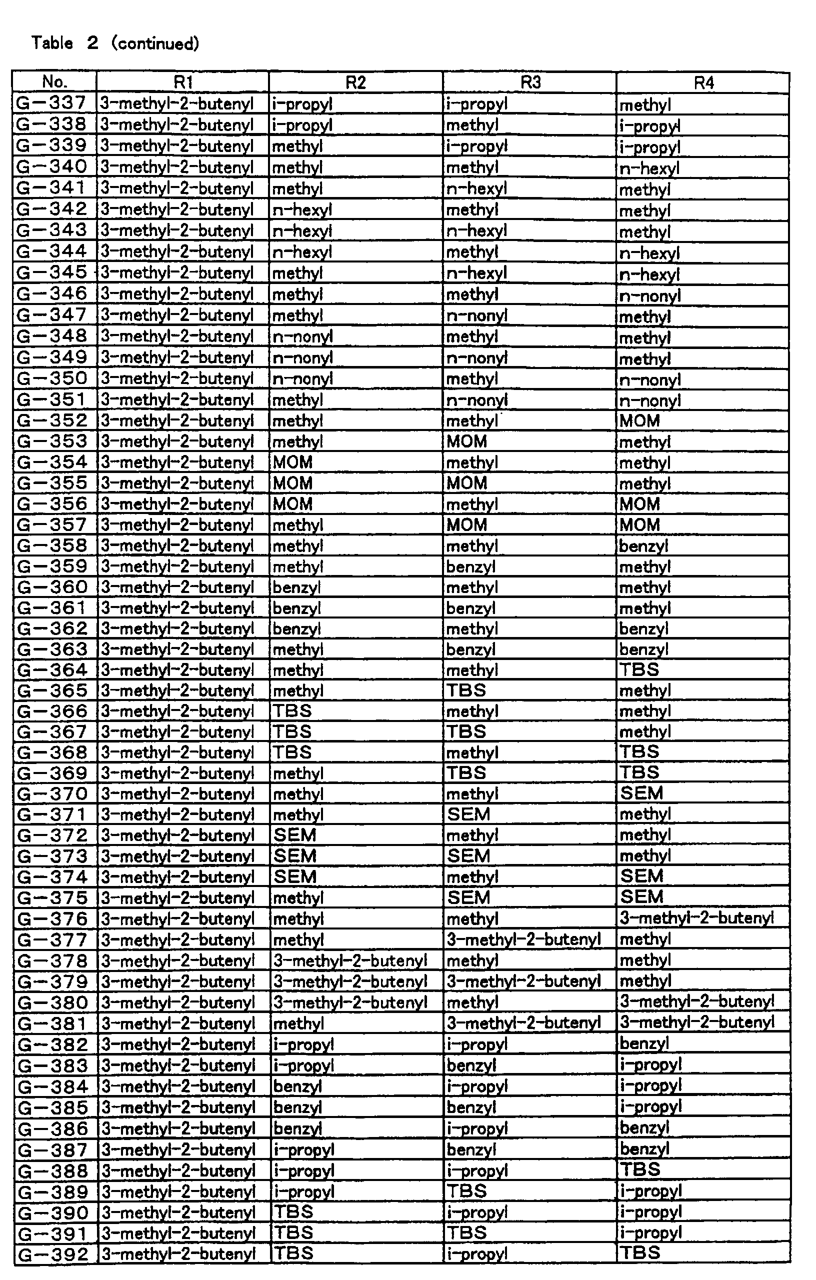

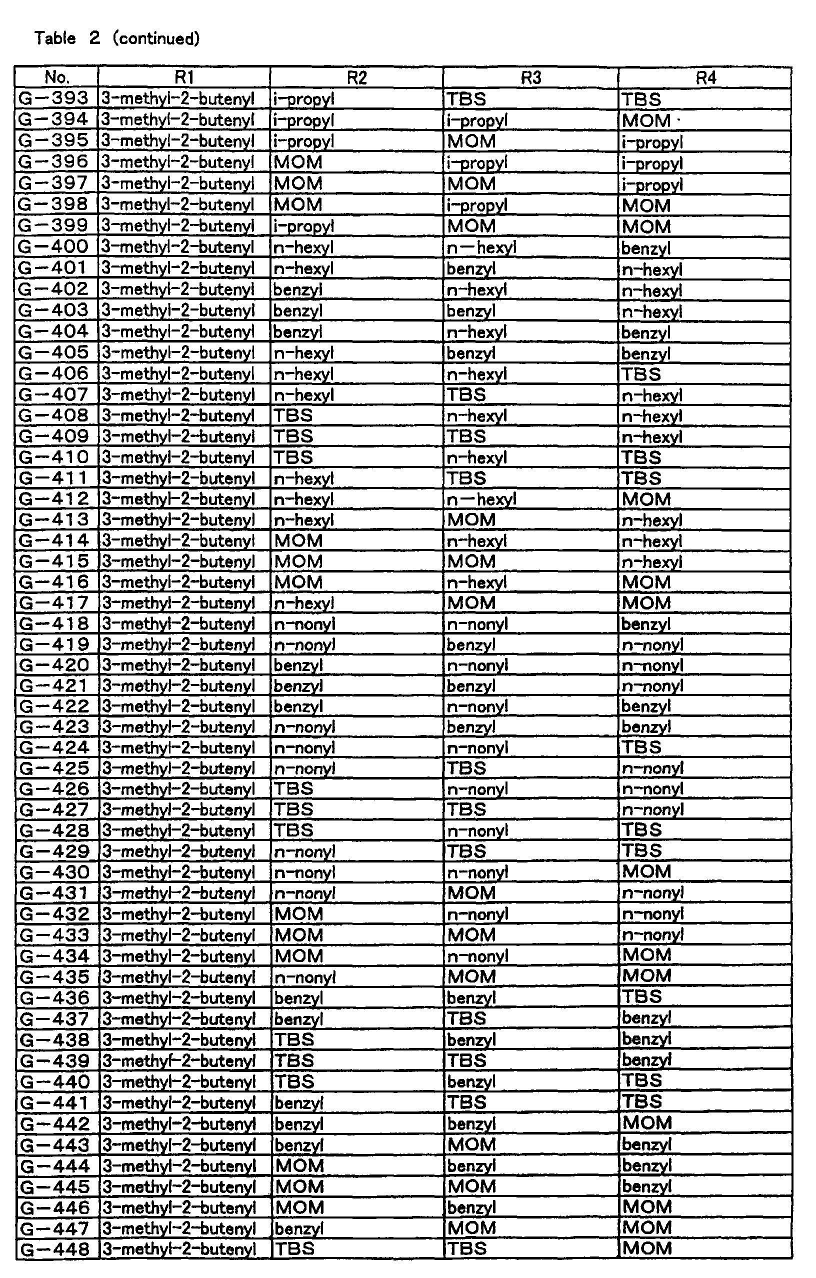

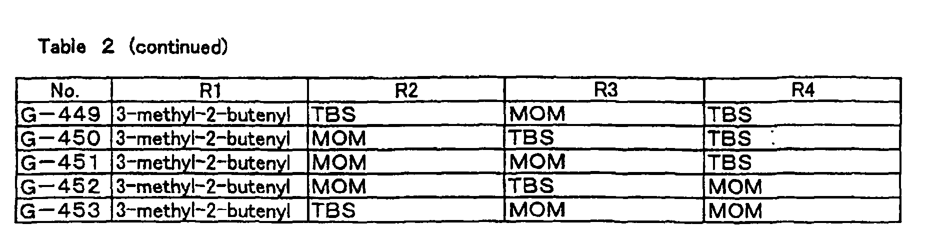

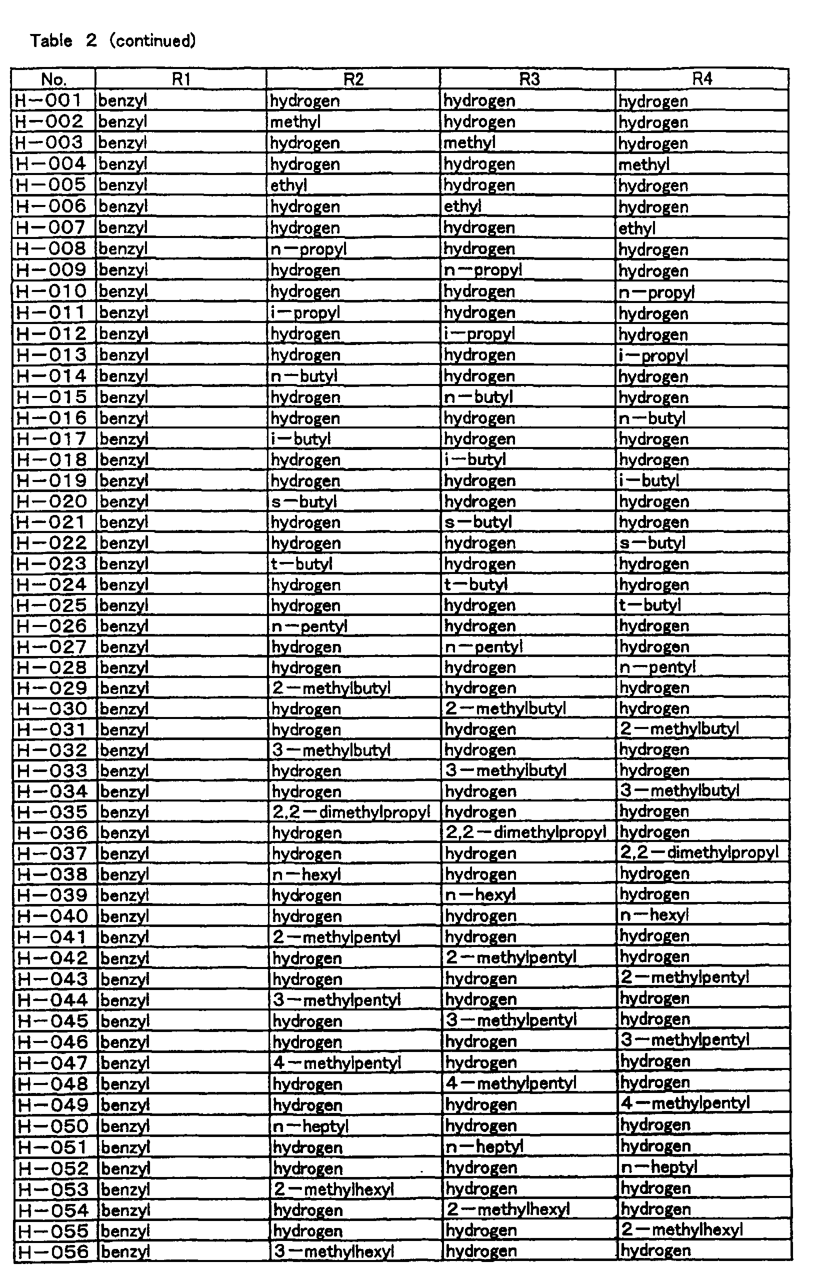

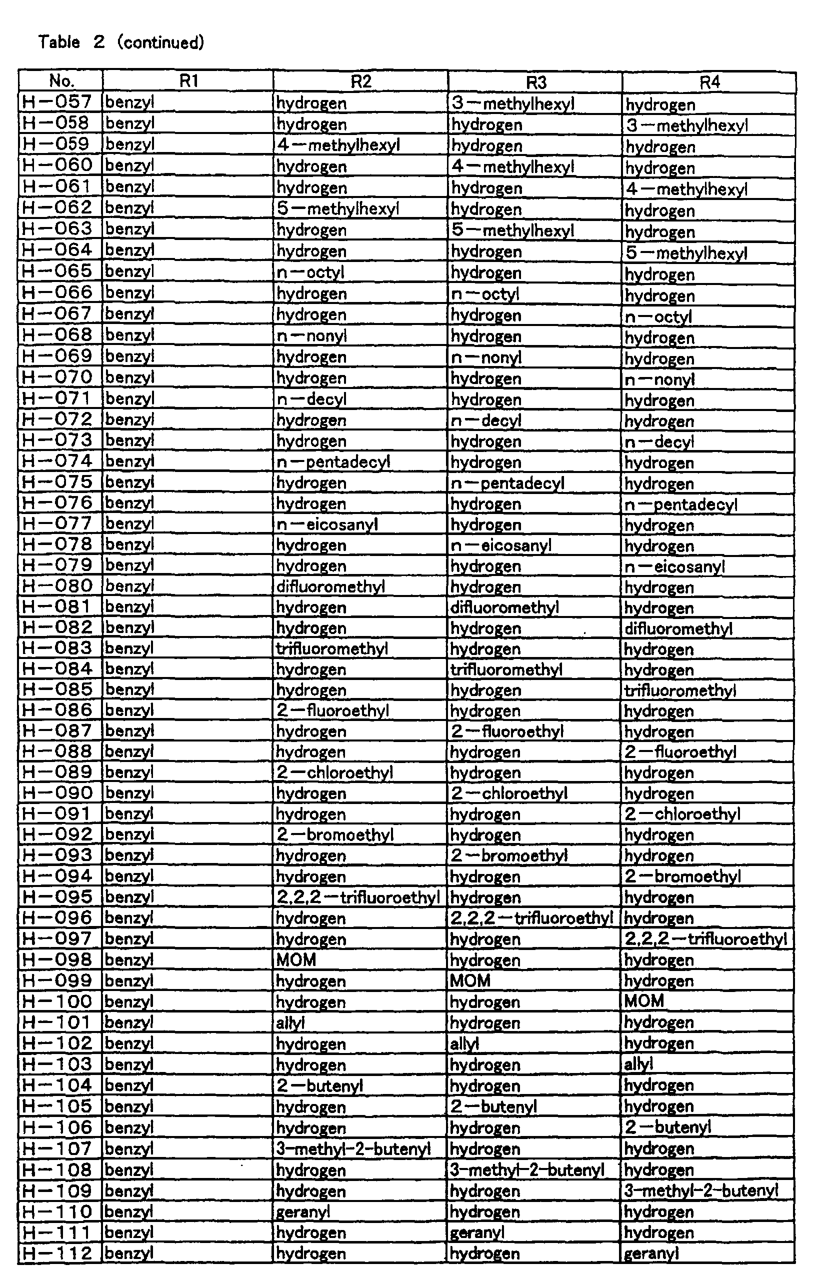

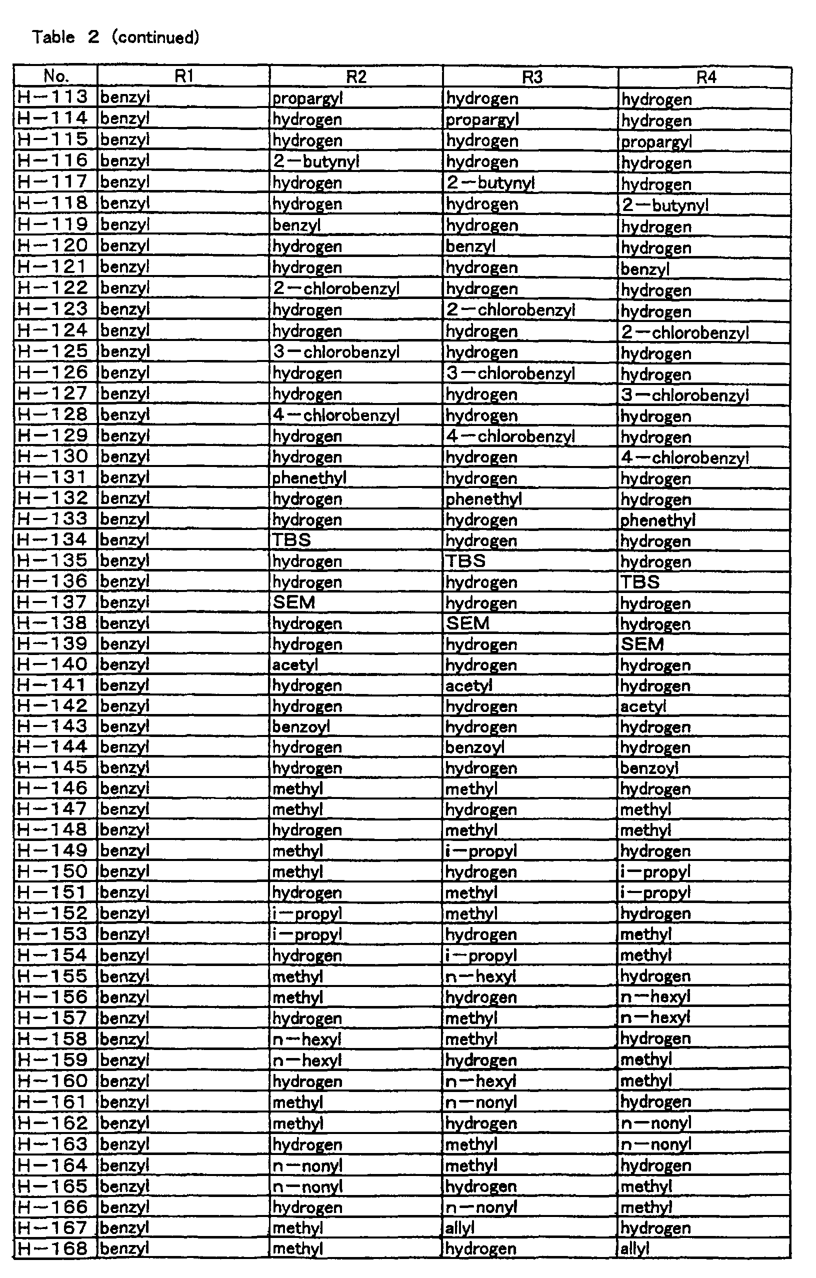

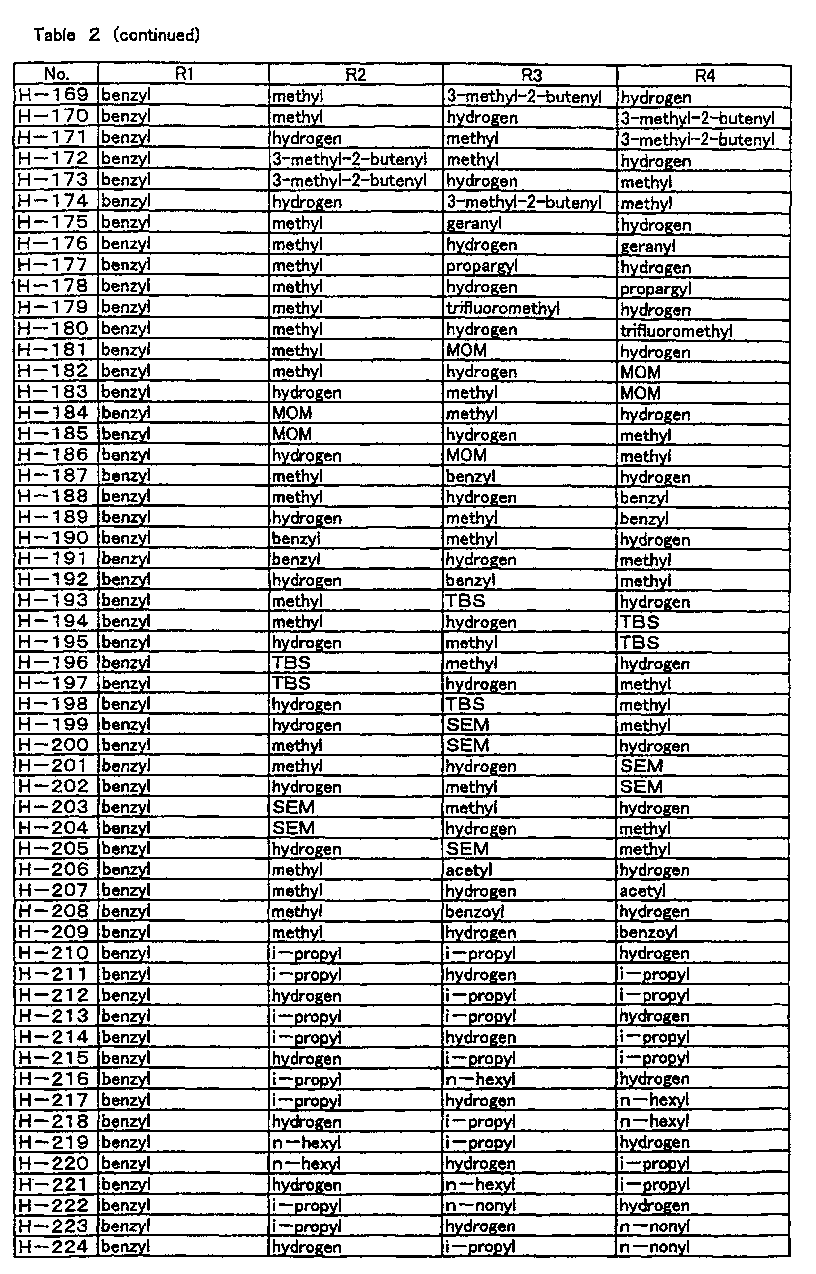

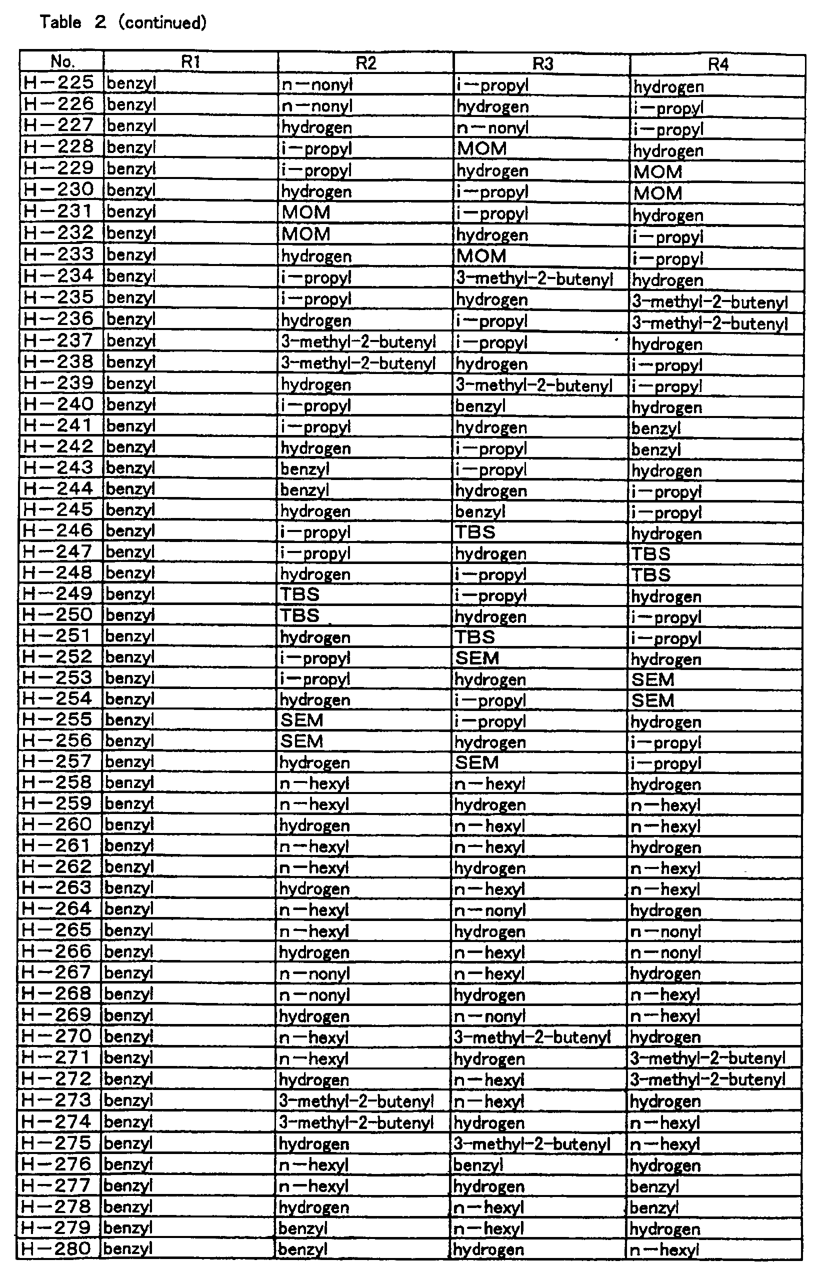

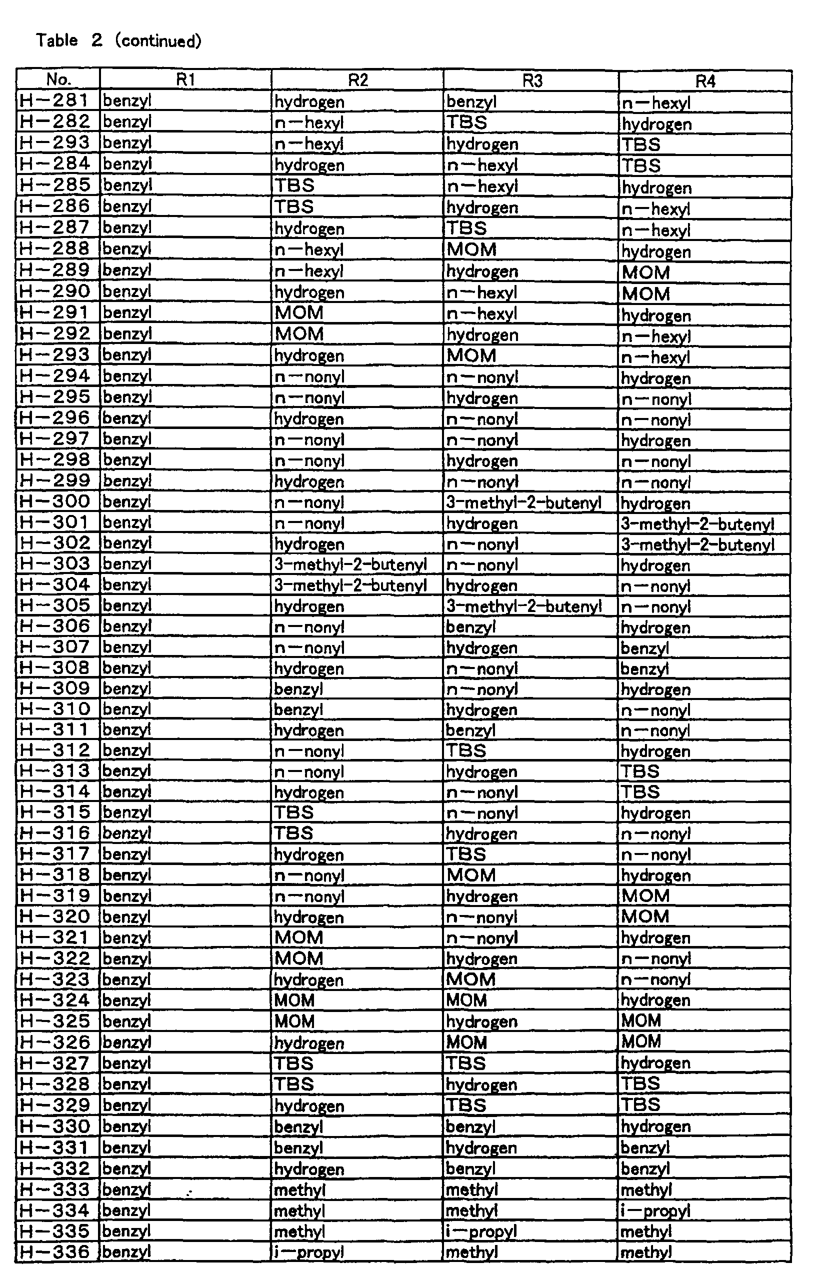

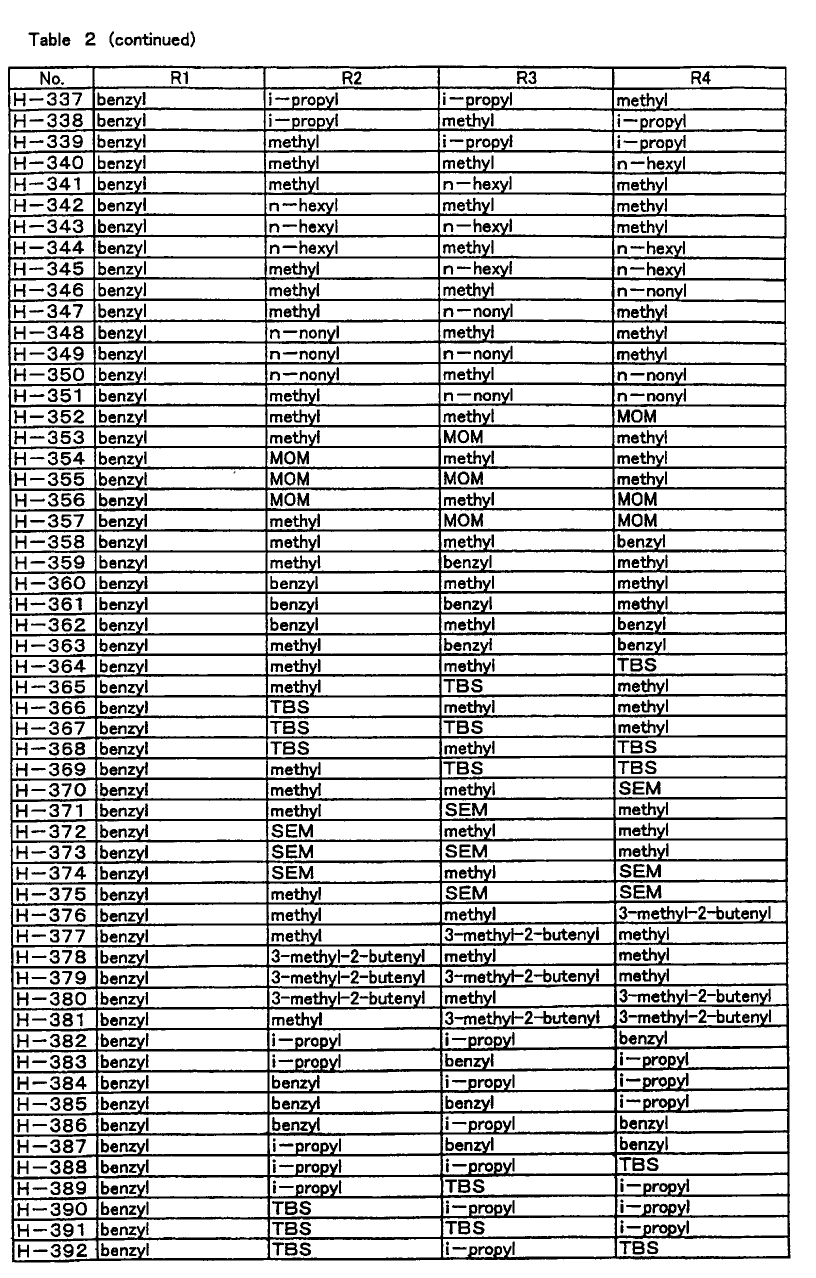

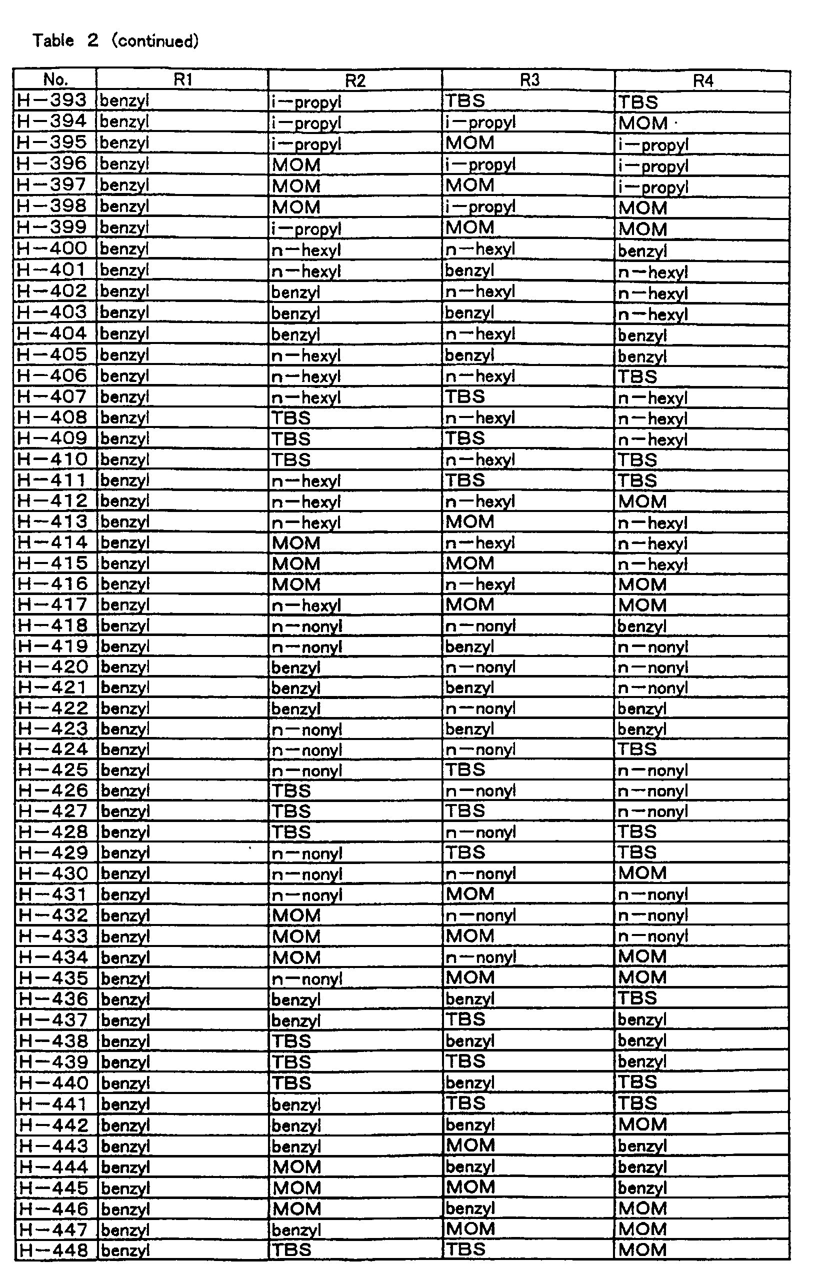

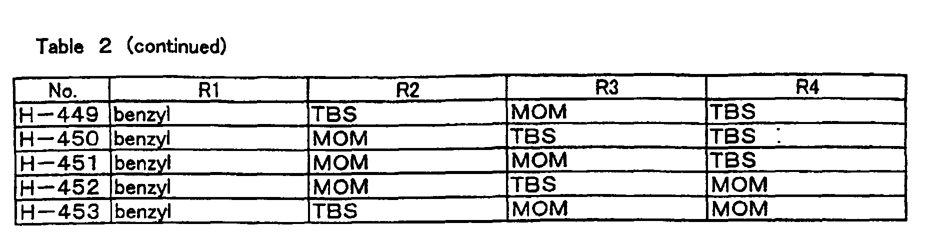

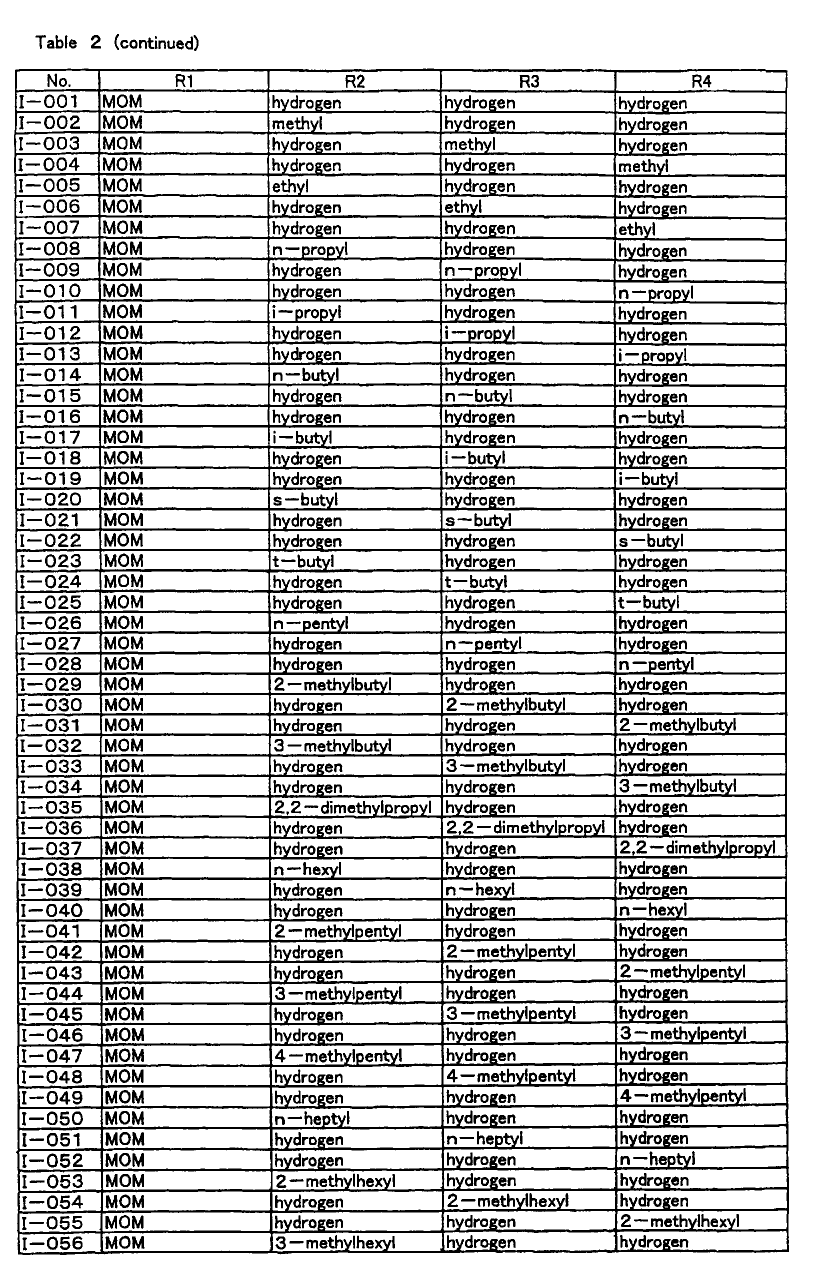

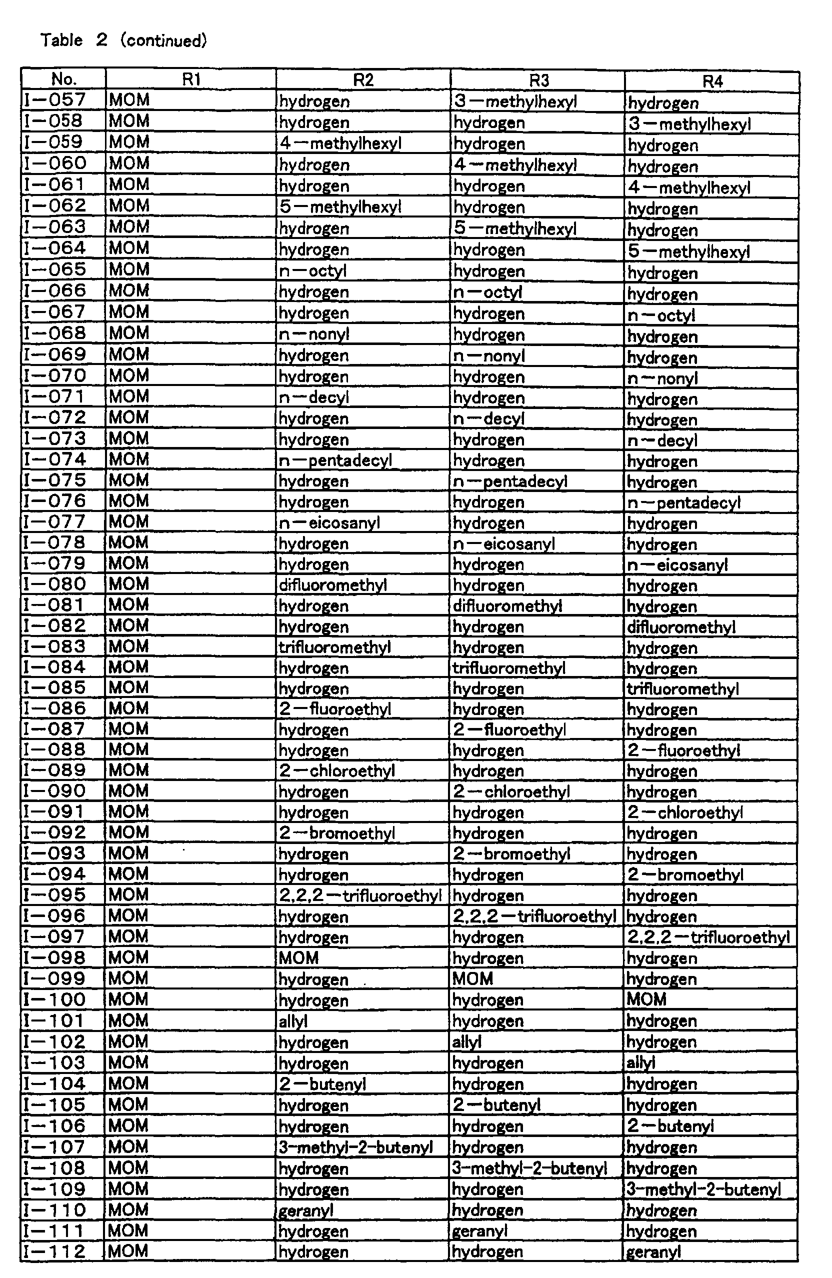

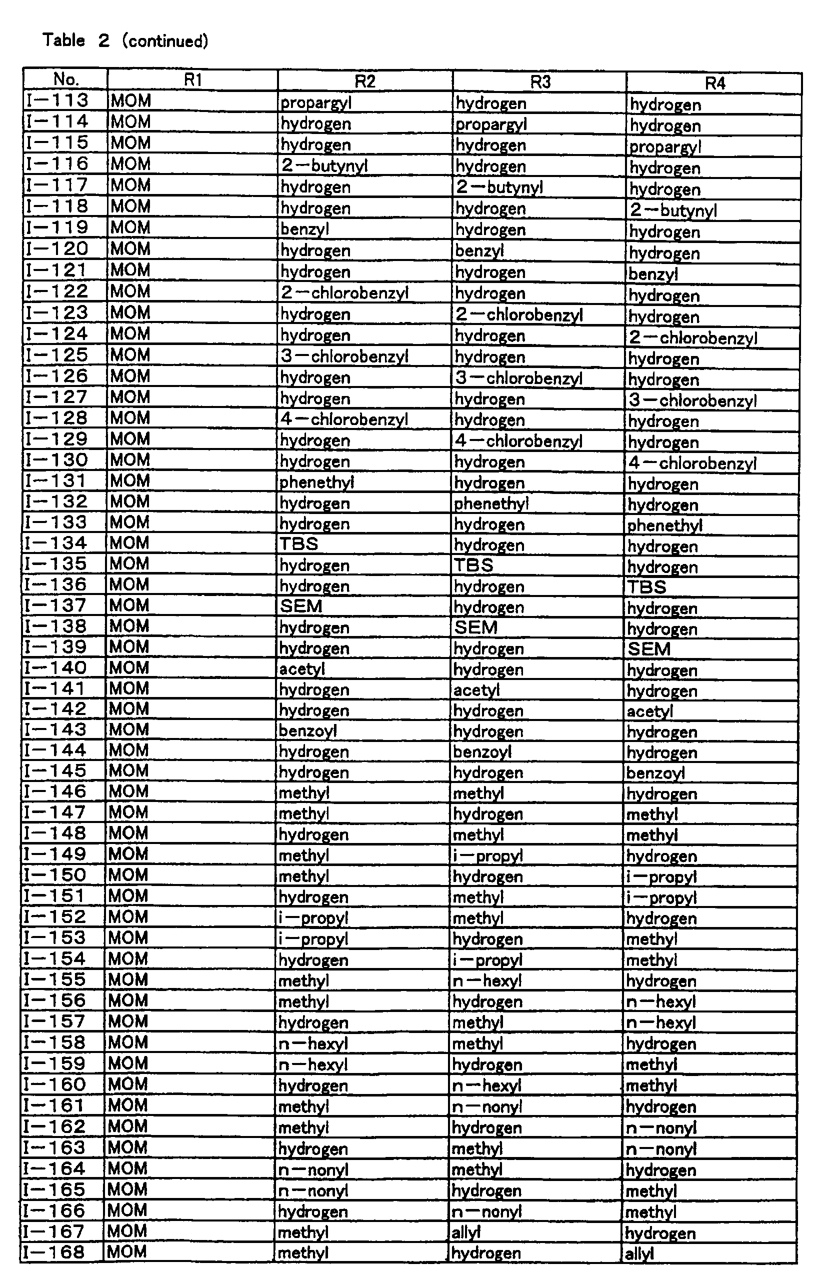

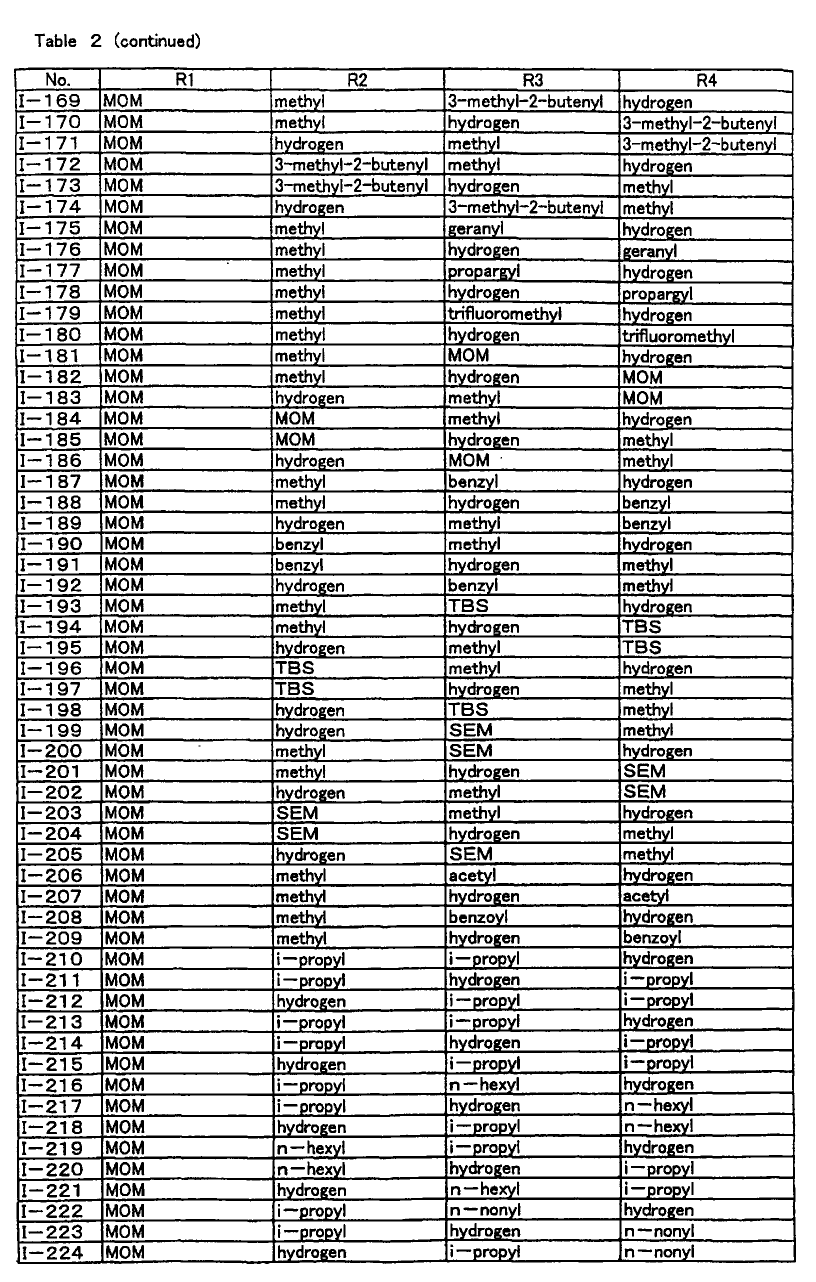

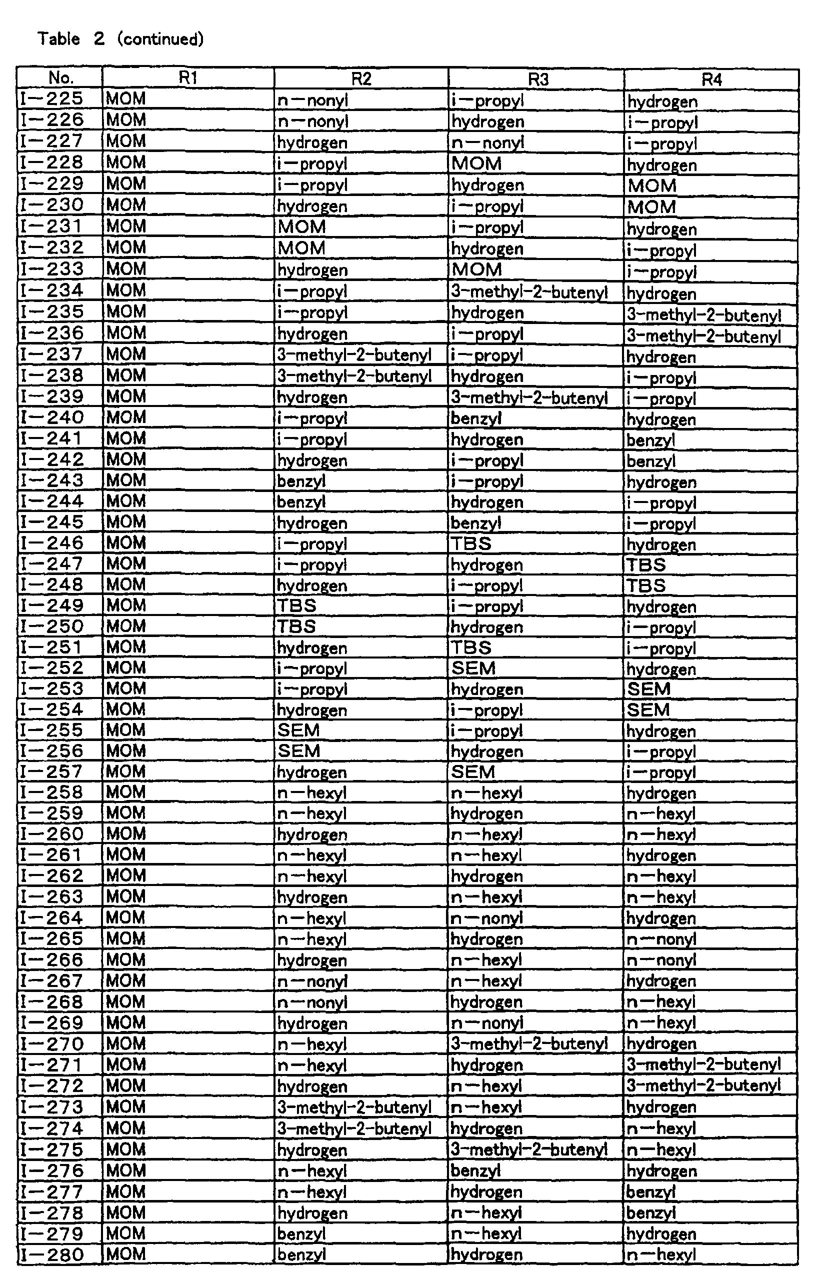

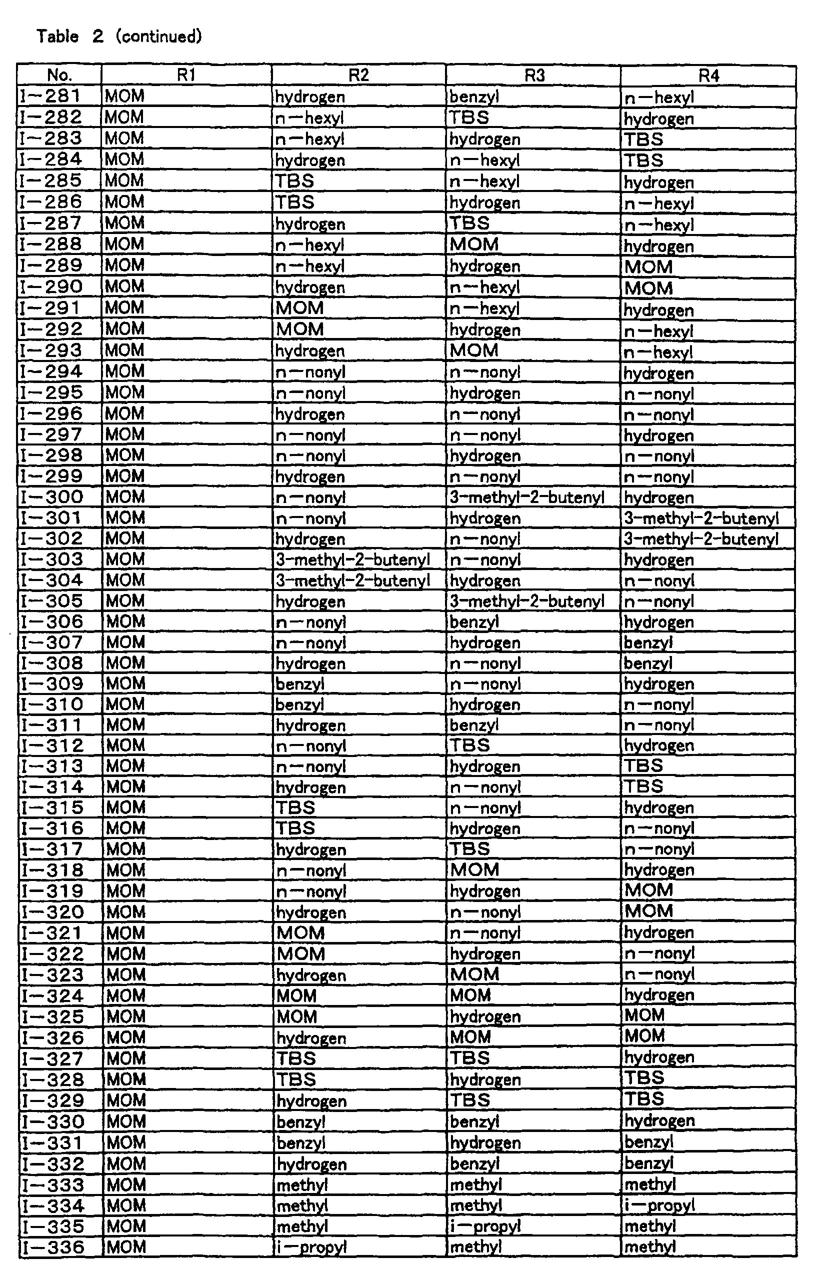

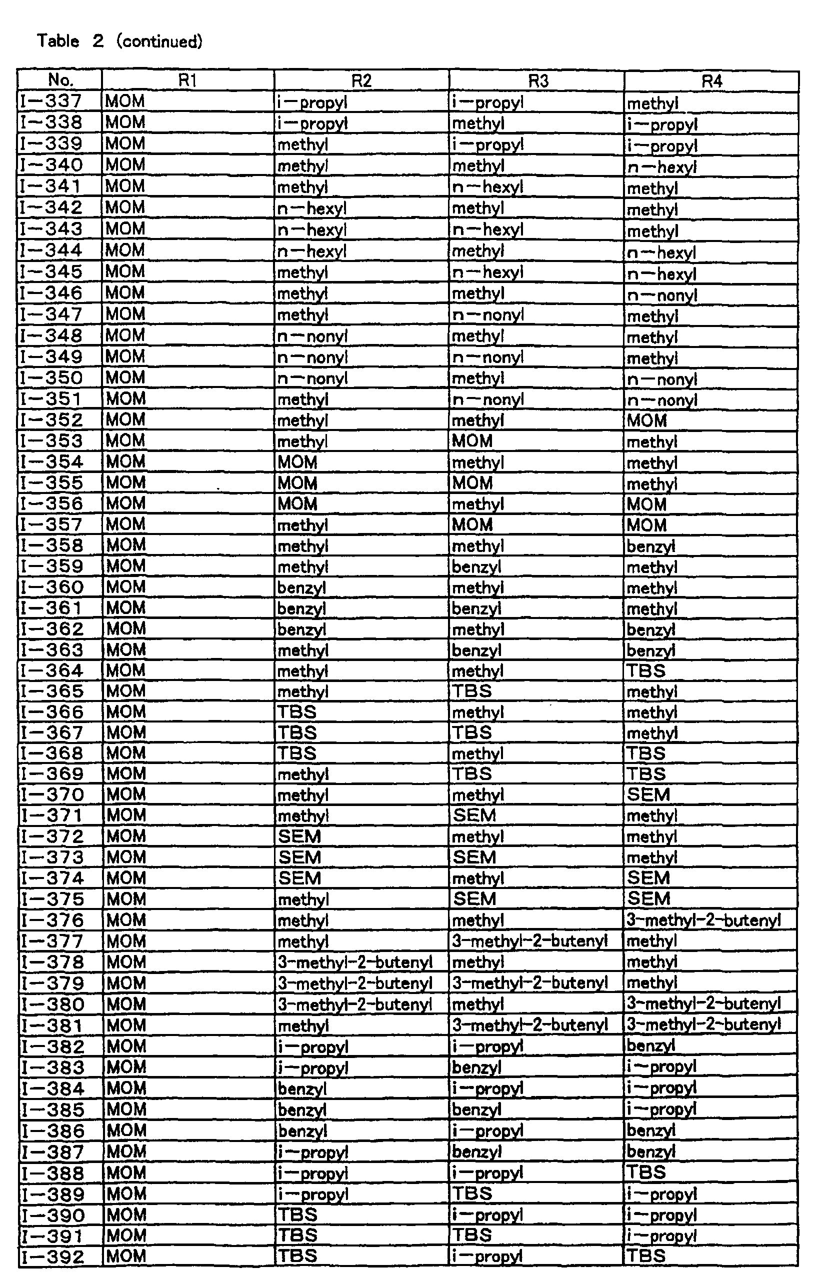

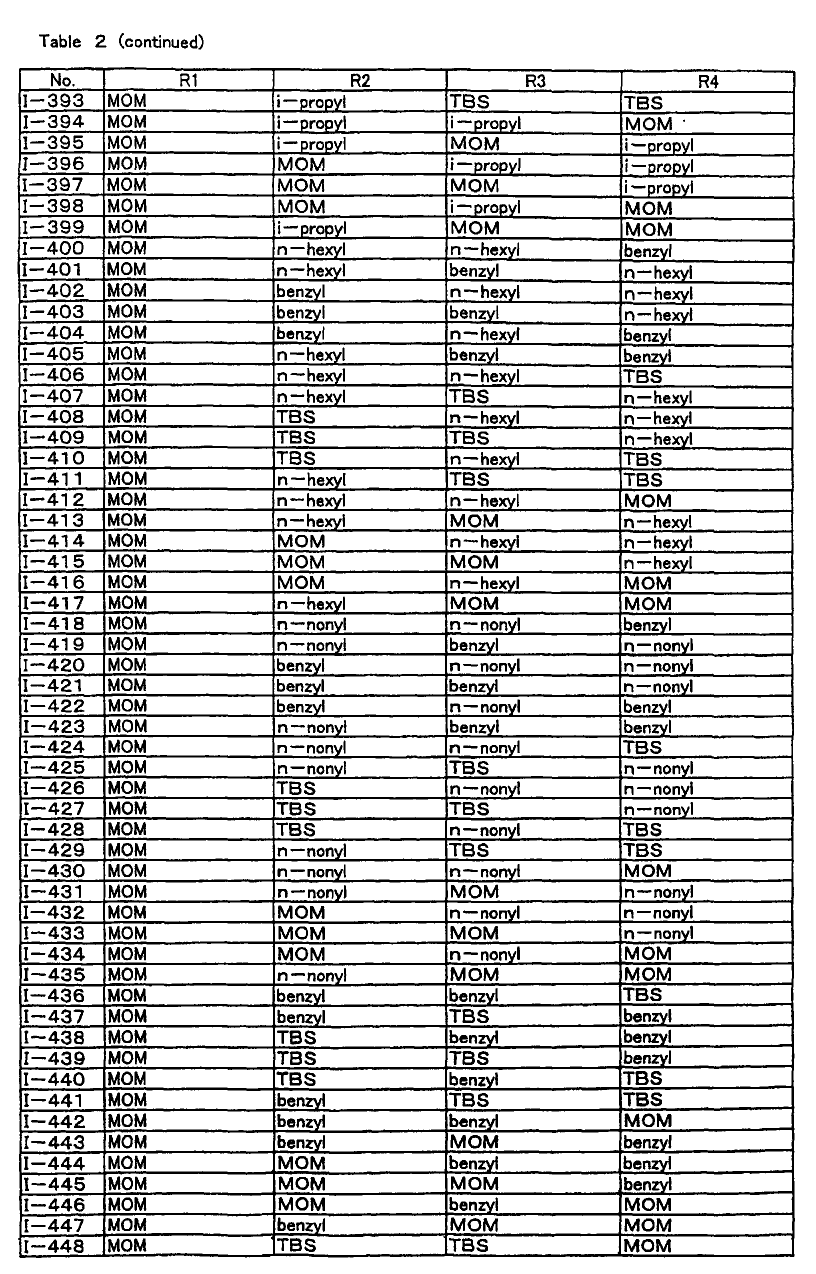

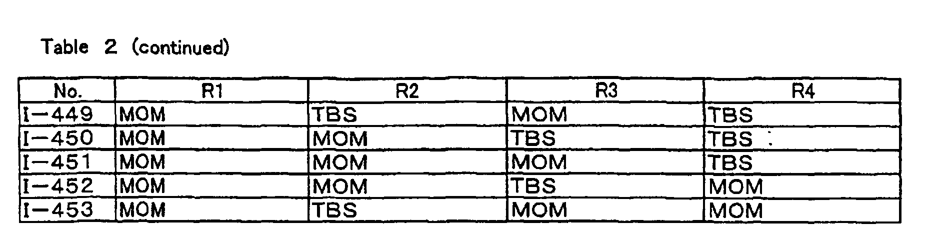

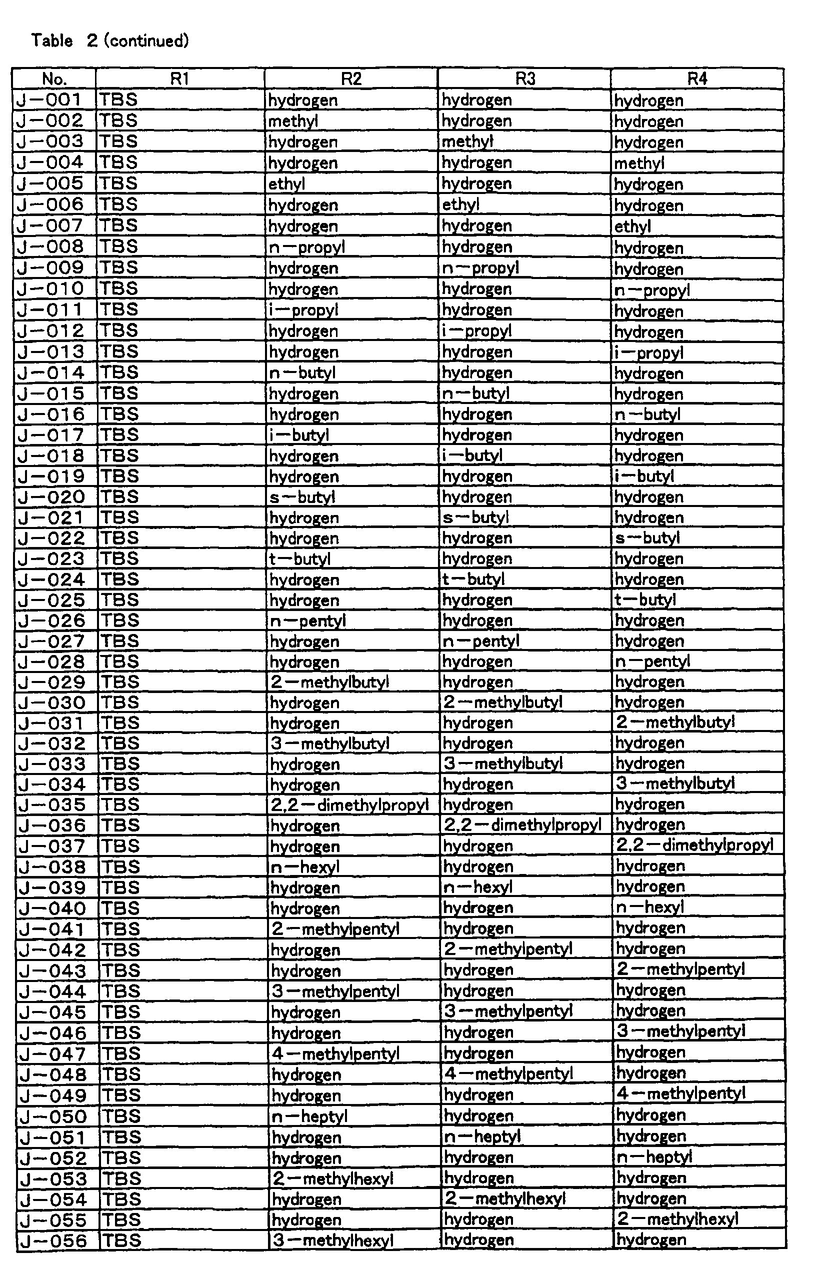

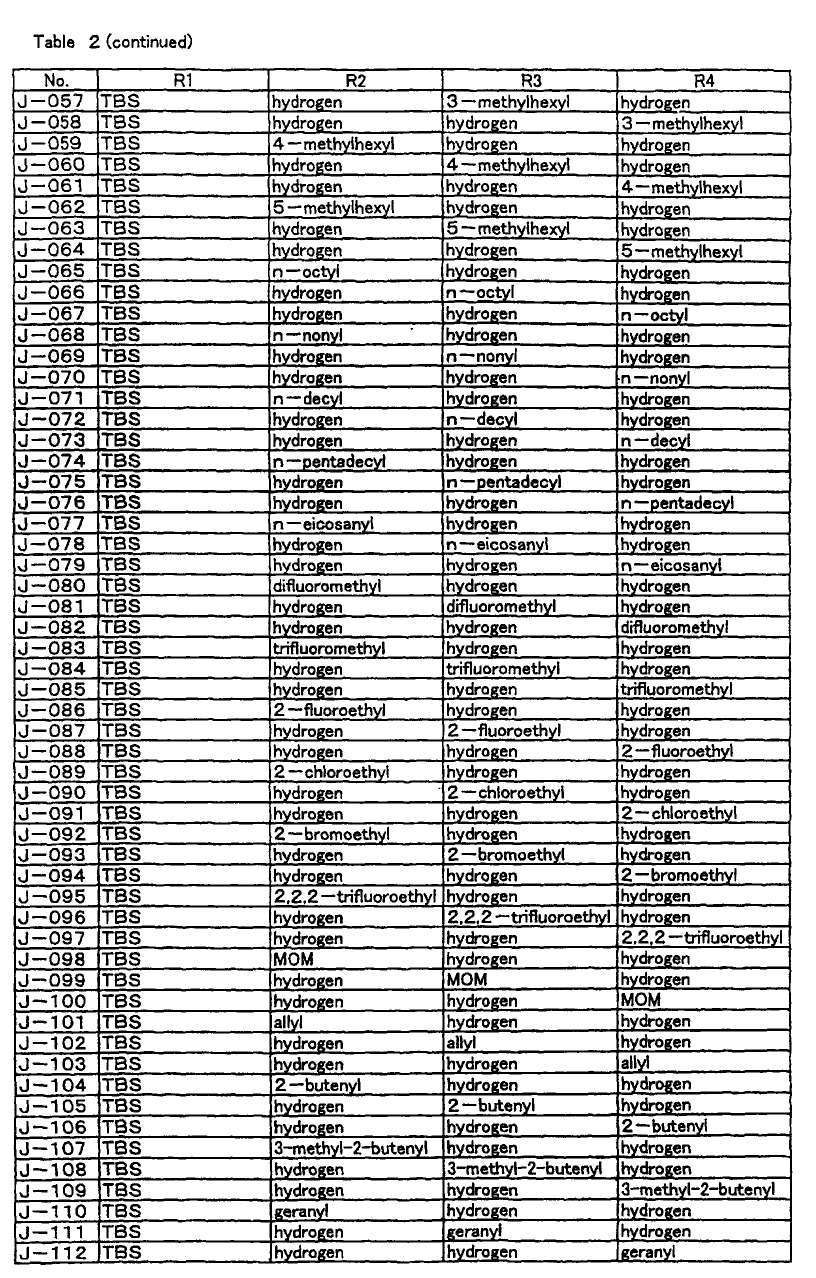

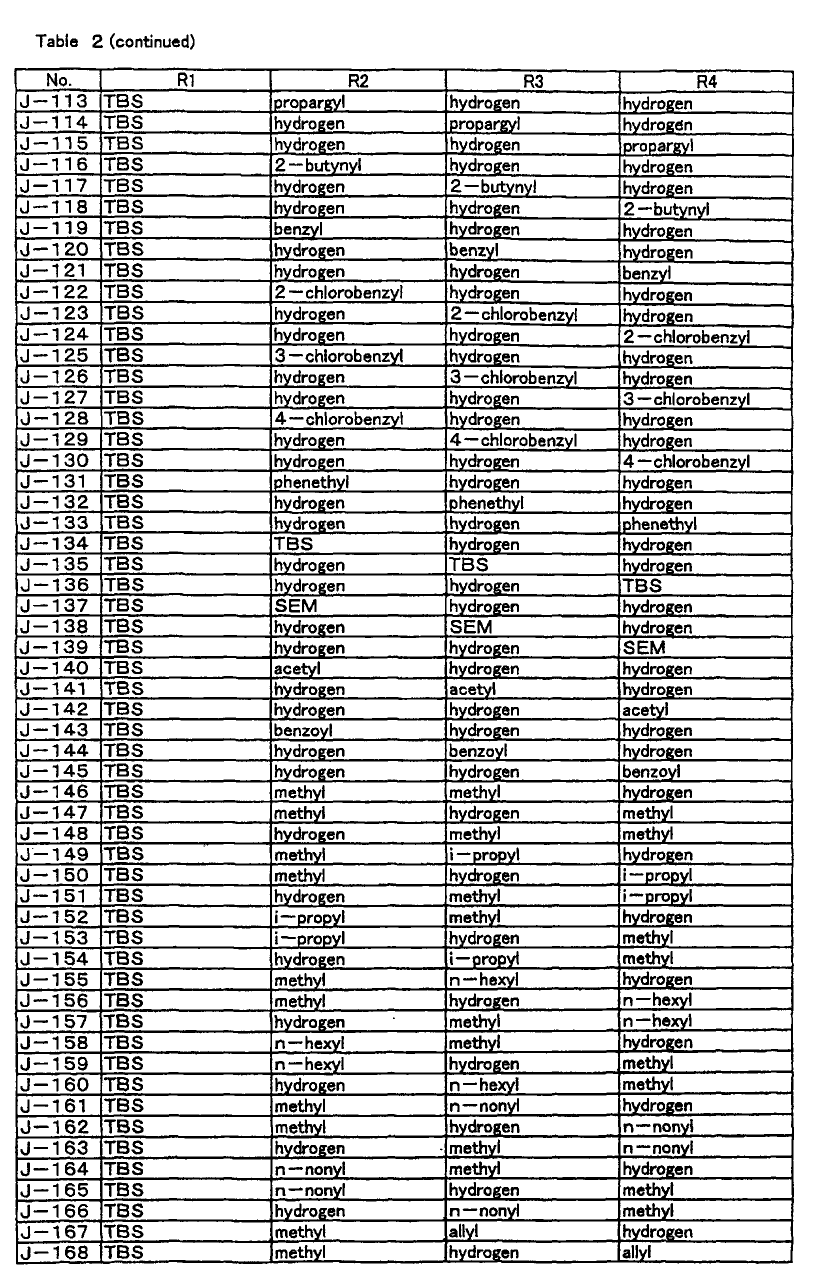

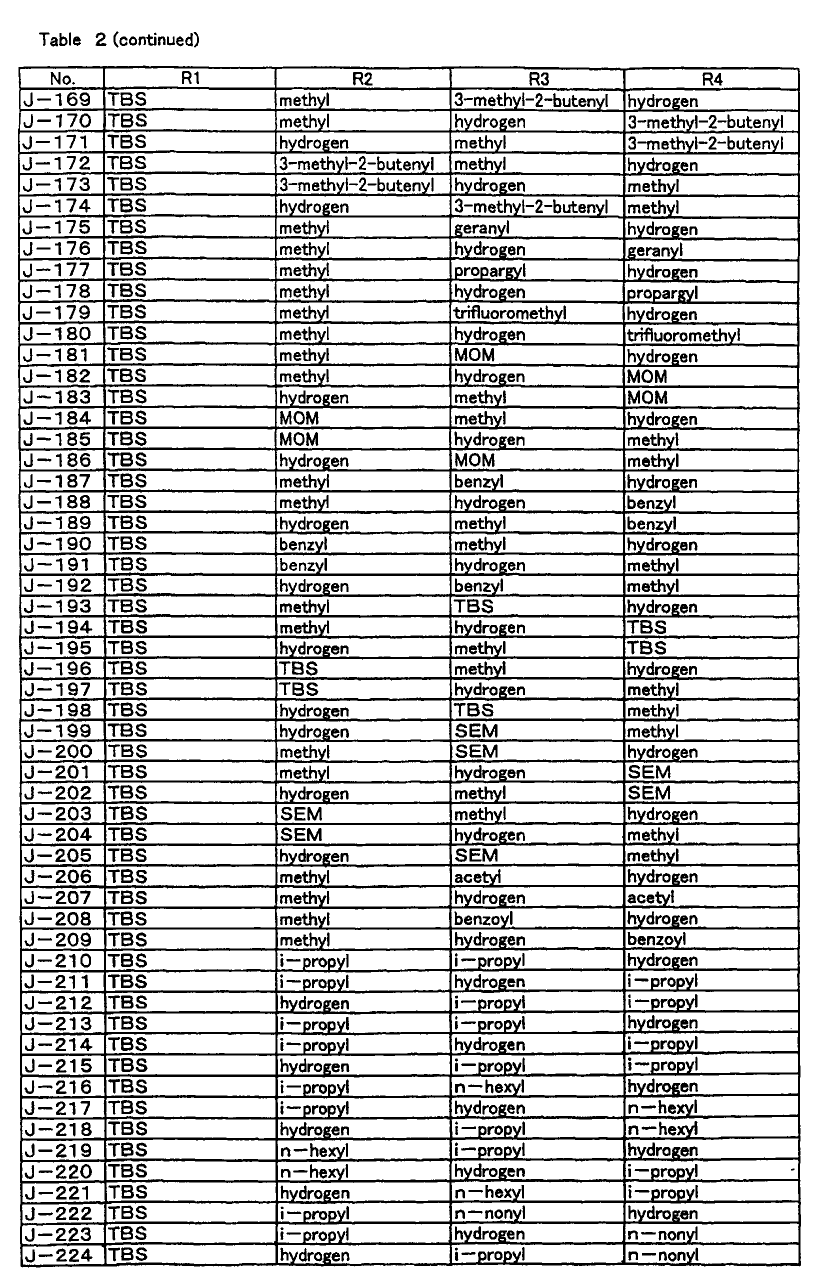

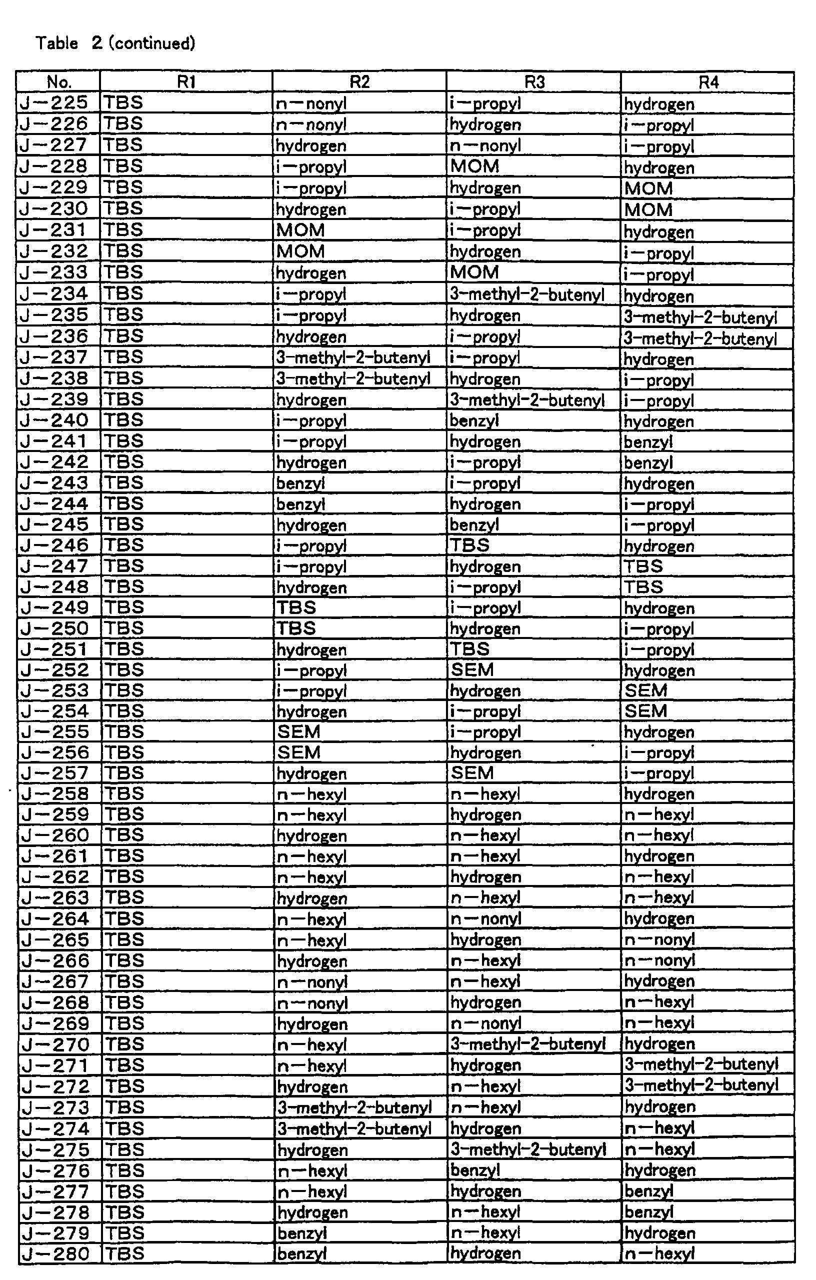

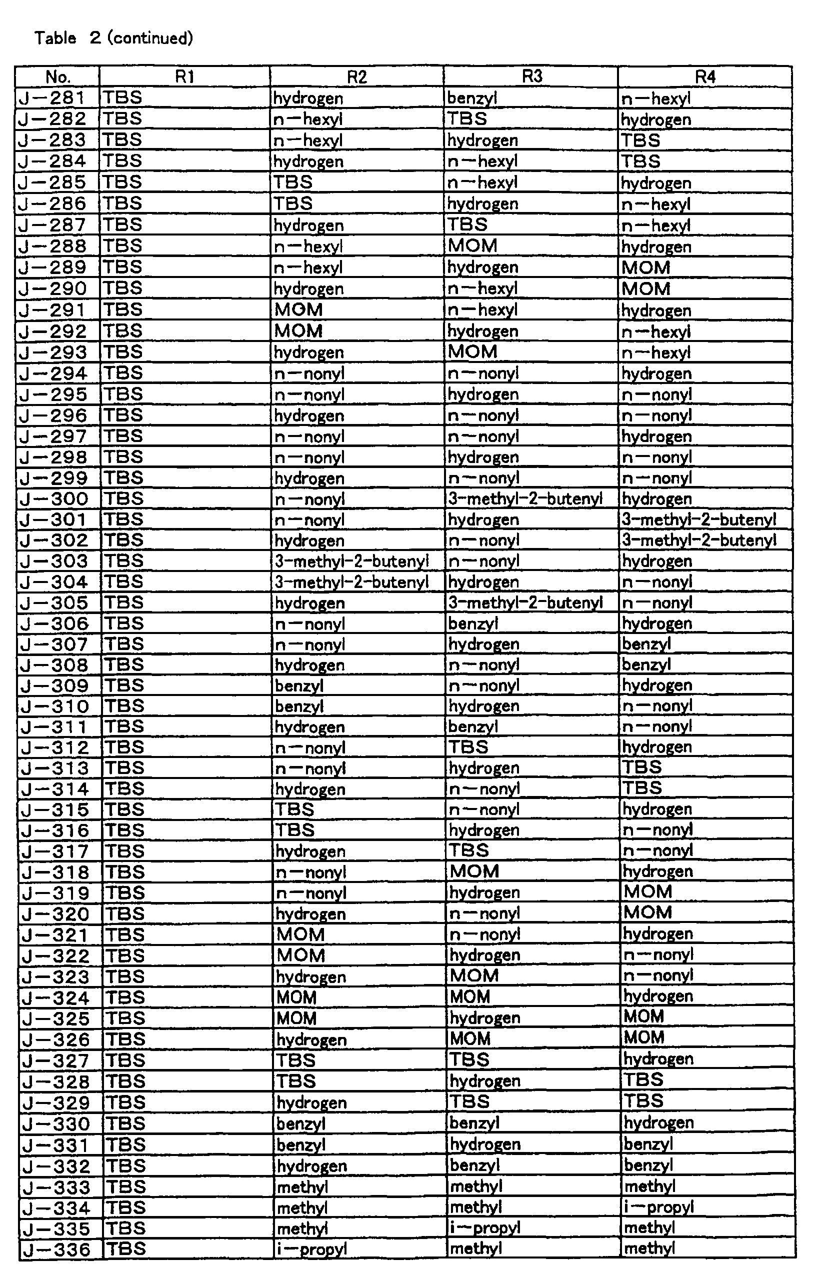

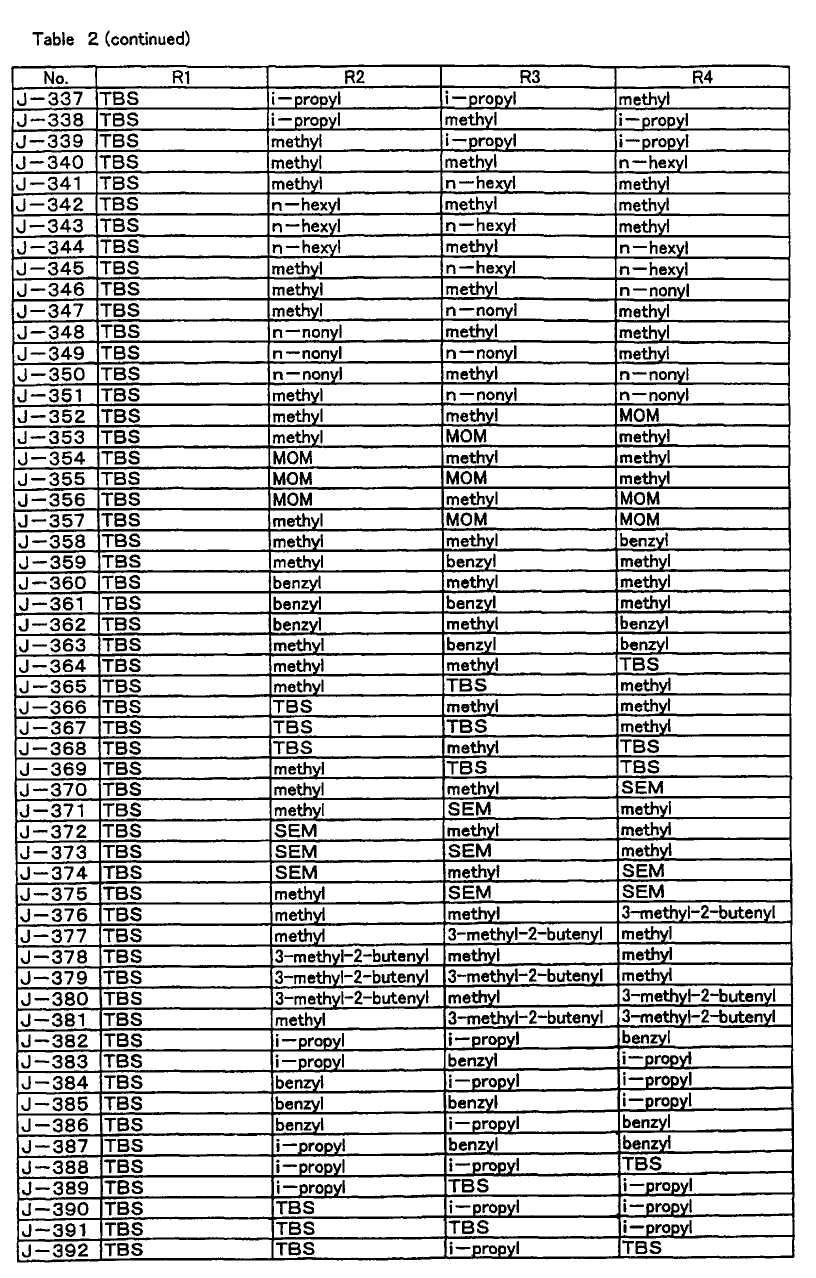

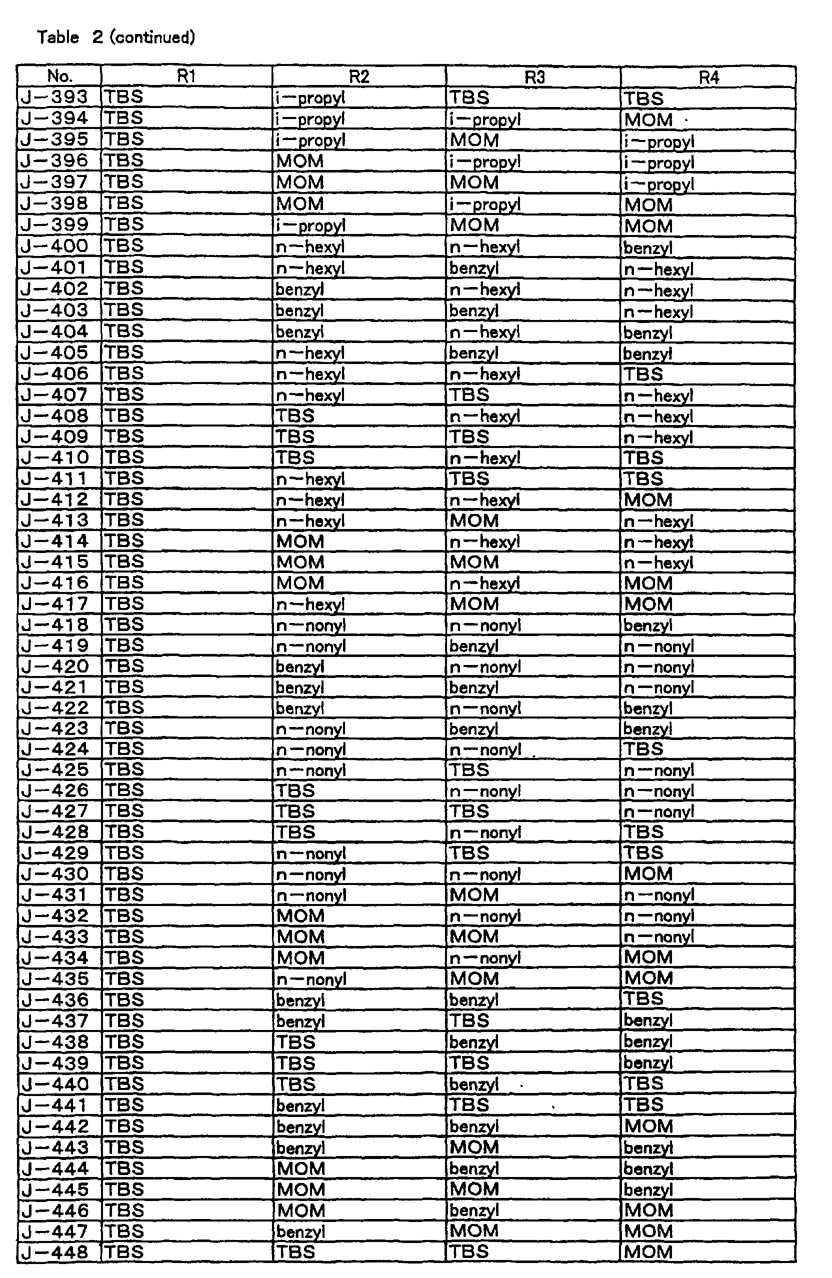

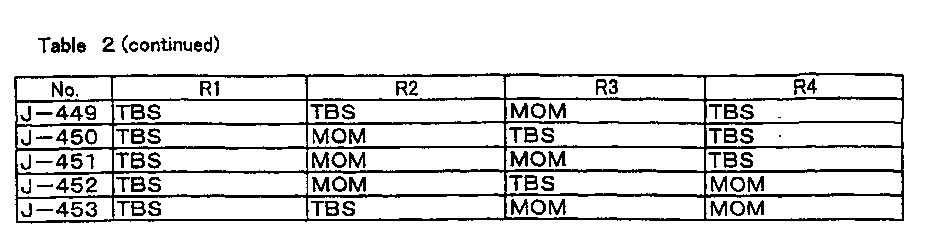

In Table 3 below, the structures of Compound Nos. A-1 to A-22 and B-1 to B-15 according to the Examples are shown. Table 3 shows preferable combinations of above substituents as well as ¹H-NMR data of compounds obtained by combinations of such substituents. Referring to the symbols used in the tables, Compound No. is indicated in the column captioned as "No."; H represents hydrogen; Me represents methyl: i-Pr represents isopropyl; TBS represents t-butyldimethylsilyl; and SEM represents 2-(trimethylsilyl)ethoxymethyl.

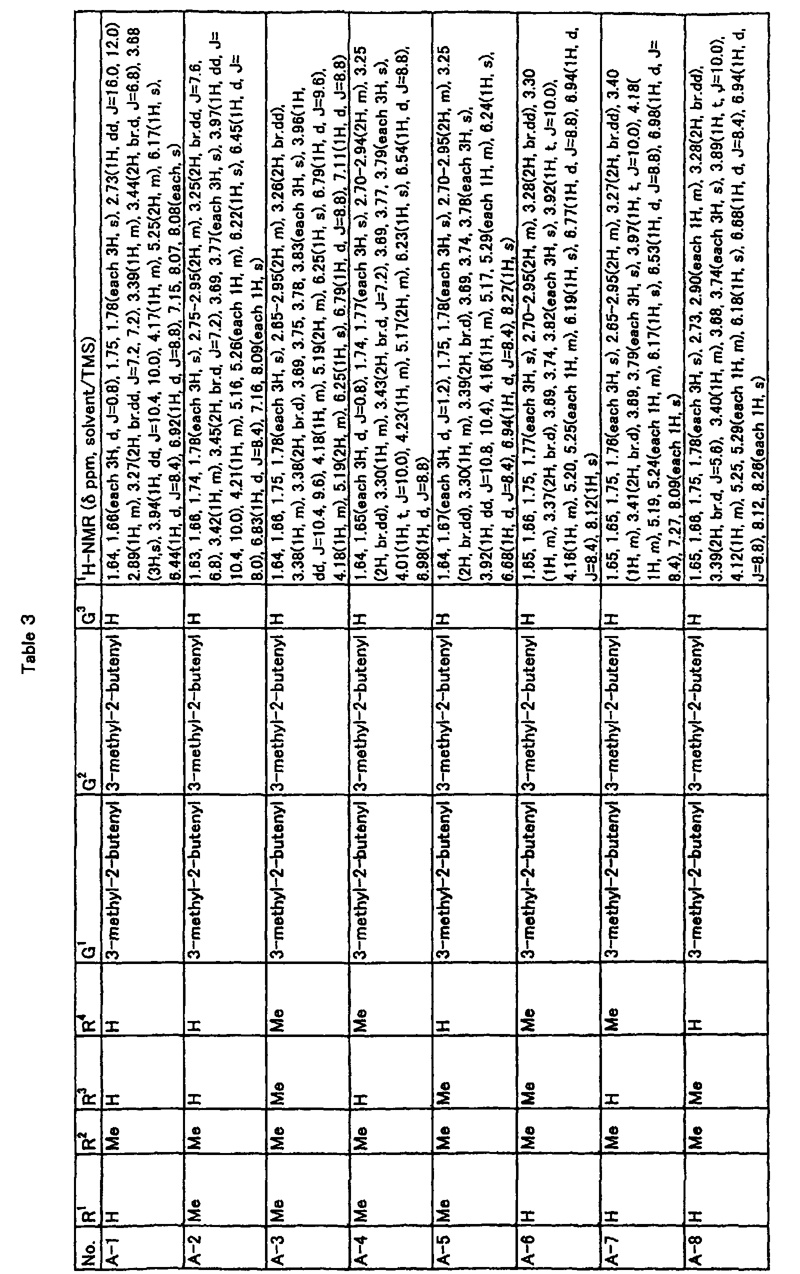

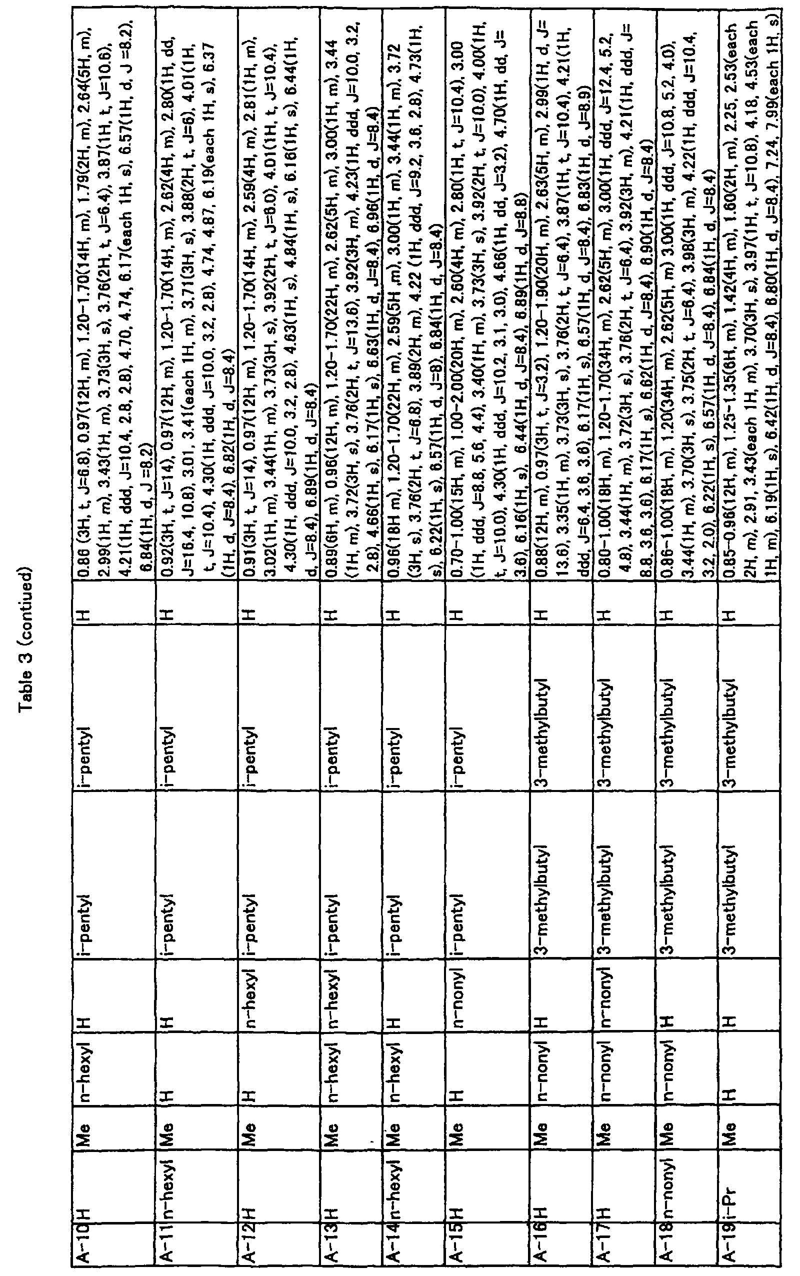

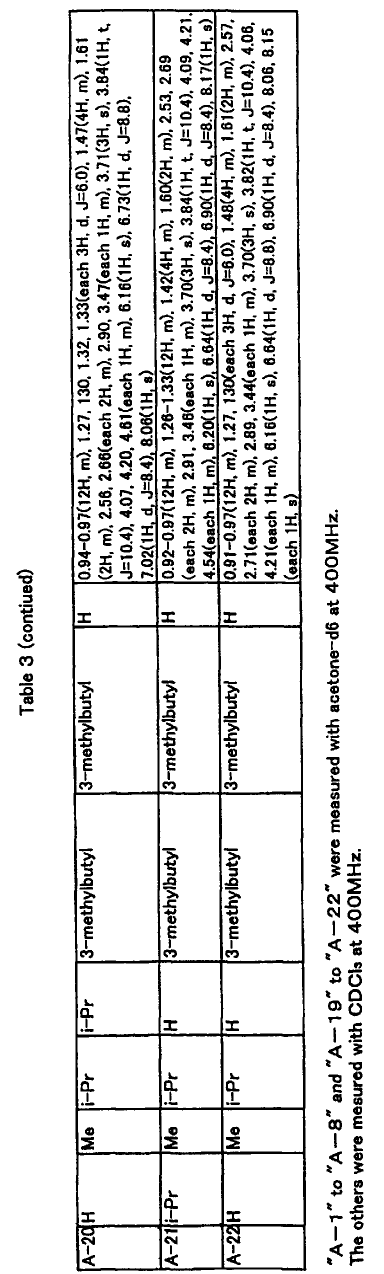

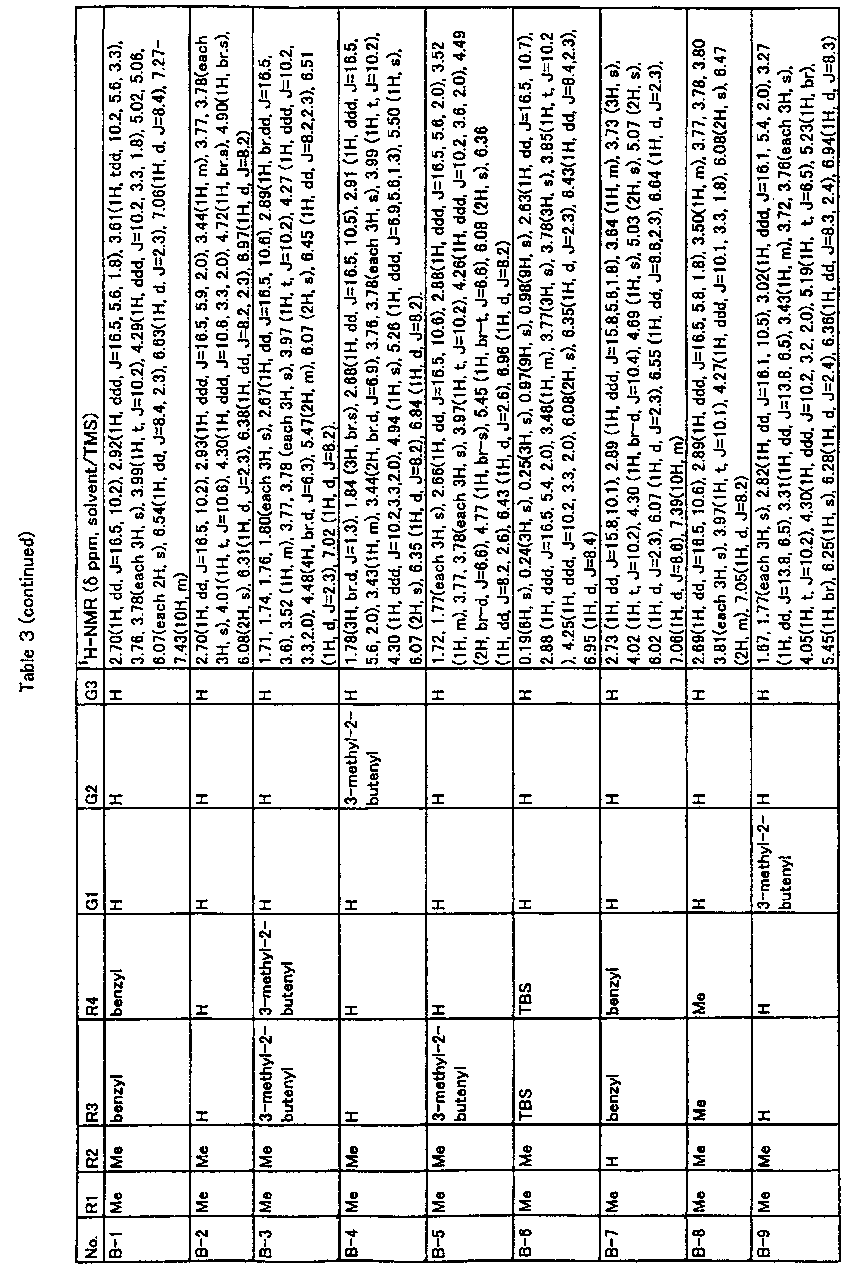

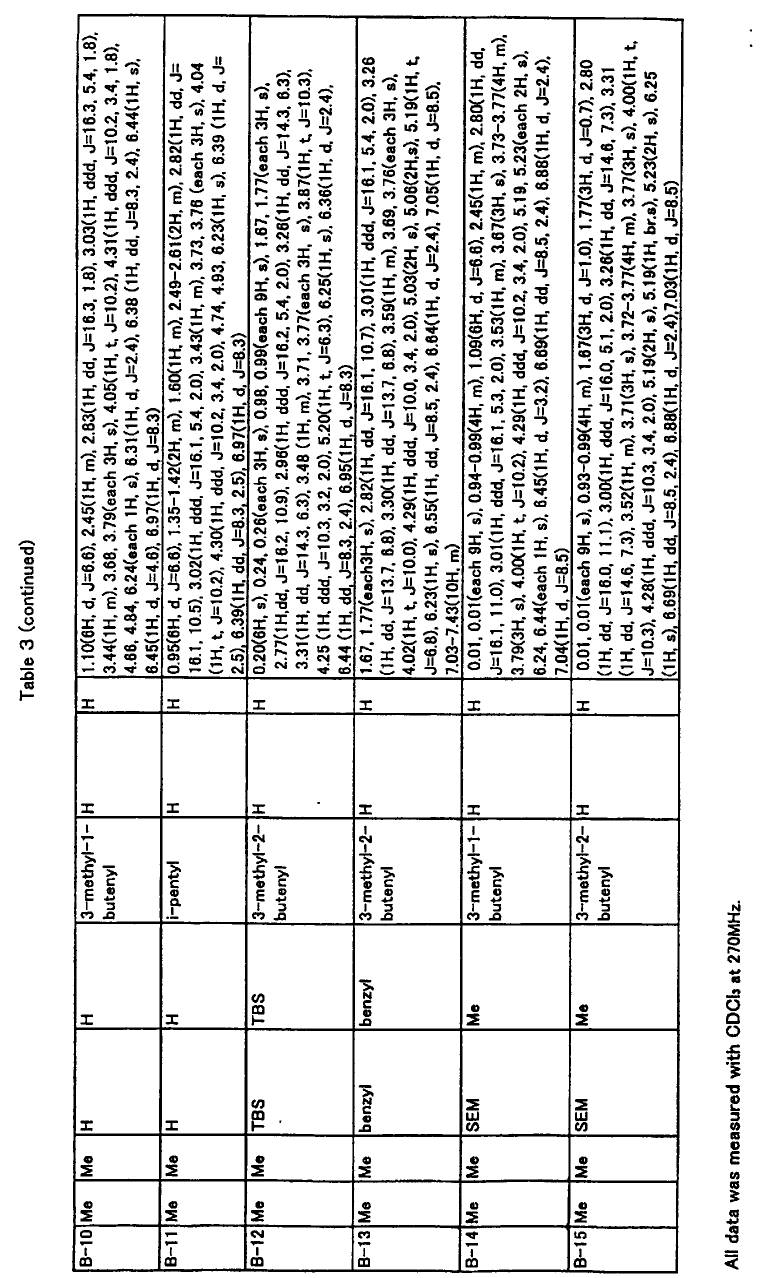

As the "salts" of the compounds of interest in the present invention, pharmacologically acceptable salts are preferable, e.g., salts formed with inorganic bases, salts formed with organic bases, salts formed with inorganic acids, salts formed with organic acids, and salts formed with basic or acidic amino acids. Examples of salts formed with inorganic bases include alkaline metal salts, e.g., sodium salts or potassium salts, alkaline earth metal salts, e.g., calcium salts, magnesium salts or barium salts, aluminum salts or ammonium salts, and the like. Examples of salts formed with organic bases include salts formed with trimethylamine, triethylamine, pyridine, picoline, ethanolamine, diethanolamine, triethanolamine, dicyclohexylamine, N,N'-dibenzylethylenediamine, and the like. Examples of salts formed with inorganic acids include salts formed with hydrochloric acid, hydrofluoric acid, hydrobromic acid, nitric acid, sulfuric acid, phosphoric acid, perchloric acid, hydroiodic acid, and the like. Examples of salts formed with organic acids include salts formed with formic acid, acetic acid, trifluoroacetic acid, fumaric acid, oxalic acid, tartaric acid, maleic acid, citric acid, succinic acid, malic acid, mandelic acid, ascorbic acid, lactic acid, gluconic acid, methanesulfonic acid, p-toluenesulfonic acid, benzenesulfonic acid, and the like. Examples of salts formed with basic amino acids include salts formed with arginine, lysine, ornithine, and the like. Examples of salts formed with acidic amino acids include salts formed with aspartic acid, glutamic acid, and the like.

As the "hydrates" of the compounds of interest in the present invention, pharmacologically acceptable hydrates are preferable, including water-containing salts. Specific examples include monohydrates, dihydrates, hexahydrates, and the like.

An "immunoactivating action" means enhancement of immune functions, that is, enhancing biophylaxis functions against viral or bacterial infections (including secondary infections) (e.g., opportunistic infections or intractable infections), enhancing the deteriorated immune functions of patients with malignant tumors to immunologically attack cancerous cells for treatment, or otherwise activating the biophylaxis functions against diseases associated with immunodeficiency such as disorders due to radiation. Since they are also effective against immune deterioration due to the administration of carcinostatic agents, the compounds according to the present invention can be suitably used as immunoactivators (excluding carcinostatic agents).

Quantitative measurement of immunoactivating actions can be achieved by measuring the blastogenesis rate or cellular metabolism activity, for example.

The blastogenesis rate can be measured by, for example, measuring actions on the blastogenesis of mouse spleen cells. Specifically, the following measurement method is possible:

Serial two-fold dilutions (100 µl/well) with 10% FBS of test compounds which are dissolved in a dimethyl sulfoxide (DMSO) solution are obtained on a 96-well microplate, and used as test samples.

The spleen of a BALB/c mouse is aseptically isolated, and gently ground on a wire mesh with dropwise addition of sterilzed saline. The filtrate is passed through a nylon mesh (Becton Dickinson; pore size: 70 µm), thereby preparing a unicellular suspension.

After being washed twice with sterilized saline, the spleen cells are suspended in RPMI1460 containing 10% FCS and containing 1 µg/ml of concanavalin A (abbreviated as ConA: Sigma, U.S.) as well as an antibiotic (Sigma: Antibiotic Antimycotic), and dispensed into a 96-well microplate, in which 100 µl each of diluted test samples are previously dispensed, in amounts of 3 × 10⁵ cells/100 µl/well.

Thereafter, culturing is carried out under 5% carbon dioxide gas at 37°C for 3 days, and then the blastogenesis of lymphocytes is measured by an MTT reduction method [Mosmann T, Journal of Immunological Method, vol. 65, p. 55, 1983]. The blastogenesis rate can be quantitated as a rate against the blastogenesis of those not having test compounds added thereto, which is defined as one. The presence of an immunoactivating action can be determined if the blastogenesis rate exceeds one.

As for the cellular metabolism activity, test compounds are added at a concentration of, e.g., 0.39 to 12.5 µg/ml to an RPMI1640 medium containing 2 × 10⁶ cells/ml of BALB/c mouse bone marrow cells, antibiotics, 10% bovine fetal serum, and cultured under 5% carbon dioxide gas at 37°C for 5 days, and the mitochondrial metabolism activity of the bone marrow cells is measured by an MTT reduction method. The presence of an immunoactivating action can be determined if the magnitude of metabolism exceeds one, where the cellular metabolism of cells not having test compounds added thereto is defined as one.

A "bone marrow cellular metabolism promoting action" refers to an action of promoting the growth of bone marrow cells via various action mechanisms, broadly encompassing a leukocyte growing action and a lymphocyte function regulating action as described below.

Lymphocytes are cells which have the function of recognizing foreign antigens at their surface receptors, and convert themselves into cells which play the most important roles in immune phenomena through cell division and maturation. Antigens and lectins are known as substances which cause activation of lymphocytes. Such activation causes biochemical changes in cell membranes and cytoplasm. In the present specification, the actions of regulating immune phenomena chiefly ascribable to lymphocytes are referred to as "lymphocyte function regulating actions".

Leukocytes, which are one of hemocyte components, are composed of neutrophils, eosinophils, basophils, monocytes, and lymphocytes (T lymphocytes, B lymphocytes). Leukocytes other than T lymphocytes grow, differentiate, and mature within the bone marrow. T lymphocytes grow within the bone marrow, and thereafter grow, differentiate, and mature in the thymus. The leukocyte growing action can be utilized for therapeutic or prophylactic agents for leukopenia due to various causes, e.g., radiation therapies or chemotherapies for cancer. Furthermore, it can also be utilized for hematopoiesis promoters or drugs for achieving a prompt restoration of the number of leukocytes in a bone marrow transplant.

The compounds according to the present invention also include substances having other immunoactivating actions, e.g., increasing G-CSF and the like, so that some synergetic effects can be expected. Moreover, they can be utilized in the therapeutic fields of post-bone marrow transplant thrombocytopenia and autoimmune diseases accompanied by a decrease in platelets, e.g., aplastic anemia and spontaneous thrombocytopenic purpura.

As compositions, medical compositions (including quasi drugs), animal drugs (zootechnical drugs, aquicultural drugs, etc.) compositions, as well as food compositions, cosmetic compositions, and the like are included. Thus, the present invention can be applied to various uses, that is, as an immunofunction activator for humans or animals, or as a quasi drug, a cosmetic, a food, a specific healthcare food, a beverage, or a feed for domestic animals which is mixed for their expected immunofunction activating action.

### BEST MODES FOR CARRYING OUT THE INVENTION

The compounds which are used for the present invention, or salts thereof, or hydrates thereof can be easily produced by methods which in themselves are known. The following production methods or similar methods can be presented as specific examples of such methods.

### (Production Method 1)

The compound represented by general formula (II) can be obtained by, for example, subjecting licoricidin (Compound No. A-1) and licorisoflavan A (Compound No. A-2) described in literature (Shibata, S., et al., Ibid. 1968, 16, 1932.; Kinoshita, T., et al., Chem. Pharm. Bull., 1978, 26, 141.), which are naturally occurring and may be extracts from glycyrrhiza, as well as their similar compounds, to a chemical reaction such as alkylation or reduction, or by protecting the hydroxyl groups which are unrelated to the reaction with suitable protecting groups, e.g., acetyl groups, methoxymethyl groups, or silyl groups, before carrying out the aforementioned reaction and then canceling the protection by the protecting groups by a usual method. The compounds which are obtained through such chemical modification are novel compounds.

### (Production Method 2)

The compound represented by general formula (II) can be generally synthesized also from known compounds, as described in known literature (Shih, Thomas L., Wyvratt, Matthew J., et al., J. Org. Chem. (1987), 52(10), 2029-33).

In Steps 1 to 10 below, in conjunction with certain kinds of substituents having reactivity, protecting groups may be introduced at the substituents as appropriate. The general types of such protecting groups and their removal, etc., will all be described later. The introduction/removal of protecting groups is partially included with the steps.

### (Step 1)

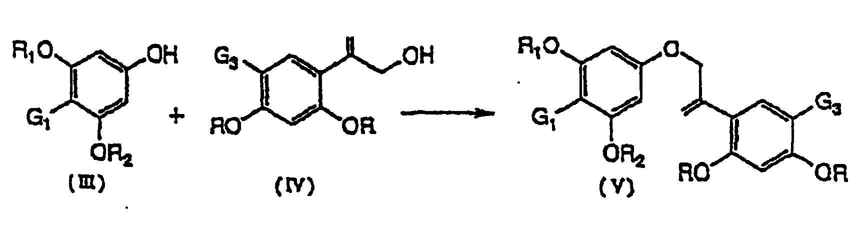

[where G₁, G₃, R₁ and R₂ have the same meanings as in the aforementioned general formula (II); R represents a hydroxyl group protecting group (e.g., 2-(trimethylsilyl)ethoxymethyl (SEM)).]

Compound (V) can be produced as above by utilizing a Mitsunobu reaction in which Compound (III) is reacted with Compound (IV), as described in known literature (Mitsunobu, O. Synthesis 1981, 1.), for example.

Preferable examples of reagents used for the Mitsunobu reaction include dialkylazodicarboxylates (e.g., diisopropylazodicarboxylate or diethylazodicarboxamide) or azodicarbonyls (e.g., 1,1-(azodicarbonyl)dipiperidine or tetraalkylazodicarboxamide (such as tetramethylazodicarboxamide)) combined with phosphines (e.g., triarylphosphine (such as triphenylphosphine, tri-o-tolylphosphine, tri-m-tolylphosphine, or tri-p-tolylphosphine) and trialkylphosphines (such as trimethylphosphine, triethylphosphine, tri-n-propylphosphine, triisopropylphosphine, tri-n-butylphosphine, or tri-t-butylphosphine)), although not particularly limited so long as they are reagents used for Mitsunobu reactions. Diethylazocarboxylate and triphenylphosphine are a more preferable combination.

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), and sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 70°C, and preferably 0°C to 30°C.

The reaction time differs depending on the solvents that are chiefly used, reaction temperature, and types of the raw compounds or reaction reagents, but is preferably 0.03 to 48 hours, and more preferably 1 to 6 hours.

Compound (IV) is used in an amount of 1.0 to 2.0 equivalents, and preferably 1.1 to 1.2 equivalents, per equivalent of Compound (III).

Dialkylazodicarboxylate or tetraalkylazodicarboxamide is used in an amount of 1.0 to 2.0 equivalents, and preferably 1.5 to 1.7 equivalents, per equivalent of Compound (III). Phosphine is used in an amount of 1.0 to 2.0 equivalents, and preferably 1.5 to 1.7 equivalents, per equivalent of Compound (III).

It is also possible to effect the reaction in the presence of argon or nitrogen.

In a subsequent step, the resultant Compound (V) may be used in the form of the reaction solution or a crude product, or after being purified by usual methods (e.g., column chromatography or recrystallization).

### (Step 2)

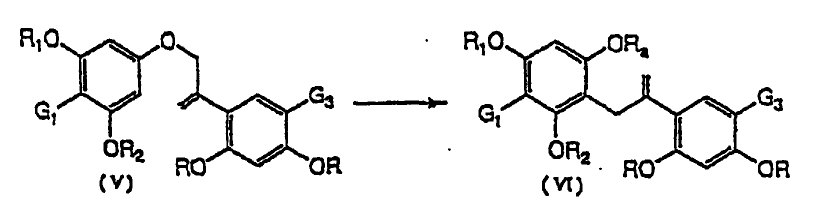

[where G₁, G₃, R, R₁ and R₂ have the same meanings as above; Rₐ represents hydrogen or an acetyl group.]

Compound (VI) can be produced as above by subjecting Compound (V) to a rearrangement reaction, for example.

The rearrangement reaction can be effected by heating, addition of reagents, etc. In the case of using reagents, such reagents are appropriately selected depending on the kinds of raw compounds and are not particularly limited so long as they are used as reagents for usual rearrangement reactions. For example, acids or bases can be used. Examples of acids include, for example, Brönsted acids such as inorganic acids (e.g., hydrogen chloride, hydrobromic acid, sulfuric acid, perchloric acid, or phosphoric acid) or organic acids (e.g., acetic acid, formic acid, oxalic acid, methanesulfonic acid, p-toluenesulfonic acid, trifluoroacetic acid, or trifluoromethanesulfonic acid) or Lewis acids such as boron trichloride, boron trifluoride, or boron tribromide. Examples of bases include, for example, alkaline metal carbonates (e.g., sodium carbonate or potassium carbonate), alkaline metal hydrogen carbonates (e.g., sodium hydrogen carbonate, potassium hydrogen carbonate, or lithium hydrogen carbonate), alkaline metal hydrides (e.g., sodium hydride, lithium hydride, or potassium hydride), alkaline metal hydroxides (e.g., sodium hydroxide, potassium hydroxide, barium hydroxide, or lithium hydroxide), alkaline metal fluorides (e.g., sodium fluoride or potassium fluoride), alkaline metal alkoxides (e.g., sodium methoxide, sodium ethoxide, potassium methoxide, potassium ethoxide, potassium t-butoxide, or lithium metboxide), lithium diisopylamide, tetra-n-butylammonium, anhydrous sodium acetate, and mercury acetate.

The solvents to be used are not particularly limited so long as they do not have unfavorable effects on the reaction and so long as they are capable of dissolving the starting materials to an extent that is not problematic with respect to the reaction, but preferable examples include alcohol solvents (e.g., methanol, ethanol, n-propanol, or isopropanol), ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), and sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 350°C, and preferably -20°C to 300°C.

The reaction time differs depending on the solvents that are chiefly used, reaction temperature, and types of the raw compounds or reaction reagents, but is preferably 0.03 to 48 hours, and more preferably 0.5 to 10 hours.

It is also possible to effect the reaction in the presence of argon or nitrogen.

After being purified by usual methods (e.g., column chromatography or recrystallization), the resultant Compound (VI) may be used in a subsequent step.

### (Step 3)

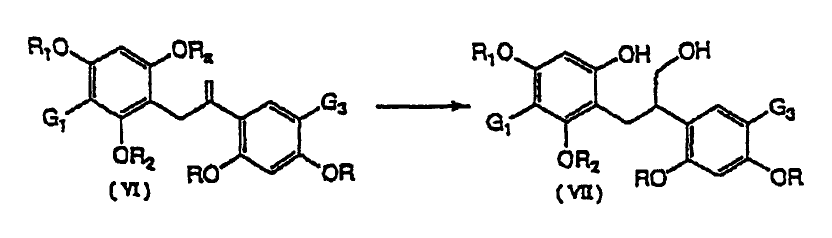

[where the symbols have the same meanings as above.]

Compound (VII) can be produced, for example, by oxidizing Compound (VI) with an oxidizing agent in an appropriate solvent, with or without the use of a hydroborating agent.

In the case of using a hydroborating agent, examples of the hydroborating agent include, for example, borane, diborane, 9-borabicyclo[3.3.1]nonane (9-BBN), borohydride salts (e.g., sodium borohydride), and Lewis acids, as appropriately selected depending on the kind of Compound (VI). Examples of the oxidizing agent to be used include, for example, hydrogen peroxide, or peroxides (e.g., t-butylperoxide) although not particularly limited so long as they are usual oxidizing agents.

The solvents to be used in the case where a hydroborating agent is used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), alcohol solvents (e.g., methanol, ethanol, n-propanol, or isopropanol), water, and the like, or mixed solvents thereof. They are appropriately selected in view of the solubility of the compounds, types of reaction reagents, and the like.

The reaction temperature is -78°C to 70°C, and preferably -10°C to 35°C.

The reaction time differs depending on the solvents that are chiefly used, reaction temperature, and types of the raw compounds or reaction reagents, but is preferably 0.03 to 48 hours, and more preferably 1 to 6 hours.

Examples of the oxidizing agent to be used include, in addition to the aforementioned peroxides, osmium compounds (e.g., potassium osmate dihydrate or osmium tetraoxide), ruthenium oxides (e.g., ruthenium oxide(IV)), selenium compounds (e.g., selenium dioxide), manganese oxide (e.g., manganese peroxide or manganese dioxide), metal ferricyanides (e.g., potassium ferricyanide), nitrite esters (e.g., ethyl nitrite), hypochlorite compounds (e.g., ethyl hypochlorite), and persulfate compounds (e.g., potassium persulfate), for example. These oxidizing agents are preferably used in the presence of an acid or a base; examples of acids include Lewis acids (e.g., aluminum chloride) and the like; examples of bases include alkaline metal hydroxides (e.g., sodium hydroxide) and organic bases (e.g., tetramethylethylenediamine).

The solvents to be used in the case where an oxidizing agent is used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), alcohol solvents (e.g., methanol, ethanol, n-propanol, isopropanol, n-butanol, t-butanol), esters (e.g., ethyl acetate, butyl acetate, or diethyl carbonate), and water, as well as pyridine, acetone, etc., or mixed solvents thereof. They are appropriately selected in view of the solubility of the compounds, reaction types, and the like.

The reaction temperature is -78°C to 70°C, and preferably -10°C to 35°C.

The reaction time differs depending on the solvents that are chiefly used, reaction temperature, and types of the raw compounds or reaction reagents, but is preferably 0.03 to 48 hours, and more preferably 1 to 6 hours.

In order to produce Compound (VII) from Compound (VI), the step which uses a hydroborating agent and the step which uses an oxidizing agent may be performed in a stepwise manner. The step which uses an oxidizing agent alone may allow Compound (VI) to be obtained under certain reaction conditions.

In a subsequent step, the resultant Compound (VII) may be used in the form of the reaction solution or a crude product, or after being purified by usual methods (e.g., column chromatography or recrystallization).

### (Step 4)

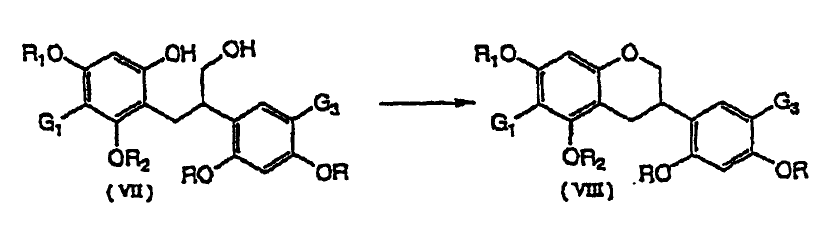

[where the symbols have the same meanings as above.]

Compound (VIII) can be produced by, for example, subjecting Compound (VII) to a dehydration reaction in the presence or absence of a solvent, and in the presence or absence of an acid or base, thereby closing the ring.

The acids to be used are not particularly limited so long as they are used as acids for usual reactions, but preferable examples include Brönsted acids such as inorganic acids (e.g., hydrogen chloride, hydrobromic acid, sulfuric acid, perchloric acid, or phosphoric acid) or organic acids (e.g., acetic acid, formic acid, oxalic acid, methanesulfonic acid, p-toluenesulfonic acid, trifluoroacetic acid, or trifluoromethanesulfonic acid), Lewis acids such as boron trichloride, boron trifluoride, or boron tribromide, and phosphine combined with dialkylazodicarboxylate or tetraalkylazodicarboxamide (e.g., 1:1 triphenylphosphinediethylazodicarboxylate (TPP-DEAD)). The bases to be used are not particularly limited so long as they are used as bases for usual reactions, but preferable examples include alkaline metal carbonates (e.g., sodium carbonate) or organic bases (e.g., pyridine).

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), as well as pyridine, acetone, etc., or mixed solvents thereof. They are appropriately selected in view of the solubility of the compounds, reaction types, and the like. In some cases, acids or bases may themselves be used as solvents.

The reaction temperature is -78°C to 250°C, and preferably 0°C to 100°C.

The reaction time differs depending on the solvents that are chiefly used, reaction temperature, and types of the raw compounds or reaction reagents, but is preferably 0.03 to 48 hours, and more preferably 1 to 25 hours.

The acids are to be used in an amount of 0.8 to 2.0 equivalents, per equivalent of Compound (VII).

It is also possible to effect the reaction in the presence of argon or nitrogen.

In a subsequent step, the resultant Compound (VIII) may be used in the form of the reaction solution or a crude product, or after being purified by usual methods (e.g., column chromatography or recrystallization).

### (Step 5)

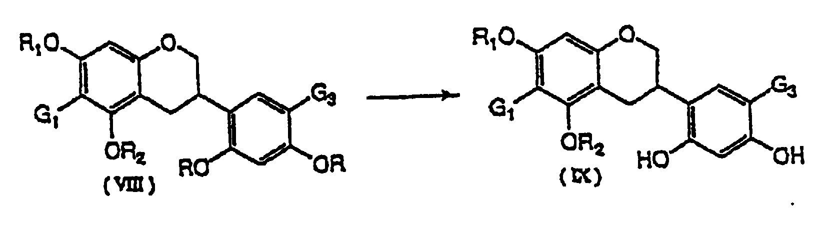

[where the symbols have the same meanings as above.]

Compound (IX) can be produced by, for example, subjecting Compound (VIII) to a deprotection reaction.

A deprotection reaction can be carried out, for example, under hydrolytic conditions (concentrated sulfuric acid, disodium carbonate). In general, it can be effected by a method for removing the protecting groups for hydroxyl groups using acids or bases (ester hydrolysis), as described later.

In a subsequent step, the resultant Compound (IX) may be used in the form of the reaction solution or a crude product, or after being purified by usual methods (e.g., column chromatography or recrystallization).

### (Step 6)

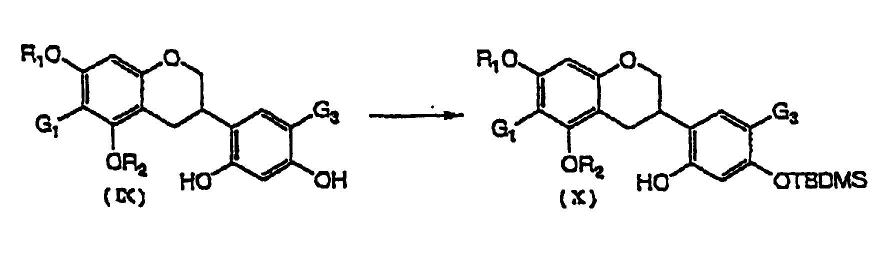

[where TBDMS represents a tert-butyldimethylsilyl group, and the other symbols have the same meanings as above.]

Compound (X) can be produced by, for example, allowing Compound (IX) to react with tert-butyldimethylsilylchloride. Alternatively, a common protecting group for hydroxyl groups (described later) other than tert-butyldimethylsilyl groups may be used in the protection of the hydroxyl groups, for use in subsequent steps.

The introduction method can be carried out in the presence or absence of a base; for example, it can be carried out by adding an organic base (e.g., trimethylamine, triethylamine, diisopropylethylamine, pyridine, picoline, N-methylpyrrolidine, piperidine, N-methylpiperidine, N-methylmorpholine, 1,5-diazabicyclo[4.3.0]non-5-ene, 1,4-diazabicyclo[2.2.2]octane, or 1.8-diazabicyclo[5.4.0]-7-undecene).

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 200°C, and preferably 0°C to 35°C.

The reaction time differs depending on the solvents that are chiefly used, reaction temperature, and types of the raw compounds or reaction reagents, but is preferably 0.03 to 48 hours, and more preferably 2 to 25 hours.

In a subsequent step, the resultant Compound (X) may be used in the form of the reaction solution or a crude product, or after being purified by usual methods (e.g., column chromatography or recrystallization).

### (Step 7)

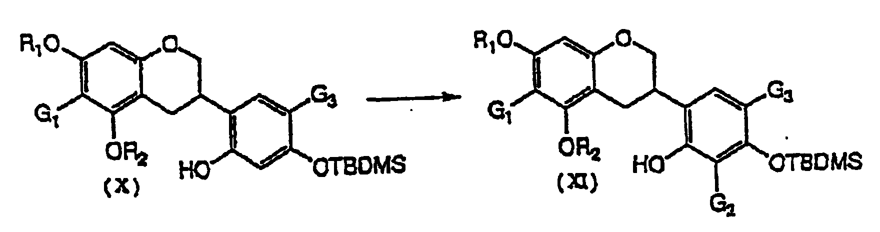

[where the symbols have the same meanings as above.]

Compound (XI) can be produced as above by allowing a compound: G₂-X (where X is a halogen) to react with Compound (X) in the presence of a base, as described in known literature (Jain, A. C. et al., Indian J. Chem. (1969), 7, 1072.; Bigi, F. et al., Tetrahedron (1983), 39, 169.), for example. Alternatively, Compound (XI) can be produced by a Friedel-Crafts alkylation reaction or the like.

The bases to be used in the case of allowing the compound: G₂-X (where X is a halogen) to react are not particularly limited so long as they are used as bases for usual reactions, but preferable examples include alkaline metal carbonates (e.g., sodium carbonate or potassium carbonate), alkaline metal hydrogen carbonates (e.g., sodium hydrogen carbonate, potassium hydrogen carbonate, or lithium hydrogen carbonate), alkaline metal hydrides (e.g., sodium hydride, lithium hydride, or potassium hydride), alkaline metal hydroxides (e.g., sodium hydroxide, potassium hydroxide, barium hydroxide, or lithium hydroxide), alkaline metal fluorides (e.g., sodium fluoride or potassium fluoride), alkaline metal alkoxides (e.g., sodium methoxide, sodium ethoxide, potassium methoxide, potassium ethoxide, potassium t-butoxide, or lithium methoxide), n-butyl lithium, and the like.

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 70°C, and preferably 0°C to 25°C.

The reaction time differs depending on the solvents that are chiefly used, reaction temperature, and types of the raw compounds or reaction reagents, but is preferably 0.03 to 48 hours, and more preferably 2 to 6 hours.

The Lewis acids to be used in the case of performing a Friedel-Crafts alkylation reaction are not particularly limited so long as they are usually used. For example, trilower-alkylsilyltrifluoromethanesulfonate, aluminum chloride, aluminum bromide, tin tetrachloride, zinc bromide, titanium tetrachloride, perchloric acid trimethylsilyl ester, perchloric acid triphenylmethyl ester, boron trifluorides (BF₃), hydrogen fluoride, and phosphoric acid may be used.

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), saturated hydrocarbons (e.g., heptane or hexane), or mixed solvents thereof. They are appropriately selected in view of the solubility of the compounds, reaction types, and the like.

The reaction temperature is -78°C to 70°C, and preferably 0°C to 25°C.

The reaction time differs depending on the solvents that are chiefly used, reaction temperature, and types of the raw compounds or reaction reagents, but is preferably 0.03 to 48 hours, and more preferably 2 to 6 hours.

In a subsequent step, the resultant Compound (XI) may be used in the form of the reaction solution or a crude product, or after being purified by usual methods (e.g., column chromatography or recrystallization).

### (Step 8)

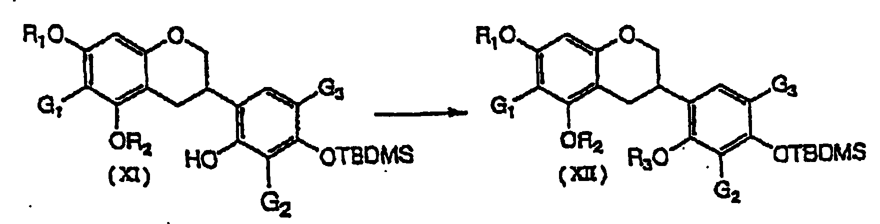

[where the symbols have the same meanings as above.]

Compound (XII) can be produced by, for example, subjecting Compound (XI) to an alkylation reaction in the presence of a base.

The bases to be used are not particularly limited so long as they are used as bases for usual reactions, but preferable examples include organic bases (e.g., trimethylamine, triethylamine, diisopropylethylamine, pyridine, picoline, N-methylpyrrolidine, piperidine, N-methylpiperidine, N-methylmorpholine, 1,5-diazabicyclo[4.3.0]non-5-ene, 1,4-diazabicyclo[2.2.2]octane, or 1.8-diazabicyclo[5.4.0]-7-undecene), alkaline metal carbonates (e.g., sodium carbonate or potassium carbonate), alkaline metal hydrogen carbonates (e.g., sodium hydrogen carbonate, potassium hydrogen carbonate, or lithium hydrogen carbonate), alkaline metal hydrides (e.g., sodium hydride, lithium hydride, or potassium hydride), alkaline metal hydroxides (e.g., sodium hydroxide, potassium hydroxide, barium hydroxide, or lithium hydroxide), alkaline metal fluorides (e.g., sodium fluoride or potassium fluoride), alkaline metal alkoxides (e.g., sodium methoxide, sodium ethoxide, potassium methoxide, potassium ethoxide, potassium t-butoxide, or lithium methoxide), and the like.

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 70°C, and preferably 0°C to 25°C.

The reaction time differs depending on the solvents that are chiefly used, reaction temperature, and types of the raw compounds or reaction reagents, but is preferably 0.03 to 48 hours, and more preferably 2 to 6 hours.

In a subsequent step, the resultant Compound (XII) may be used in the form of the reaction solution or a crude product, or after being purified by usual methods (e.g., column chromatography or recrystallization).

### (Step 9)

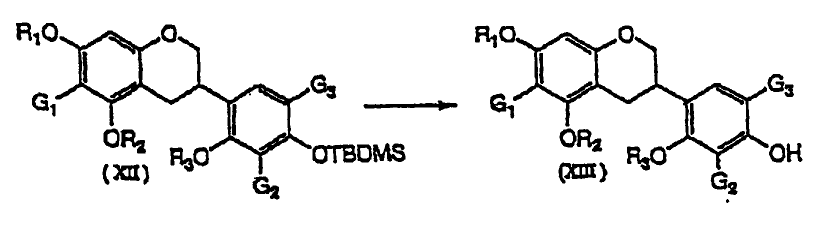

[where the symbols have the same meanings as above.]

Compound (XIII) can be produced as above by, for example, subjecting Compound (XII) to a deprotection reaction.

As the method of deprotection, general methods used in conjunction with protecting groups for hydroxyl groups (hydrolysis of a silyl ether by an acid), as described later, are included.

In a subsequent production step, the resultant Compound (XIII) may be used in the form of the reaction solution or a crude product, or after being purified by usual methods (e.g., column chromatography or recrystallization).

### (Step 10)

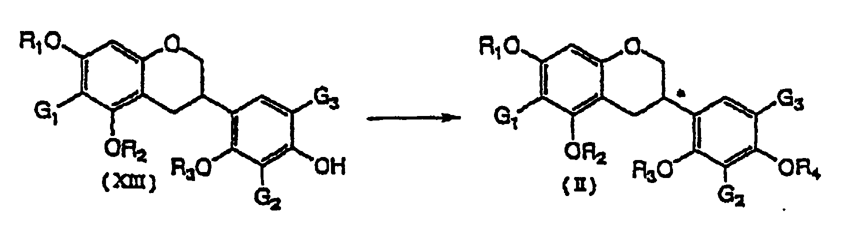

[where the symbols have the same meanings as above.]

Compound (II) can be produced as above by, for example, subjecting Compound (XIII) to an alkylation reaction.

The aforementioned alkylation reaction can be performed substantially in the same manner as in Step 8.

The bases to be used are not particularly limited so long as they are used as bases for usual reactions, but preferable examples include organic bases (e.g., trimethylamine, triethylamine, diisopropylethylamine, pyridine, picoline, N-methylpyrrolidine, piperidine, N-methylpiperidine, N-methylmorpholine, 1,5-diazabicyclo[4.3.0]non-5-ene, 1,4-diazabicyclo[2.2.2]octane, or 1.8-diazabicyclo[5.4.0]-7-undecene), alkaline metal carbonates (e.g., sodium carbonate or potassium carbonate), alkaline metal hydrogen carbonates (e.g., sodium hydrogen carbonate, potassium hydrogen carbonate, or lithium hydrogen carbonate), alkaline metal hydrides (e.g., sodium hydride, lithium hydride, or potassium hydride), alkaline metal hydroxides (e.g., sodium hydroxide, potassium hydroxide, barium hydroxide, or lithium hydroxide), alkaline metal fluorides (e.g., sodium fluoride or potassium fluoride), alkaline metal alkoxides (e.g., sodium methoxide, sodium ethoxide, potassium methoxide, potassium ethoxide, potassium t-butoxide, or lithium methoxide), and the like.

The solvents to be used are not particularly limited so long as they do not have unfavorable effects on the reaction and so long as they are capable of dissolving the starting materials to an extent that is not problematic with respect to the reaction, but preferable examples include ether solvents (e.g., tetrahydrofuran, diethyl ether, dioxane, diisopropyl ether, dimethoxyethane, or diethyleneglycol dimethyl ether), halogenated hydrocarbons (e.g., carbon tetrachloride, dichloromethane, dichlorobenzene, chlorobenzene, dichloroethane, or methylene chloride), aromatic hydrocarbons (e.g., benzene, toluene, or xylene), saturated hydrocarbons (e.g., heptane or hexane), nitriles (e.g., acetonitrile or isobutyrylnitrile), amides (e.g., formamide, N,N-dimethylformamide, N,N-dimethylacetamide, N-methyl-2-pyrrolidone, N-methylpyrrolidinone or hexamethylenephosphorotriamide), sulfoxides (e.g., dimethylsulfoxide or sulfolane), as well as pyridine, acetone, etc., or mixed solvents thereof. They are appropriately selected in view of the solubility of the compounds, reaction types, and the like.

The reaction temperature is -78°C to 70°C, and preferably 0°C to 25°C.

The reaction time differs depending on the solvents that are chiefly used, reaction temperature, and types of the raw compounds or reaction reagents, but is preferably 0.03 to 48 hours, and more preferably 2 to 6 hours.

The final resultant Compound (II) of interest may be obtained in the form of the reaction solution or a crude product, or purified by usual methods (e.g., column chromatography or recrystallization).

As various protecting groups for amino groups, carboxyl groups, hydroxyl groups, carbonyl groups, etc., used in the aforementioned series of synthesis reactions (including cases where they exist as substituents), the following may be used.

Examples of protecting groups for amino groups include, for example, protecting groups of the type which form amides (e.g., formyl, acetyl, chloroacetyl, dichloroacetyl, trichloraacetyl, trifuluoroacetyl, acetoacetyl, or o-nitrophenylacetyl), protecting groups of the type which form carbamates (e.g., t-butoxycarbonyl, 2,2,2-trichloroethoxycarbonyl, benzyloxycarbonyl, p-methoxybenzyloxycarbonyl, 2-chlorobenzyloxycarbonyl, 2,4-dichlorobenzyloxycarbonyl, p-nitrobenzyloxycarbonyl, benzhydryloxycarbonyl, 2-trimethylsilylethoxycarbonyl, 1-methyl-1-(4-biphenylyl)ethoxycarbonyl, 9-anthrylmethoxycarbonyl, 9-fluorenylmethoxycarbonyl, isonicotinyloxycarbonyl, or 1-adamantyloxycarbonyl), as well as trityl, phthaloyl, and the like.

Examples of protecting groups for hydroxyl groups include, for example, protecting groups of the type which form ethers (e.g., methoxymethyl, t-butoxymethyl, 2-methoxyethoxymethyl, 2-(trimethylsilyl)ethoxymethyl, benzyloxymethyl, methylthiomethyl, 2-tetrahydropyranyl, 4-methoxy-4-tetrahydropyranyl, 2-tetrahydropyranyl, benzyl, p-methoxybenzyl, p-nitrobenzyl, o-nitrobenzyl, 2,6-dichlorobenzyl, or trityl), protecting groups of the type which form silyl ethers (e.g., trimethylsilyl, triethylsilyl, triisopropylsilyl, isopropyldimethylsilyl, diethylisopropylsilyl, t-butyldimethylsilyl, t-butyldiphenylsilyl, triphenylsilyl, methyldiphenylsilyl, or tribentylsilyl), and protecting groups of the type which form esters (e.g., formyl, acetyl, chloroacetyl, dichloroacetyl, trichloroacetyl, pivaloyl, benzoyl, or benzyloxycarbonyl, 2-bromobenzyloxycarbonyl).

Preferable examples of protecting groups for carboxy groups include, for example, protecting groups of the type which form esters (e.g., methyl, ethyl, t-butyl, methoxymethyl, methoxyethoxymethyl, 2,2,2-trichloroethyl, benzyloxymethyl, 2-trimethylsilylethyl, allyl, benzyl, p-methoxybenzyl, o-nitrobenzyl, p-nitrobenzyl, benzhydryl, trityl, cyclohexyl, cyclopentyl, or phenacyl), and protecting groups of the type which form silyl esters (e.g., trimethylsilyl, triethylsilyl, t-butyldimethylsilyl, dimethylphenylsilyl, or isopropyldimethylsilyl).

Examples of protecting groups for carbonyl groups include, for example, protecting groups of the type which form acetals or ketals, or dithioacetals or dithioketals (e.g., dimethyl, diethyl, diacetyl, or dibenzyl), protecting groups of the type which form 1,3-dioxanes or 1,3-dioxolanes which may be substituted, protecting groups of the type which form 1,3-dithianes or 1,3-dithiolanes, and protecting groups of the type which form substituted hydrazones (e.g., N,N-dimethyl or 2,4-dinitrophenyl).

Examples of methods for removing the aforementioned protecting groups for amino groups, protecting groups for hydroxyl groups, protecting groups for carbonyl groups, and protecting groups for carboxyl groups include, for example, methods utilizing bases, methods utilizing acids, methods utilizing reduction, methods utilizing ultraviolet light, methods utilizing hydrazines, methods utilizing phenylbydrazines, methods utilizing sodium N-methyldithiocarbamate, methods utilizing tetrabutylammonium fluoride, methods utilizing palladium acetate, methods utilizing mercury chloride, and methods utilizing Lewis acids. These common methods or other known means may be appropriately selected and used.

Methods utilizing bases, as well as methods utilizing acids, constitute a common type of methods for hydrolyzing amides, esters, etc., and are applied to the removal of corresponding protecting groups. For example, organic bases may be effectively used for the deprotection of amino groups which are protected by 9-fluorenylmethoxycarbonyl. Preferable examples of the bases to be used include, for example, inorganic bases such as alkaline metal hydroxides (e.g., lithium hydroxide, sodium hydroxide, or potassium hydroxide), alkaline-earth metal hydroxides (e.g., magnesium hydroxide or calcium hydroxide), alkaline metal carbonates (e.g., sodium carbonate or potassium carbonate), alkaline-earth metal carbonates (e.g., magnesium carbonate or calcium carbonate), alkaline metal hydrogen carbonates (e.g., sodium hydrogen carbonate or potassium hydrogen carbonate), alkaline metal acetates (e.g., sodium acetate or potassium acetate), alkaline-earth metal phosphates (e.g., calcium phosphate or magnesium phosphate), alkaline metal hydrogen phosphates (e.g., disodium hydrogen-phosphate or dipotassium hydrogen-phosphate), and aqueous ammonia, as well as organic bases such as trimethylamine, triethylamine, diisopropylethylamine, pyridine, picoline, N-methylpyrrolidine, piperidine, N-methylpiperidine, N-methylmorpholine, 1,5-diazabicyclo[4.3.0]non-5-ene, 1,4-diazabicyclo[2.2.2]octane, and 1.8-diazabicyclo[5.4.0]-7-undecene.

Methods utilizing acids constitute a common type of methods for hydrolyzing amides, esters, silyl esters, silyl ethers, etc., and are applied to the removal of corresponding protecting groups. They may also be applied for the deprotection of protected amino groups (e.g., amino groups protected by t-butoxycarbonyl, p-methoxybenzyloxycarbonyl, benzhydryloxycarbonyl, benzhydryloxycarbonyl, 9-anthrylmethoxycarbonyl, 1-methyl-1-(4-biphenylyl)ethoxycarbonyl, 1-adamantyloxycarbonyl, trityl, or the like), protected hydroxyl groups (e.g., hydroxyl groups protected by methoxymethyl, t-butoxymethyl, 2-tetrahydropyranyl, 4-methoxy-4-tetrahydropyranyl, 2-tetrahydropyranyl, 2-tetrahydrofuranyl, trityl, or the like), and the like. Preferable examples of the acids to be used include, for example, organic acids (e.g., formic acid, trifluoroacetic acid, benzenesulfonic acid, or p-toluenesulfonic acid), inorganic acids (e.g., hydrochloric acid, hydrobromic acid, or sulfuric acid).

Methods utilizing reduction are applied to the deprotection of protected amino groups (e.g., amino groups protected by trichloroacetyl, trifluoroacetyl, o-nitrophenylacetyl, 2,2,2-trichloroethoxycarbonyl, benzyloxycarbonyl, p-nitrobenzyloxycarbonyl, 2,4-dichlorobenzyloxycarbonyl, isonicotinyloxycarbonyl, or trityl), protected hydroxyl groups (e.g., hydroxyl groups protected by benzyl or p-nitrobenzyl), protected carboxyl groups (e.g., carboxyl groups protected by benzyloxymethyl, benzyl, p-nitrobenzyl, phenacyl, 2,2,2-trichloroethyl, or benzhydryl). Preferable examples of reduction methods to be used include, for example, reduction utilizing sodium borohydride, reduction utilizing zinc/acetic acid, or catalytic reduction.

Methods utilizing ultraviolet light are applied to the deprotection of hydroxyl groups and carboxyl groups protected by o-nitrobenzyl, for example.

Methods utilizing hydrazines are applied to the deprotection of amino groups which are protected by phthaloyl (e.g., phthalimide), for example.

Methods utilizing phenylbydrazines are applied to the deprotection of amino groups which are protected by acetoacetyl groups, for example.

Methods utilizing sodium N-methyldithiocarbamate are applied to the deprotection of amino groups and hydroxyl groups which are protected by chloroacetyl, for example.

Methods utilizing tetrabutylammonium fluorides are used as a method for, for example, removing the protecting groups from 2-trimethylsilylethylcarbamate, silyl ethers, or silyl esters, so as to obtain amino groups, hydroxyl groups, or carboxyl groups therefrom, respectively.

Methods utilizing mercury chloride are applied to the deprotection of hydroxyl groups which are protected by methylthiomethyl, for example.

Methods utilizing Lewis acids are applied to the deprotection of hydroxyl groups which are protected by 2-methoxyethoxymethyl. Preferable examples of Lewis acids to be used include, for example, mercury bromide and titanium tetrachloride.

Hereinafter, a general method for preparing the medical composition according to the present invention will be described. Animal drug compositions, quasi drugs, aquicultural drug compositions, food compositions, cosmetic compositions, and the like can also be produced by known preparation methods.

The compounds according to the present invention can be blended with a pharmacologically acceptable carrier, and administered either orally or parenterally in the form of solid formulations such as tablets, capsules, granules, powder, dust, suppositories, or liquid formulations such as syrup, injections, suspensions, solutions, or spray. Examples of pharmacologically acceptable carriers include excipients, lubricants, binders, disintegrators, disintegration inhibitors, absorption promoters, absorbents, moisturizers, solubilizing adjuvants, or stabilizers for solid formulations; and solvents, solubilizing adjuvants, suspending agents, isotonizing agents, buffer agents, soothing agents, and the like. Formulation additives such as antiseptics, antioxidants, colorants, or sweeteners may be used as necessary. It is also possible to blend substances that have immunoactivating actions into the composition according to the present invention in addition to the compounds represented by the aforementioned general formula (II), salts thereof, or hydrates thereof. Examples of parenteral administration routes include intravenous injection, intramuscular injection, nasal, rectal, vaginal, and transcutaneous routes.

Examples of excipients for solid formulations include, for example, glucose, lactose, sucrose, D-mannitol, crystalline cellulose, starch, calcium carbonate, light anhydrous silicic acid, sodium chloride, kaolin, and urea.

Examples of lubricants for solid formulations include, for example, magnesium stearate, calcium stearate, boric acid powder, coloidal silicic acid, talc, and polyethylene glycol.

Examples of binders for solid formulations include, for example, water, ethanol, propanol, sucrose, D-mannitol, crystalline cellulose, dextrin, methyl cellulose, hydroxypropyl cellulose, hydroxypropylmethyl cellulose, carboxymethyl cellulose, starch solutions, gelatin solutions, polyvinylpyrrolidone, calcium phosphate, potassium phosphate, and shellac.

Examples of disintegrators for solid formulations include, for example, starch, carboxymetbyl cellulose, carboxymethyl cellulose calcium, agar powder, laminaran powder, closcarmellose sodium, carboxymethyl starch sodium, sodium alginate, sodium hydrogen carbonate, calcium carbonate, polyoxyethylene sorbitan fatty acid esters, sodium lauryl sulfate, starch, stearic acid monoglyceride, lactose, and calcium carboxymethyl cellulose.

Preferable examples of disintegration inhibitors for solid formulations include hydrogenated oil, sucrose, stearin, cocoa butter, hardened oil, and the like.

Examples of absorption promoters for solid formulations include, for example, quaternary ammonium bases, and sodium lauryl sulfate.

Examples of absorbents for solid formulations include, for example, starch, lactose, kaolin, bentonite, and coloidal silicic acid.

Examples of moisturizers include for solid formulations include, for example, glycerine, and starch.

Examples of solubilizing adjuvants for solid formulations include, for example, arginine, gultamic acid, and aspartic acid.

Examples of stabilizers for solid formulations include, for example, human serum albumin and lactose.

When solid formulations are prepared in the form of tablets, pills, etc., they may be coated with a film of a gastrosoluble or enterosoluble substance (e.g., sucrose, gelatin, hydroxypropyl cellulose, or hydroxypropyl methylcellulose phthalate) as necessary. Tablets include tablets having usual coatings as necessary, e.g., sugar-coated tablets, gelatin-coated tablets, enteric coated tablets, film coated tablets, double tablets, and multilayer tablets. Capsules include hard capsules and soft capsules. When formed into suppositories, higher alcohols, higher alcohol esters, semisynthetic glycerides, or the like may be added in addition to the aforementioned additives.

Preferable examples of solvents for liquid formulations include water for injection, alcohols, propyleneglycol, macrogol, sesame oil, corn oil, and the like.

Preferable examples of solubilizing adjuvants for liquid formulations include, for example, polyethyleneglycol, propyleneglycol, D-mannitol, benzyl benzoate, ethanol, trisaminomethane, cholesterol, triethanolamine, sodium carbonate, sodium citrate, and the like.

Preferable examples of suspending agents for liquid formulations include surfactants such as stearyltriethanolamine, sodium lauryl sulfate, lauryl aminopropionate, lecithin, benzalkonium chloride, benzethonium chloride, or glycerine monostearate, and hydrophilic polymers such as polyvinyl alcohol, polyvinylpyrrolidone, carboxymethyl cellulose sodium, methyl cellulose, hydroxymethyl cellulose, hydroxyethyl cellulose, hydroxyethyl cellulose, or hydroxypropyl cellulose.

Preferable examples of isotonizing agents for liquid formulations include sodium chloride, glycerine, D-mannitol, and the like.

Preferable examples of buffer agents for liquid formulations include buffer solutions such as phosphates, acetates, carbonates, and citrates.

Preferable examples of soothing agents for liquid formulations include benzyl alcohol, benzalkonium chloride, procaine hydrochloride, and the like.

Preferable examples of antiseptics for liquid formulations include paraoxybenzoic acid esters, chlorobutanol, benzyl alcohol, 2-phenylethyl alcohol, dehydroacetic acid, sorbic acid, and the like.

Preferable examples of antioxidants for liquid formulations include sulfites, ascorbic acid, α-tocopherol, cysteine, and the like.

When prepared as an injection, the solution and the suspension are preferably sterilized and isotonic with respect to blood. Usually, they are sterilized through filtration with a bacteria catching filter, blending of an antimicrobial agent, or irradiation. After these treatments, they may be further formed into solids by techniques such as lyophilization, and sterilized water or a sterile injection diluent (e.g., lidocaine hydrochloride aqueous solution, saline, an aqueous solution of glucose, ethanol, or a mixed solution thereof) may be added immediately before use.

If necessary, the medical composition may further contain colorants, preservatives, aromatics, correctives, sweeteners, and other chemicals.

The dose of the effective components may differ depending on the disease state, administration route, and age or weight of the patient. However, in the case of oral administration to an adult, the dose is usually 0.1 to 100 mg/kg/day, and preferably within the range of 1 to 50 mg/kg/day.

### (Embodiments of the Invention)

Hereinafter, the present invention and its usefulness will be described in detail with reference to examples (including formulation examples) and test examples, which are not in any way limitative of the present invention.

### (Examples)

### (Example 1)

A compound described in literature, licoricidin, (Compound No. A-1) (500 mg) was dissolved in tetrahydrofuran (10 ml), to which sodium hydride (50 mg) and iodomethane (1.0 ml) were added. After being left at room temperature for 15 minutes, the reaction solution was poured into iced water, and adjusted to a pH of 4 with dilute hydrochloric acid, extracted with ethyl acetate. Following a usual method, the solvent was distilled off, thereby obtaining a residue (546 mg). The residue was eluted by silica gel column chromatography using toluene and mixed solutions which contained gradually increasing amounts of acetonitrile, so as to be isolated into fractions 1 to 5. Fraction 1 (25 mg) was further eluted by a reverse phase high performance liquid chromatography using methanol, whereby 8.6 mg of Compound No. A-3 and 3.8 mg of Compound No. A-3 were obtained.

### Compound No. A-3

¹H-NMR (δ ppm, acetone-d₆, 400 MHz) of Compound No. A-3 : 1.64, 1.66, 1.75, 1.78 (3H, s, 3-methyl-2-butenyl-CH₃ for each), 2.65 ∼ 2.95 (2H, m, 4-H₂), 3.26 (2H, br. dd, Penyl-CH2), 3.38 (1H, m, 3-H), 3.38 (2H, br-d, penyl-CH2), 3.69 (3H, s, 5-OMe), 3.75 (3H, s, 2'-OMe), 3.78 (3H, s, 7-OMe), 3.83 (3H, s, 4'-OMe), 3.96 (1H, dd, *J* = 9.6, 10.4 Hz, 2-H), 4.18 (1H, m, 2-H), 5.19 (2H, m, 3-methyl-2-butenyl-CH), 6.25 (1H, s, 8-H), 6.79 (1H, d, *J* = 8.8 Hz, 5'-H), 7.11 (1H, d, *J* = 8.8 Hz, 6'-H).
¹³C-NMR data of Compound No. A-3 ( δ ppm, acetone-d₆, 100 MHz) : 17.86, 17.95, 23.22, 23.94, 25.84, 25.84, 28.04, 31.83, 55.86, 55.97, 60.69, 62.41, 71.00, 96.39, 107.87, 109.02, 115.71, 124.11, 124.31, 125.08, 126.13, 127.78, 130.43, 131.23, 154.66, 157.91, 157.95, 158.12, 158.38.

### Compound No. A-4 powder

¹H-NMR of Compound No. A-4 (δ ppm, acetone-d₆, 400 MHz) : 1.64, 1.65 (3H, d, *J*=0.8 Hz, 3-methyl-2-butenyl-CH₃ for each), 1.74, 1.77 (3H, s, 3-methyl-2-butenyl-CH₃ for each), 2.7∼2.94 (1H, m, 4-H), 3.25 (2H, br-dd, 3-methyl-2-butenyl-CH₂), 3.3 (1H, m, 3-H), 3.43 (2H, br-d, *J*=7.2Hz, 3-methyl-2-butenyl-CH₂), 3.69 (3H, s, 5-OMe), 3.77 (3H, s, 7-OMe), 3.79 (3H, s, 4'-OMe), 4.01 (1H, t, *J*=10.0 Hz, 2-H), 4.23 (1H, m, 2-H), 5.17 (2H, m, 3-methyl-2-butenyl-CH), 6.23 (1H, s, 8-H), 6.54 (1H, d, *J*=8.8 Hz, 5'-H), 6.98 (1H, d, *J*=8.8 Hz, 6'-H).
¹³C-NMR data of Compound No. A-4 ( δ ppm, acetone-d₆, 100 MHz) : 17.84, 17.89, 23.16, 23.21, 25.84, 25.86, 27.12, 32.11, 55.86, 55.90, 60.69, 70.51, 96.35, 103.99, 109.04, 115.61, 117.71, 122.30, 123.74, 125.11, 125.33, 130.40, 131.82, 153.74, 154.75, 157.86, 157.97, 158.07.

### (Example 2)

Fraction 2 (71 mg) was further eluted by a reverse phase high performance liquid chromatography using methanol, whereby 22 mg of Compound No. A-5 and 24 mg of Compound No. A-6 were obtained.

### Compound No. A-5 powder

¹H-NMR of Compound No. A-5 (δ ppm, acetone-d₆, 400 MHz) : 1.64, 1.67 (3H, d, *J*=1.2 Hz, 3-methyl-2-butenyl-CH₃ for each), 1.75, 1.78 (3H, s, 3-methyl-2-butenyl-CH₃ for each), 2.7∼2.95 (1H, m, 4-H), 3.25 (2H, br-dd, 3-methyl-2-butenyl-CH₂), 3.3 (1H, m, 3-H), 3.39 (2H, br-d, 3-methyl-2-butenyl-CH₂), 3.69 (3H, s, 5-OMe), 3.74 (3H, s, 7-OMe), 3.78 (3H, s, 4'-OMe), 3.92 (1H, dd, *J*=10.4, 10.8 Hz, 2-H), 4.16 (1H, m, 2-H), 5.17, 5.29 (1H, m, 3-methyl-2-butenyl-CH for each), 6.24 (1H, s, 8-H), 6.68 (1H, d, *J*=8.4 Hz, 5'-H), 6.94 (1H, d, *J*=8.4 Hz, 6'-H), 827 (1H, s, OH).
¹³C-NMR data of Compound No. A-5 ( δ ppm, acetone-d₆, 100 MHz) : 17.84, 17.97, 23.21, 24.03, 25.83, 25.83, 28.08, 31.82, 55.87, 60.70, 62.33, 71.11, 96.41, 109.12, 112.28, 115.68, 122.58, 124.46, 125.10, 125.89, 126.46, 130.42, 131.10, 154.69, 156.04, 157.98, 158.12, 158.30.

### Compound No. A-6 powder

¹H-NMR of Compound No. A-6 (δ ppm, acetone-d₆, 400 MHz) : 1.65, 1.66, 1.75, 1.77 (3H, s, 3-methyl-2-butenyl-CH₃ for each), 2.7∼2.95 (1H, m, 4-H), 3.28 (2H, br-dd, 3-methyl-2-butenyl-CH₂), 3.3 (1H, m, 3-H), 3.37 (2H, br-d, 3-methyl-2-butenyl-CH₂), 3.69 (3H, s, 5-OMe), 3.74 (3H, s, 7-OMe), 3.82 (3H, s, 2'-OMe), 3.92 (1H, t, *J*=10 Hz 2-H), 4.16 (1H, m, 2-H), 5.20, 5.25 (1H, m, 3-methyl-2-butenyl-CH for each), 6.19 (1H, s, 8-H), 6.77 (1H, d, *J*=8.8 Hz, 5'-H), 6.94 (1H, d, *J*=8.4 Hz, 6'-H), 8.12 (1H, s, OH).
¹³C-NMR data of Compound No. A-6 ( δ ppm, acetone-d₆, 100 MHz) : 17.89, 17.93, 23,32, 23.94, 25.83, 25.83, 28.12, 31.92, 55.99, 60.58, 62.43, 70.91, 99.85, 107.88, 108.25, 114.55, 124.10, 124.32, 125.24, 126.15, 127.91, 130.31, 131.21, 154.32, 155.56, 157.91, 158.29, 158.37.

### (Example 3)

Fraction 3 (93 mg) was further eluted by a reverse phase high performance liquid chromatography using methanol containing 5% water, whereby 15.6 mg of licorisoflavan A (Compound No. A-2) and 11.8 mg of Compound No. A-7 were obtained.

### Licorisoflavan A (Compound No. A-2)

m.p. 98 °C (needles from Hexane-EtOAc), [α]_{D} + 19.4° (C=0.4, MeOH) (lit.²⁾ m.p. 65∼67°C, [α]_{D} +5.7° , C=0.3, MeOH),
Anal. Calc'd for C₂₇H₃₄O₅ C:73.94 H:7.81 Found C:73.73 H:7.80,
MS(EI-MS) m/z 438 [M]⁺ Calc'd for C₂₇H₃₄O₅ (438),
IR ν (KBr) cm-1 3456 (OH), 1612, 1588 (ph),
UV (λ max in MeOH, nm, ε) 282.0 (5400),
¹H-NMR of licorisoflavan A (Compound No. A-2) (δ ppm, acetone-d₆, 400 MHz) : 1.63, 1.66, 1.74, 1.78 (3H, s, 3-methyl-2-butenyl-CH₃ for each), 2.75∼2.95 (2H, m, 4-H), 3.25 (2H, br-dd, *J*=6.8, 7.6 Hz, 3-methyl-2-butenyl-CH₂), 3.42 (1H, m, 3-H), 3.45 (2H, br-d, *J*=7.2, 3-methyl-2-butenyl-CH₂), 3.69 (3H, s, 5-OMe), 3.77 (3H, s, 5-OMe), 3.97 (1H, dd, *J*=10.0, 10.4 Hz, 2-H), 4.21 (1H, m, 2-H), 5.16, 5.26 (1H, m, 3-methyl-2-butenyl-CH for each), 6.22 (1H, s, 8-H), 6.45 (1H, d, *J*=8.0 Hz, 5'-H), 6.83 (1H, d, *J*=8.4 Hz, 6'-H), 7.16, 8.09 (1H, s, OH for each).
¹³C-NMR data of licorisoflavan A (Compound No. A-2) ( δ ppm, acetone-d₆, 100 MHz):17.82, 17.92, 23.18, 23.27, 25.81, 25.84, 27.14, 32.06, 55.82, 60.65, 70.59, 96.32, 108.28, 109.11, 115.54, 116.32, 120.67, 123.89, 125.11, 125.11, 130.36, 131.78, 154.11, 154.75, 155.34, 157.96, 158.02.

### Compound No. A-7

m.p. 148∼149 °C (needles from Hexane-EtOAc), [α]_{D} +7.8° (C=0.4, MeOH),
Anal. Calc'd for C₂₇H₃₄O₅ 1/5·H₂O C:73.34 H:7.84 Found C:73.40 H:7.74,
MS(EI-MS) m/z 438 : [M]⁺ Calc'd for C₂₇H₃₄O₅ (438),
IR ν (KBr) cm-1 3389 (OH), 1614 (ph),
UV (λ max in MeOH, nm, ε) 281.8 (5400),
¹H-NMR of Compound No. A-7 (δ ppm, acetone-d₆, 400 MHz) : 1.65, 1.65, 1.75, 1.76 (3H, s, 3-methyl-2-butenyl-CH₃ for each), 2.65∼2.95 (2H, m, 4-H), 3.27 (2H, br-dd, 3-methyl-2-butenyl-CH₂), 3.4 (1H, m, 3-H), 3.41 (2H, br-d, 3-methyl-2-butenyl-CH₂), 3.69 (3H, s, 5-OMe), 3.79 (3H, s, 4'-OMe), 3.97 (1H, t, *J*=10.0 Hz, 2-H), 4.18 (1H, m, 2-H), 5.19, 5.24 (1H, m, 3-methyl-2-butenyl-CH for each), 6.17 (1H, s, 8-H), 6.53 (1H, d, *J*=8.8 Hz, 5'-H), 6.98 (1H, d, *J*=8.4 Hz, 6'-H), 7.27, 8.09 (1H, s, OH for each).
¹³C-NMR data of Compound No. A-7 (δ ppm, acetone-d₆, 100 MHz) : 17.88, 17.88, 23.16, 23.30, 25.84, 25.86, 27.23, 32.17, 55.89, 60.55, 70.40, 99.80, 103.98, 108.27, 114.42, 117.68, 122.39, 123.75, 125.27, 125.32, 130.28, 131.78, 153.73, 154.39, 155.47, 157.82, 158.29.

### (Example 4)

Fraction 4 (117 mg) was further eluted by a reverse phase high performance liquid chromatography using methanol, whereby 66.5 mg of Compound No. A-8 was obtained.

### Compound No. A-8

m.p. 130∼132 °C (needles from Hexane-EtOAc),
¹H-NMR of Compound No. A-8 (δ ppm, acetone-d₆, 400 MHz) : 1.65, 1.66, 1.75, 1.78 (3H, s, 3-methyl-2-butenyl-CH₃ for each), 2.73 (1H, m, 4-H), 2.90 (1H, m, 4-H), 3.28 (2H, br-dd, *J*=7.2, 8.0 Hz, 3-methyl-2-butenyl-CH₂), 3.4 (1H, m, 3-H), 3.39 (2H, br-d, *J*=5.6, 3-methyl-2-butenyl-CH₂), 3.68 (3H, s, 5-OMe), 3.74 (3H, s, 2'-OMe), 3.89 (1H, t, *J*=10.0 Hz, 2-H), 4.12 (1H, m, 2-H), 5.25, 5.29 (1H, m, 3-methyl-2-butenyl-CH for each), 6.18 (1H, s, 8-H), 6.68 (1H, d, *J*=8.4 Hz, 5'-H), 6.94 (1H, d, *J*=8.8 Hz, 6'-H), 8.12, 8.26 (1H, s, OH for each).
¹³C-NMR data of Compound No. A-8 (δ ppm, acetone-d₆, 100 MHz) : 17.88, 17.96, 23.31, 24.02, 25.82, 25.82, 28.16, 31.90, 60.57, 62.33, 71.01, 99.84, 108.34, 112.25, 114.51, 122.54, 124.47, 125.26, 125.89, 126.58, 130.29, 131.08, 154.33, 155.53, 156.00, 158.28, 158.28.

### (Formulation Preparation Example 1)

Hereinafter, an exemplary formulation of a composition in the form of a medicine, for example, will be described.

### Formulation Example 1. powder

Five grams of licoricidin (Compound No. A-1), 800 g of lactose, and 100 g of corn starch are mixed by using a blender, thereby obtaining a powder drug.

### Formulation Example 2. capsules

Five grams of licoricidin (Compound No. A-1), 100 g of lactose, 50 g of corn starch, and 2 g of magnesium stearate are mixed, and then 0.20 g of this is filled in a capsule, thereby obtaining a capsule drug.

### (Test Example 1)

### Action on the blastogenesis of mouse spleen cells

Serial two-fold dilutions (100 µl/well) with RPMI1640 (Nikken Biomedical Laboratory) containing 10% FBS (bovine fetal serum: Dainippon Pharmaceutical Co., Ltd.) of test compounds which were dissoloved in a dimethylsulfoxide (DMSO) solution were obtained on a 96-well microplate, and used as test samples.

The spleen of a BALB/c mouse (a 6 week-old female) was aseptically isolated, and gently ground on a wire mesh with dropwise addition of sterilzed saline. The filtrate was passed through a nylon mesh (Becton Dickinson; pore size: 70 µm), thereby preparing a unicellular suspension.

After being washed twice with sterilized saline, the spleen cells were suspended in RPMI1460 containing 10% FCS and containing 1 µg/ml of concanavalin A (abbreviated as ConA: Sigma, U.S.) as well as an antibiotic (Sigma: Antibiotic Antimycotic), and dispensed into a 96-well microplate, in which 100 µl each of diluted test samples had previously been dispensed, in amounts of 3×10⁵ cells/100 µl/well.

Thereafter, culturing was carried out under 5% carbon dioxide gas at 37°C for 3 days, and then the blastogenesis of lymphocytes was measured by an MTT reduction method [Mosmann T, Journal of Immunological Method, vol. 65, p. 55, 1983]. The results are shown in [Table 4].

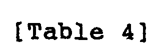

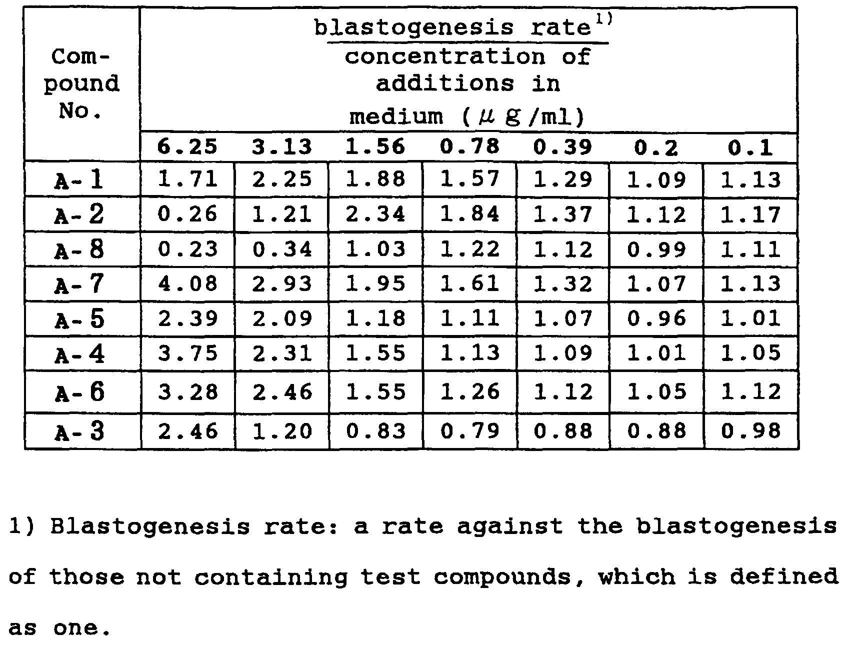

### (Test Example 2)

The metabolism promoting action of the compound according to the present invention on mouse bone marrow cells is demonstrated in [Table 5]. Test compounds were added at a concentration of 0.39 to 12.5 µg/ml to an RPMI1640 medium containing 2×10⁶ cells/ml of BALB/c mouse bone marrow cells, antibiotics, 10% bovine fetal serum, and cultured under 5% carbon dioxide gas at 37°C for 5 days, and the mitochondrial metabolism activity of the bone marrow cells was measured by an MTT reduction method. The data shown in the table shows magnitudes of metabolism where the cellular metabolism of those not containing test compounds is defined as one. In the table, "N.T." indicates that no test was performed.

**[Table 5]**

| metabolism activity of mouse bone marrow cells | | | | | | |
|---|---|---|---|---|---|---|
| Compound No. | Test compound concentration 12.5 µg/ml | Test com-pound concentration 6.25 µg/ml | Test compound concentration 3.13 µg/ml | Test compound concentration 1.56 µg/ml | Test compound concentration 0.78 µg/ml | Test compound concentration 0.39 µg/ml |
| A-1 | 0.58 | 1.98 | 1.27 | 0.93 | 1.01 | 1.14 |
| A-3 | 0.62 | 1.11 | 1.14 | 1.14 | 1.08 | 1.11 |
| A-4 | 0.58 | 1.33 | 1.32 | 1.21 | 1.07 | 1.07 |
| A-5 | 0.53 | 0.55 | 1.46 | 1.38 | 1.20 | 1.16 |
| A-6 | 0.51 | 0.77 | 1.52 | 1.20 | 1.05 | 0.97 |
| A-7 | 0.51 | 0.51 | 1.52 | 1.39 | 1.12 | 1.03 |
| A-8 | 0.51 | 0.50 | 1.56 | 1.61 | 1.27 | 1.13 |
| A-10 | 0.47 | 0.37 | 1.43 | 1.17 | 1.02 | 1.09 |
| A-11 | 0.84 | 0.43 | 1.69 | 1.36 | 1.08 | 1.05 |
| A-12 | 0.50 | 1.22 | 1.39 | 1.08 | 0.98 | 0.97 |
| A-13 | 2.46 | 2.04 | 1.74 | 1.44 | 1.43 | 1.20 |
| A-14 | 2.09 | 1.99 | 1.84 | 1.66 | 1.49 | 1.34 |
| A-17 | 1.56 | 1.77 | 1.64 | 1.26 | 1.17 | 1.16 |
| A-18 | 1.14 | 1.19 | 1.15 | 1.09 | 1.02 | 1.03 |
| A-19 | 0.53 | 1.26 | 1.28 | 1.20 | N.T. | N.T. |
| A-20 | 0.94 | 1.16 | 1.34 | 1.35 | N.T. | N.T. |
| A-21 | 0.44 | 0.89 | 1.22 | 1.31 | N.T. | N.T. |
| A-22 | 0.50 | 0.40 | 1.76 | 1.86 | N.T. | N.T. |
| B-1 | 1.78 | 1.88 | 1.57 | 1.36 | 1.16 | 1.14 |
| B-2 | 1.00 | 2.93 | 1.46 | 1.10 | 1.16 | 1.06 |
| B-3 | 2.04 | 1.83 | 1.62 | 1.10 | 1.11 | 1.14 |
| B-4 | 0.44 | 2.58 | 1.76 | 1.25 | 1.15 | 1.10 |
| B-5 | 0.31 | 2.05 | 2.45 | 1.65 | 1.15 | 1.00 |
| B-6 | 1.56 | 1.73 | 1.44 | 1.20 | 1.03 | 1.00 |
| B-7 | 0.67 | 1.32 | 1.41 | 1.25 | 1.00 | 0.99 |
| B-8 | 0.83 | 1.18 | 0.90 | 0.90 | 0.91 | 0.94 |
| B-9 | 0.25 | 1.10 | 1.65 | 1.50 | 1.33 | 1.10 |
| B-10 | 0.23 | 0.45 | 1.19 | 2.04 | 1.45 | 1.18 |
| B-11 | 0.32 | 0.88 | 1.39 | 1.25 | 1.29 | 1.02 |
| B-12 | 1.36 | 1.29 | 1.22 | 1.22 | N.T. | N.T. |
| B-13 | 1.62 | 1.52 | 1.45 | 1.21 | N.T. | N.T. |
| B-14 | 1.48 | 1.42 | 1.35 | 1.16 | 1.08 | 1.11 |
| B-15 | 1.58 | 1.40 | 1.45 | 1.22 | 1.21 | 1.09 |

### INDUSTRIAL APPLICABILITY

According to the present invention, licorisoflavan A, licoricidin, and novel isoflavan derivatives are provided, the use of which is expected to provide effects as a biophylaxis function promoter, by way of lymphocyte function promoting actions and bone marrow function promoting actions.

## Claims

1. A compound represented by general formula (I), or a salt thereof, or a hydrate thereof: 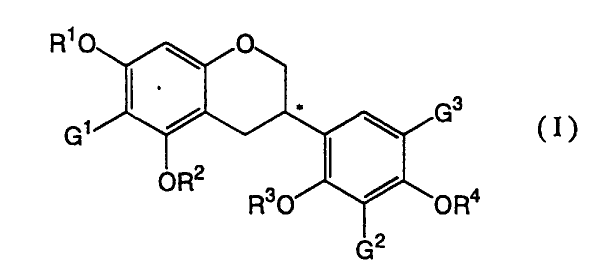 [where R¹, R², R³, and R⁴ may be identical with or different from one another and may each be hydrogen, an alkyl group which may be substituted, an alkenyl group which may be substituted, an alkynyl group which may be substituted, an acyl group which may be substituted, an aralkyl group which may be substituted, or a tri-substituted silyl group; G¹, G², and G³ may be identical with or different from one another and may each be a hydrogen, an acyl group which may be substituted, or an aliphatic hydrocarbon group which may be substituted; symbol (*) indicates the presence of an asymmetric carbon atom, meaning an R form, an S form, or a mixture thereof, with the provisos that:
i) G³ is not hydrogen in the case where R¹ is hydrogen, R² is methyl, R³ and R⁴ are hydrogen, and G¹ and G² are 3-methyl-2-butenyl;
ii) G³ is not hydrogen in the case where R¹ is acetyl, R² is methyl, R³ and R⁴ are acetyl, and G¹ and G² are 3-methyl-2-butenyl;
iii) G³ is not hydrogen in the case where R¹ is methyl, R², R³, and R⁴ are hydrogen, and G¹ and G² are 3-methyl-2-butenyl;
iv) G³ is not hydrogen in the case where R¹ and R² are methyl, R³ and R⁴ are hydrogen, and G¹ and G² are 3-methyl-2-butenyl;
v) G³ is not hydrogen in the case where R¹ is hydrogen, R² is methyl, R³ and R⁴ are hydrogen, G¹ is 3-methyl-2-butenyl, and G² is hydrogen;
vi) G³ is not hydrogen in the case where R¹ and R² are methyl, R³ and R⁴ are hydrogen, G¹ is 3-methyl-2-butenyl, and G² is hydrogen;
vii) G³ is not hydrogen in the case where R¹ and R² are methyl, R³ and R⁴ are acetyl, G¹ is 3-methyl-2-butenyl, and G² is hydrogen;
viii) G³ is not 3-methylbutyl in the case where R¹ is hydrogen, R² is methyl, R³ and R⁴ are hydrogen, G¹ is 3-methylbutyl, and G² is hydrogen;
ix) G³ is not hydrogen in the case where R¹ is hydrogen, R² and R³ are methyl, R⁴ is hydrogen, G¹ is 3-methyl-2-butenyl, and G² is hydrogen;
x) G³ is not hydrogen in the case where R¹ and R² are methyl, R³ and R⁴ are 2-(trimethylsilyl)ethoxymethyl (SEM), G¹ is 3-methyl-2-butenyl, and G² is hydrogen;
xi) G³ is not hydrogen in the case where R¹ and R² are methyl, R³ and R⁴ are 2-(trimethylsilyl)ethoxymethyl (SEM), G¹ is 2-hydroxy-3-methylbutyl, and G² is hydrogen;
xii) G³ is not hydrogen in the case where R¹ and R² are methyl, R³ is hydrogen, R⁴ is t-butyldimethylsilyl (TBS), G¹ is 3-methyl-2-butenyl, and G² is hydrogen;
xiii) G³ is not 3-methyl-2-butenyl in the case where R¹ and R² are methyl, R³ and R⁴ are hydrogen, and G¹ and G² are 3-methyl-2-butenyl;
xiv) G³ is not hydrogen in the case where R¹ and R² are methyl, R³ is hydrogen, R⁴ is t-butyldimethylsilyl (TBS), and G¹ and G² are 3-methyl-2-butenyl;
xv) G³ is not hydrogen in the case where R¹ and R² are methyl, R³ and R⁴ are t-butyldimethylsilyl (TBS), G¹ is 3-methyl-2-butenyl, and G² is hydrogen;
xvi) G³ is not hydrogen in the case where R¹ is hydrogen, R² is methyl, R³ is hydrogen, R⁴ is methyl, G¹ is hydrogen, and G² is 3-methyl-2-butenyl;
xvii) G³ is not hydrogen in the case where R¹ and R² are methyl, R³ and R⁴ are acetyl, and G¹ and G² are hydrogen;
xviii) G³ is not hydrogen in the case where R¹ and R² are methyl, and R³, R⁴, G¹ and G² are hydrogen;
xix) G³ is not hydrogen in the case where R¹ is benzyl, R² is methyl, R³ is benzyl, R⁴ is methyl, and G¹ and G² are hydrogen;
xx) G³ is not hydrogen in the case where R¹ is hydrogen, R² is methyl, R³ is hydrogen, R⁴ is methyl, and G¹ and G² are hydrogen;
xxi) G³ is not hydrogen in the case where R¹, R², R³ and R⁴ are methyl, and G¹ and G² are hydrogen; and
xxii) G³ is not hydrogen in the case where R¹ is acetyl, R² is methyl, R³ is acetyl, R⁴ is methyl, and G¹ and G² are hydrogen].

2. A compound, or a salt thereof, or a hydrate thereof according to claim 1, wherein R² is methyl.

3. A compound, or a salt thereof, or a hydrate thereof according to claim 1, wherein R³ and/or R⁴ is methyl.

4. A compound, or a salt thereof, or a hydrate thereof according to claim 1, wherein G¹ and/or G² is an aliphatic hydrocarbon group which may be substituted with a hydroxyl group, an alkoxy group, an alkenyloxy group, or an acyl group.

5. A composition having immunoactivating action containing a compound represented by general formula (II), or a salt thereof, or a hydrate thereof: 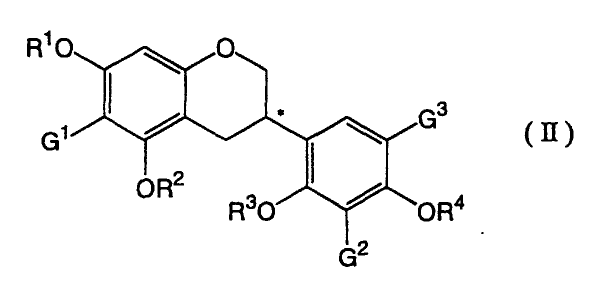 [where R¹, R², R³, and R⁴ may be identical with or different from one another and may each be hydrogen, an alkyl group which may be substituted, an alkenyl group which may be substituted, an alkynyl group which may be substituted, an acyl group which may be substituted, an aralkyl group which may be substituted, or a tri-substituted silyl group; G¹, G², and G³ may be identical with or different from one another and may each be a hydrogen, an acyl group which may be substituted, or an aliphatic hydrocarbon group which may be substituted; symbol (*) indicates the presence of an asymmetric carbon atom, meaning an R form, an S form, or a mixture thereof].

6. A composition according to claim 5 having a bone marrow cellular metabolism promoting action.

7. A composition according to claim 5 having a leukocyte growing action.

8. A composition according to claim 5 having a lymphocyte function regulating action.

9. A composition according to any of claims 5 to 8, wherein G¹ and/or G² is an aliphatic hydrocarbon group which may be substituted with a hydroxyl group, an alkoxy group, an alkenyloxy group, or an acyl group.

10. A composition according to any of claims 5 to 8, wherein G¹ and/or G² is an alkenyl which may be substituted.

11. A composition according to claim 10, wherein the alkenyl which may be substituted is 3-methyl-2-butenyl.

12. A composition according to any of claims 5 to 11, wherein R² is an alkyl.

13. A composition according to claim 12, wherein the alkyl is methyl.

14. A composition according to any of claims 5 to 13, wherein R³ and/or R⁴ is an alkyl.

15. A composition according to claim 14, wherein the alkyl is methyl.

16. A composition according to any of claims 5 to 15, wherein G³ is hydrogen.

17. A composition according to any of claims 5 to 16 which is a medicine, animal medicine, food, or cosmetic.
